# EUROPEAN PATENT SPECIFICATION

(11) **EP 2 444 494 B1**
(45) Date of publication and mention of the grant of the patent: **28.09.2016**
(21) Application number: 11181596.5
(22) Date of filing: 09.10.2007
(51) Int. Cl.: C12N 15/113

(54) **RNA ANTAGONIST COMPOUNDS FOR THE MODULATION OF PCSK9**
RNA-ANTAGONISTENVERBINDUNGEN ZUR MODULATION VON PCSK9
COMPOSÉS ANTAGONISTES DE L'ARN POUR LA MODULATION DE PCSK9

(30) Priority: 09.10.2006 US 828735 P; 17.09.2007 US 972932 P; 04.10.2007 US 977409 P
(43) Date of publication of application: 25.04.2012
(62) Divisional of application: 07821072.1
(73) Proprietor: Roche Innovation Center Copenhagen A/S, 2970 Hørsholm (DK)
(72) Inventor: Straarup, Ellen Marie, 3460 Birkerød (DK); Nielsen, Niels Fisker, DK-2800 Kgs. Lyngby (DK)
(74) Representative: Turner, Mark Frederic Paris

(56) References cited:
- WO-A2-2004/046160
- WO-A2-2008/011431
- US-A1- 2004 009 553
- LALANNE FLORENT ET AL: "Wild-type PCSK9 inhibits LDL clearance but does not affect apoB-containing lipoprotein production in mouse and cultured cells", JOURNAL OF LIPID RESEARCH, AMERICAN SOCIETY FOR BIOCHEMISTRY AND MOLECULAR BIOLOGY, INC, US, vol. 46, no. 6, 1 June 2005 (2005-06-01), pages 1312-1319, XP002471922, ISSN: 0022-2275, DOI: 10.1194/JLR.M400396-JLR200
- GRAHAM MARK J ET AL: "Antisense inhibition of proprotein convertase subtilisin/kexin type 9 reduces serum LDL in hyperlipidemic mice", JOURNAL OF LIPID RESEARCH, AMERICAN SOCIETY FOR BIOCHEMISTRY AND MOLECULAR BIOLOGY, INC, US, vol. 48, no. 4, 1 April 2007 (2007-04-01), pages 763-767, XP002471923, ISSN: 0022-2275, DOI: 10.1194/JLR.C600025-JLR200 [retrieved on 2007-01-22]
- KURRECK J ET AL: "Design of antisense oligonucleotides stabilized by locked nucleic acids", NUCLEIC ACIDS RESEARCH, OXFORD UNIVERSITY PRESS, SURREY, GB, vol. 30, no. 9, 1 May 2002 (2002-05-01), pages 1911-1918, XP002281375, ISSN: 0305-1048
- HORTON ET AL: "Molecular biology of PCSK9: its role in LDL metabolism", TRENDS IN BIOCHEMICAL SCIENCES, ELSEVIER, HAYWARDS, GB, vol. 32, no. 2, 6 February 2007 (2007-02-06), pages 71-77, XP005876225, ISSN: 0968-0004, DOI: 10.1016/J.TIBS.2006.12.008
- FREIER S M ET AL: "The ups and downs of nucleic acid duplex stability: structure-stability studies on chemically-modified DNA:RNA duplexes", NUCLEIC ACIDS RESEARCH, OXFORD UNIVERSITY PRESS, GB, vol. 25, no. 22, 1 January 1997 (1997-01-01), pages 4429-4443, XP003018113, ISSN: 0305-1048, DOI: 10.1093/NAR/25.22.4429
- UHLMANN E: "RECENT ADVANCES IN THE MEDICINAL CHEMISTRY OF ANTISENSE OLIGONUCLEOTIDES", CURRENT OPINION IN DRUG DISCOVERY AND DEVELOPMENT, CURRENT DRUGS, LONDON, GB, vol. 3, no. 2, 1 January 2000 (2000-01-01) , pages 203-213, XP008019650, ISSN: 1367-6733

## Description

### FIELD OF THE INVENTION

The present invention provides compounds, compositions and methods for modulating the expression of PCSK9. In particular, this invention relates to oligomeric compounds, such as oligonucleotide compounds, which are hybridisable with target nucleic acids encoding PCSK9, and methods for the preparation of such oligomeric compounds. The oligonucleotide compounds have been shown to modulate the expression of PCSK9, and pharmaceutical preparations thereof and their use as treatment of hypercholesterolemia and related disorders are disclosed.

### BACKGROUND

Proprotein convertase subtilisin/kexin type 9a (PCSK9) is a member of the proteinase K subfamily of subtilases. The PCSK9 gene (NARC-1) has been identified as a third locus involved in autosomal dominant hypercholesterolemia (ADH), characterised by high levels of low-density lipoprotein (LDL), xhantomas, and a high frequency of coronary heart disease. The other two loci being apolipoprotein-B (Apo-B) and the LDL receptor (LDLR). PCSK9 acts as a natural inhibitor of the LDL-receptor pathway, and both genes are regulated by depletion of cholesterol cell content and statins via sterol regulatory element-binding protein (SREBP). PCSK9 mRNA and protein levels are regulated by food intake, insulin and cell cholesterol levels (Costet et al., J. Biol. Chem. January 2006).

The human NARC1 mRNA (cDNA) sequence, which encodes human PCSK9 is shown as SEQ ID NO 2 (NCBI Acc. No. NM_174936).

The human PCSK9 polypeptide sequence (nascent) is shown as SEQ ID NO 1 (NCBI Acc. No. NP_777596. The polypeptide has a signal peptide between residues 1-30, which is co-translationally cleaved to produce a proprotein (31-692 of SEQ ID No 1), which is subsequently cleaved by a protease to produce a mature protein corresponding to amino acids 83-692 of SEQ ID NO 1. A glycosylation site has been characterised at residue 533.

Park et al., (J. Biol. Chem. 279, pp50630-50638, 2004) discloses that over-expression of PCSK9 reduced LDLR protein resulting in an increase in plasma LDL cholesterol, and suggests that an inhibitor of PCSK9 function may increase LDLR protein levels and enhance LDL clearance from plasma.

Rashid et al., (2005, PNAS 102, No 15, pp5374-5379) discloses that knockout mice lacking PCSK9 manifest increased LDLR protein leading to an increased clearance of circulating lipoproteins and decreased plasma cholesterol levels, and suggests that inhibitors of PCSK9 may be useful for the treatment of hypercholesterolemia and that there may be synergy between inhibitors of PCSK9 and statins to enhance LDLRs and reduce plasma cholesterol.

WO01/57081 discloses the NARC-1 polynucleotide sequence and discloses that antisense nucleic acids can be designed using the NARC-1 polynucleotide sequence, and that such antisense nucleic acids may comprise modified nucleotides or bases, such as peptide nucleic acids.

WO2004/097047, which discloses two mutants of PCSK9 which are associated with ADH, suggests that antisense or RNAi of such PCSK9 mutants may be used for treatment of ADH.

Lalanne Florent et al., J. Lipid Research, vol 46, No 6 pp 1312-1319 (2005) and US 2004/009553 refers to siRNA molecules which decrease the expression of PCSK9 gene.

Kurreck et al., Nucleic Acid Research 30, pp1911-1918 (2002) discloses the design of antisense oligonucleotides containing locked nucleic acids.

### OBJECT OF THE INVENTION

The invention provides therapeutic solutions for the treatment of hypercholesterolemia and related disorders, based upon antisense oligonucleotides, targeted against PCSK9 nucleic acids. The inventors have discovered that the use of nucleotide analogues which have an enhanced affinity for their complementary binding partner, such as Locked Nucleic Acid (LNA) nucleotide analogues, within antisense oligonucleotides that are targeted towards PCSK9 target nucleic acids, provide highly effective modulation, particularly the down-regulation, of PCSK9 (NARC1) expression.

### SUMMARY OF THE INVENTION

The invention provides for antisense oligonucleotides as defined in claim 1.

The antisense oligonucleotide is of between 10-25 nucleobases in length which consists or comprises a contiguous nucleobase sequence of a total of between 10-25 nucleobases, wherein said contiguous nucleobase sequence is complementary to a corresponding region of a SEQ ID NO 2. The antisense oligonucleotide is not a siRNA.

The invention further provides a conjugate comprising the antisense oligonucleotide according to the invention, such as a conjugate which, in addition to the nucloebase sequence of the antisense oligonucleotide comprises at least one non-nucleotide or non-polynucleotide moiety covalently attached to the antisense oligonucleotide of the invention.

The antisense oligonucleotide may consist of a sequence of total of between 10 - 25 nucleobases, which are complementary to a contiguous sequence which is present in SEQ ID NO 2.

The antisense oligonucleotide according to the invention, is for use as a medicament as defined in claim 15.

The antisense oligonucleotides of the invention may be used in methods of modulating the expression of PCSK9 in mammalian cells or tissues comprising contacting said mammalian cells or tissues with one or more of the oligomeric compounds or compositions of the invention. Typically the expression of PCSK9 is inhibited or reduced.

The antisense oligonucleotides of the invention may be used in methods of treating a mammal, such as a human, suspected of having or being prone to a disease or condition, associated with expression of PCSK9, such as hypercholesterolemia or related disorder, by administering a therapeutically or prophylactically effective amount of one or more of the oligomeric compounds or compositions of the invention.

The antisense oligonucleotides of the invention may be used in methods for the inhibition of expression of PCSK9 and for treatment of diseases associated with PCSK9 activity are provided, such as hypercholesterolemia and/or related disorders.

The invention provides for pharmaceutical composition comprising the antisense oligonucleotide or conjugate of the invention, and a pharmaceutically acceptable diluent, carrier, salt or adjuvant.

The invention also provides pharmaceutical compositions which comprise antisense oligonucleotide compounds according to the invention and further compounds capable of modulating blood serum cholesterol levels, such as apolipoprotein B (Apo-B100) modulators, in particular antisense oligonucleotides (oligomers) targeted to Apo-B nucleic acid targets.

The antisense oligonucleotides of the invention may be used in a method of (i) reducing the level of blood serum cholesterol or ii) reducing the level of blood serum LDL-cholesterol, or iii) for improving the HDL/LDL ratio, in a patient, the method comprising the step of administering the antisense oligonucleotide or the conjugate or the pharmaceutical composition according to the invention to the patient.

The antisense oligonucleotides of the invention may be used in a method of lowering the plasma triglyceride in a patient, the method comprising the step of administering the antisense oligonucleotide or the conjugate or the pharmaceutical composition according to the invention to the patient so that the blood serum triglyceride level is reduced.

The antisense oligonucleotides of the invention may be used in a method of treating obesity in a patient, the method comprising the step of administering the antisense oligonucleotide or the conjugate or the pharmaceutical composition according to the invention to the patient in need of treatment so that the body weight of the patient is reduced.

invention The antisense oligonucleotides of the invention may be used in a method of treating hypercholesterolemia, or related disorder, in a patient, the method comprising the step of administering the antisense oligonucleotide or the conjugate or the pharmaceutical composition according to the invention to the patient in need of treatment for hypercholesterolemia, or related disorder.

The antisense oligonucleotides of the invention may be used in a method of treating insulin resistance in a patient, the method comprising the step of administering the antisense oligonucleotide or the conjugate or the pharmaceutical composition according to the invention to the patient in need of treatment so that the patient's sensitivity to insulin is increased.

The antisense oligonucleotides of the invention may be used in a method of treating type II diabetes in a patient, the method comprising the step of administering the antisense oligonucleotide or the conjugate or the pharmaceutical composition according to the invention to the patient suffering from type II diabetes.

The antisense oligonucleotides of the invention may be used in a method for treating a metabolic disorder such as metabolic syndrome, diabetes or atherosclerosis, the method comprising the step of administering the antisense oligonucleotide or the conjugate or the pharmaceutical composition according to the invention to the patient in need thereof.

The invention provides for the antisense oligonucleotide or conjugate according to the invention for use in the treatment of a disease or disorder selected from the group consisting of: hypercholesterolemia or related disorder, as according to claim 17.

### BRIEF DESCRIPTION OF THE FIGURES

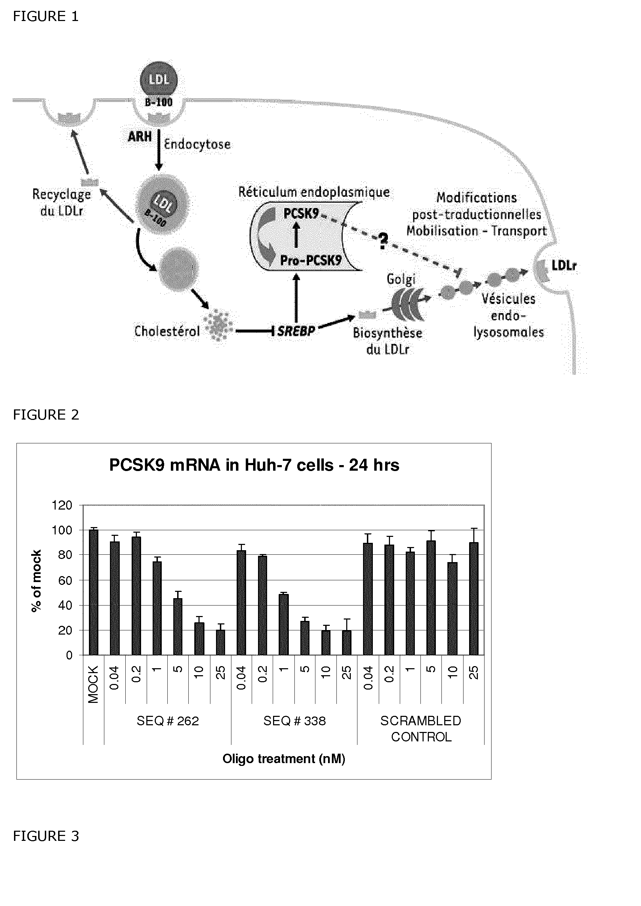

**Figure 1** shows a diagrammatic representation of the interaction of PCSK9 and the LDLr: PCSK9 alters the expression of the LDL receptor (LDLr). LDLr is expressed at the basolateral surface of hepatocytes and interacts with apoB-100, thereby allowing the uptake of plasma LDL and possibly that of nascent VLDL. The cellular internalisation of apoB-100 containing lipoproteins requires the ARH (Autosomal Recessive Hypercholesterolemia) adaptor protein. PCSK9 alters the post-translational expression of LDLr. *PCSK9* and *LDLr* genes are upregulated upon low levels of intracellular cholesterol, indicating that both genes are indirect targets of HMGCoA reductase inhibitors (statins) - (Lambert et al. 2006, TRENDS in Endocrinology and Metabolism, 17:79-81).
**Figure 2** PCSK9mRNA expression in Huh-7 cells 24 hours after transfection with Lipofectamine and LNA oligonucleotides Compound ID NO#s: 262 or 338 at 0.04, 0.2, 1, 5, 10 or 25 nM. Data are normalised to Gapdh and presented relative to the mock control.
**Figure 3** PCSK9mRNA expression in Huh-7 cells 24 hours after transfection with Lipofectamine and LNA oligonucleotides Compound ID NO#s: 98 or 101 at 0.04, 0.2, 1, 5 or 10 nM. Data are normalised to Gapdh and presented relative to the mock control.
**Figure 4****.** PCSK9mRNA expression in Huh-7 cells 24 hours after transfection with Lipofectamine and LNA oligonucleotides Compound ID NO#s: 9, 16 or 18 at 0.04, 0.2, 1, 5, 10 or 25 nM. Data are normalised to Gapdh and presented relative to the mock control.
**Figure 5****.** In vitro results in the Murine hepatocarcinoma cell line Hepa 1-6: PCSK9mRNA expression in Huh-7 cells 24 hours after transfection with Lipofectamine and LNA oligonucleotides Compound ID No#s: 262 and 338 at 0.04, 0.2, 1, 5, 10 or 25 nM. Data are normalised to Gapdh and presented relative to the mock control.
**Figure 6****.** PCSK9mRNA expression in Huh-7 cells 24 hours after transfection with Lipofectamine and LNA oligonucleotides Compound ID NO#s: 98 and 101 at 0.04, 0.2, 1, 5, 10 or 25 nM. Data are normalised to Gapdh and presented relative to the mock control.
**Figure 7****.** In vivo examination of LNA oligonucleotides in female C57BL/6 mice: PCSK9 mRNA expression in Liver following dosing 5, 10 or 15 mg/kg Compound ID NO#s: 98, 101 or 317 Days 0, 3, 7, 10,and 14 and. Day 16 the mice were sacrificed and the liver was examined by qPCR for PCSK9 mRNA expression. Data represent mean SD and is presented relative to the saline group.
**Figure 8****.** Serum total-, VLDL+LDL- and HDL cholesterol measured at sacrifice day 16 in C57BL/6 female mice dosed 10 mg/kg /dose of Compound ID NO#s: 98 or 101 at days 0, 3, 7, 10 and 14 by tail vein injections.
**Figure 9****.** Liver was sampled at sacrifice day 16 and analysed for LDL-receptor protein level by Western Blotting as described in example 13.
**Figure 10****.** NMRI female mice: PCSK9 mRNA expression in Liver following dosing 10 mg/kg of Compound ID NO#s: 98 or 101 days 0, 3, 7, 10,and 14 and. Day 16 the mice were sacrificed and the liver was examined by qPCR for PCSK9 mRNA expression. Data represent mean SD and is presented relative to the saline group.
**Figure 11****.** Total cholesterol in serum from blood sampled at sacrifice (day 16)
**Figure 12****.** Liver was sampled at sacrifice day 16 and analysed for LDL-receptor protein level by Western Blotting as described in example 13.
**Figure 13****.** Efficacy study in femal and male C57BL/6 fe at High fat diet (HFD): PCSK9 mRNA expression in Liver following dosing 10 or 15 mg/kg of Compound ID NO#s: 98, 101 or 317 Days 0, 3, 7, 10,and 14 and. Day 16 the mice were sacrificed and the liver was examined by qPCR for PCSK9 mRNA expression. Female mice were fed a high diet (HFD) for 5 month before treatment with LNA oligonucleotides and male mice were fed HFD for one month before treatment. Data represent mean SD and is presented relative to the saline group.
**Figure 14****.** Liver was sampled at sacrifice day 16 and analysed for LDL-receptor protein level by Western Blotting as described in example 13.
**Figure 15****.** 13-mer LNA oligonucleotides tested in C57BL/6 female mice: PCSK9 mRNA expression in Liver following dosing 15 mg/kg of Compound ID NO#s: 9, 16, 18 or 98 Days 0, 2 and 4 and day 6 the mice were sacrificed and the liver was examined by qPCR for PCSK9 mRNA expression. Data are normalised to Gapdh and present relative to saline group in mean SD.
**Figure 16****.** The distribution of the different lipoprotein fractions HDL, VLDL and LDL in serum.
The lipoproteins were separated on Sebia Gels and quantified using Sudan Black staining and
Densiometric analysis (Molecular Imager FX). Data are presented as mean SD, n=5.

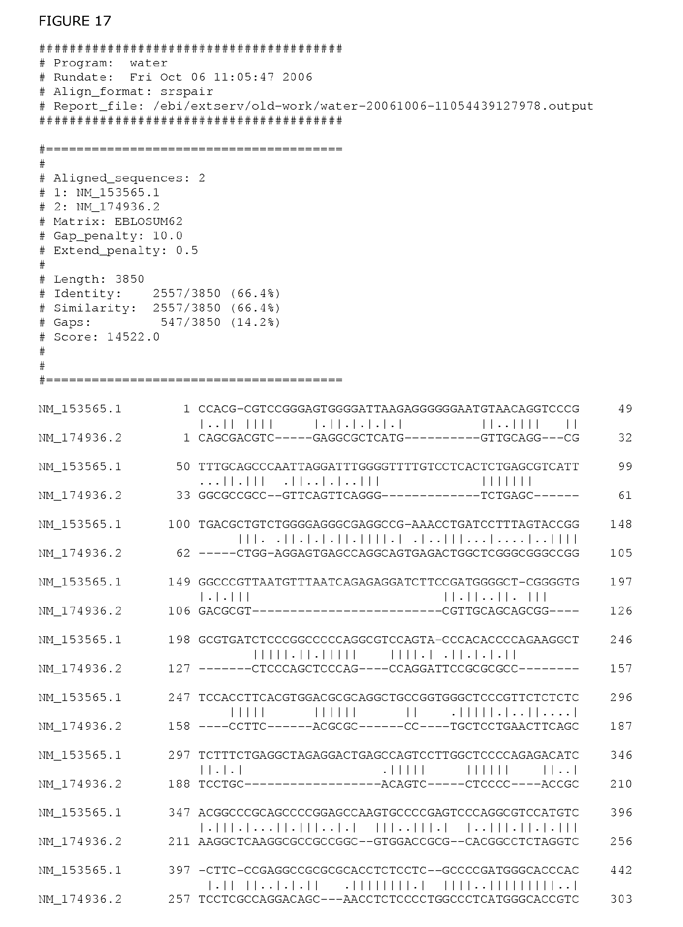

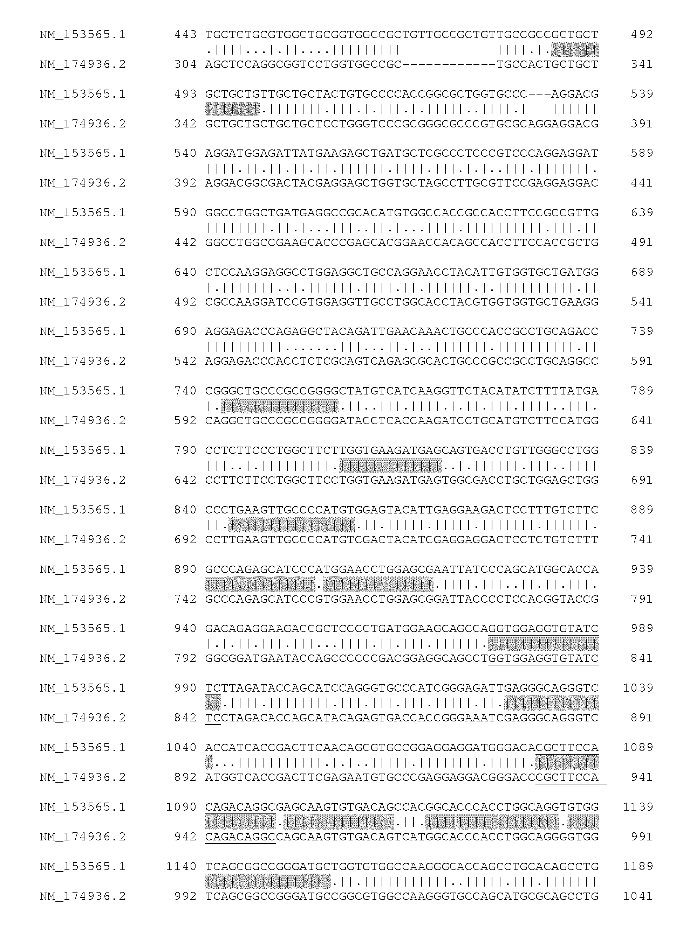

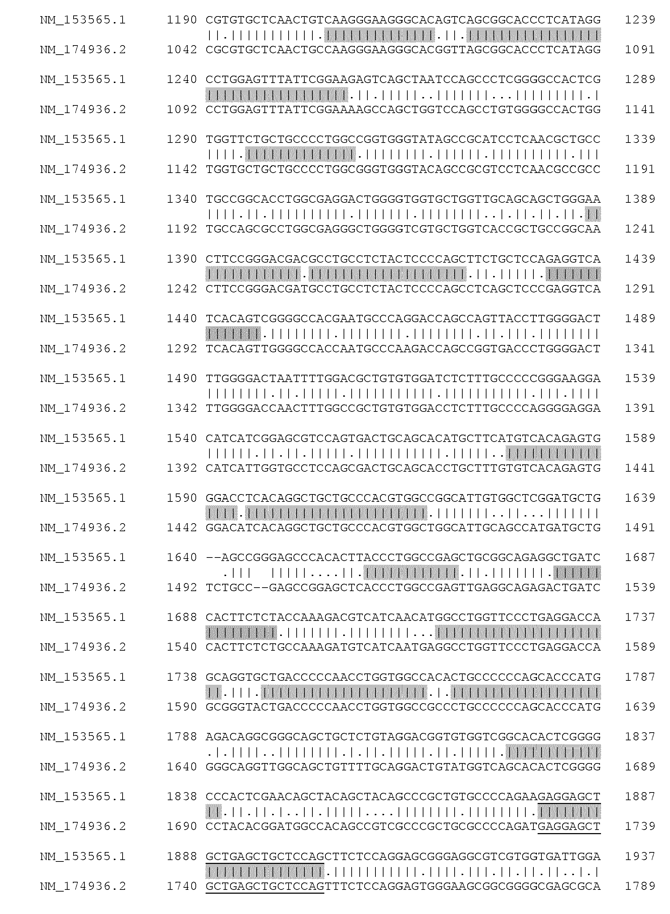

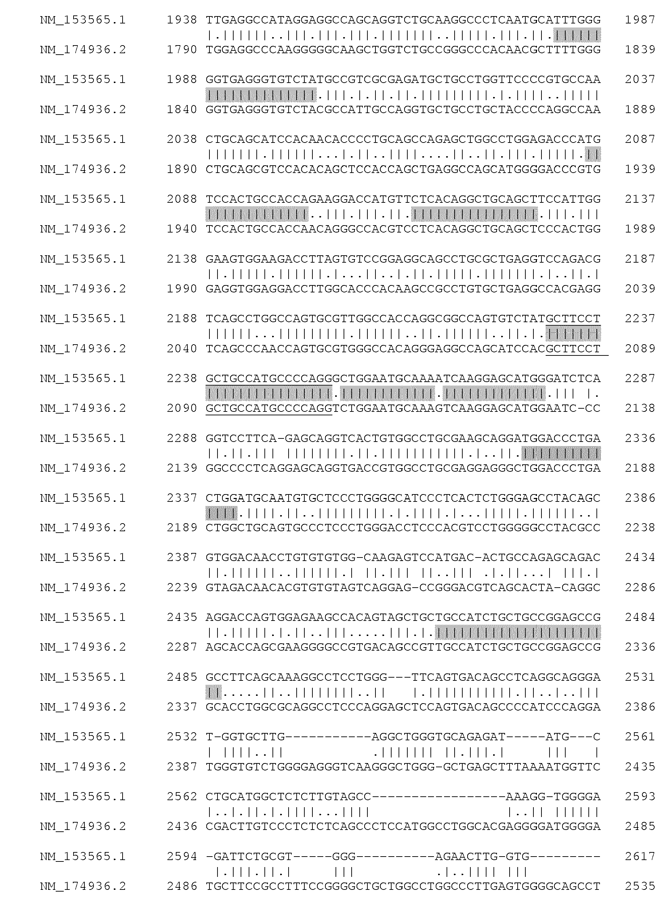

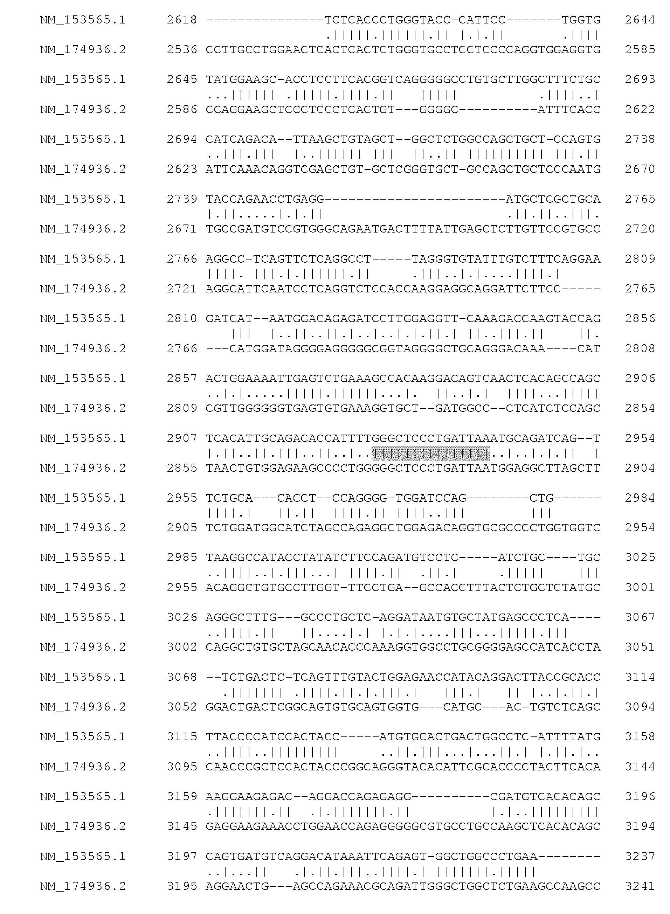

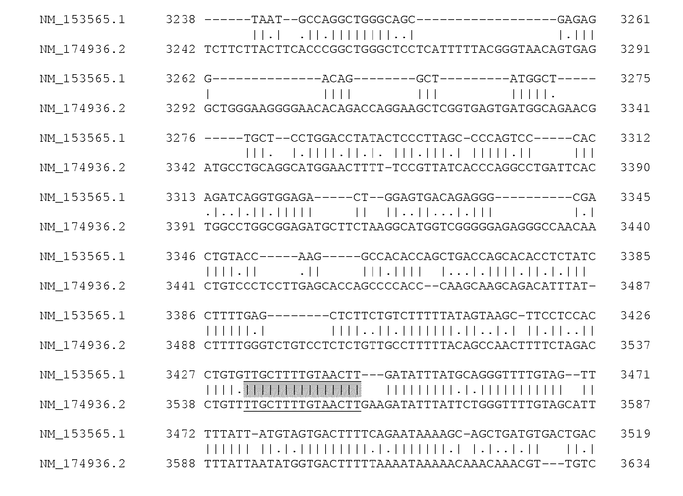

**Figure 17** shows a Clustal W local sequence alignment between the human NM_174936) and the mouse (NM_153565) PCSK9 encoding nucleic acids and illustrates regions where there are sufficient sequence homology to design oligomeric compounds which are complementary to both the human and mouse PCSK9 target nucleic acids, (illustrated by the vertical lines between the aligned nucleotides) shaded areas indicate preferred regions for targeting oligonucleotides to (preferably at a contiguous series of at least 12 conserved residues) both human and mouse PCSK9 activity, the underlined regions are regions which are particularly preferred.

### RELATED CASES

This case claims priority from US provisional application 60/828,735 and US 60/972,932,

Furthermore, this case claims priority from US 60/977,409.

### DESCRIPTION OF THE INVENTION

### Oligomers targeting PCSK9

The present invention employs antisense oligonucleotides, for use in modulating the function of nucleic acid molecules encoding human PCSK9, SEQ ID NO 1. The compound is complementary to a corresponding region of SEQ ID NO 2. The mammalian PCSK9 is human PCSK9.

The oligomer typically comprises or consists of a contiguous nucleobase sequence.

In one embodiment, the nucleobase sequence of the oligomer consists of the contiguous nucleobase sequence.

### Sub-seqeunces and flanking sequences

In one embodiment, the oligomeric compound comprises at least a core sub-sequence of at least 8, such as at least 10, such as at least 12, such as at least 13, such as at least 14 contiguous nucleobases, wherein said subsequence corresponds to a contiguous sequence present in SEQ ID NO 2.

Suitable sub-sequences may be selected from a sequence which corresponds to a contiguous sequence present in one of the nucleic acid sequences selected from the group consisting of SEQ ID NO 14, SEQ ID NO 15, SEQ ID No 16, SEQ ID NO 17, SEQ ID NO 18 and SEQ ID NO 19, or a sequence selected from the group of (antisense) sequences shown in tables 2 and 3, and (the complement of) the sequences of the highlighted (shaded) sequences (of complementarity between human and mouse PCSK9 mRNA) shown in Figure 17..

Preferred subsequences comprise or consist of at least 8, such as at least 10, such as at least 12, such as at least 13, such as at least 14 contiguous nucleobases which correspond to an equivalent nucleotide sequence present in any one of SEQ ID NO 3, SEQ ID NO 4, SEQ ID NO 5, SEQ ID NO 6, SEQ ID NO 7, or SEQ ID NO 8, most preferably SEQ ID NO 3 or SEQ ID NO 4.

The compound may further comprise a 5' flanking nucleobase sequence, or a 3' flanking sequence, or both a 5' and a 3' flanking sequence which is/are contiguous to said subsequence, wherein said flanking sequence or sequences consist of a total of between 2 and 22 nucleobase units, which when combined with said sub-sequence, the combined contiguous nucleobase sequence, *i.e.* consisting of said subsequence and said flanking sequence or sequences, is complementary to a corresponding region of SEQ ID NO 2.

The flanking sequence or sequences may consist of a total of between 2 and 22 nucleobase units, such 3, 4, 5, 6, 7, 8, 9, 10, 11, 12, 13, 14, 15, 16, 17, 18, 19, 20, or 21 nucleobases, or such as between 4 to 12 nucleobases or such as between 2 and 10 nucleobases, such as between 5 to 10 nucleobases, or between 5 and 8 nucleobases, such as between 7 to 9 nucleobases.

In one embodiment said flanking sequence comprises of at least 2 nucleobase units which are 5' to said sub-sequence.

In one embodiment said flanking sequence comprises between 1 and 6 nucleobase units which are 5' to said sub-sequence.

In one embodiment said flanking sequence comprises of at least 2 nucleobase units which are 3' to said sub-sequence.

In one embodiment said flanking sequence comprises between 1 and 6 nucleobase units which are 3' to said sub-sequence

It is preferred that the sequences of each of the flanking sequences each form a contiguous sequence.

### The combined contiguous nucleobase sequence

The combined contiguous nucleobase sequence, *i.e.* consisting of said subsequence and, if present, said flanking sequence or sequences, is complementary to a corresponding sequence present in SEQ ID NO 2.

In one embodiment, the 3' flanking sequence and/or 5' flanking sequence may, independently, comprise or consist of between 1 and 10 nucleobases, such as 2, 3, 4, 5, 6, 7, 8, or 9 nucleobases, such as between 2 and 6 nucleobases, such as 3 or 4 nucleobases, which may be, in one embodiment nucleotide analogues, such as LNA units, or in another embodiment a combination of nucleotides and nucleotide analogues.

### Nucleobase regions and conjugates

It will be recognised that the compound of the invention which consists of a contiguous sequence of nucleobases *(i.e.* a nucleobase sequence), may comprise further non-nucleobase components, such as the conjugates herein referred to.

Therefore, in one embodiment, the compound of the invention may comprise both a polynucleotide region, *i.e.* a nucleobase region, and a further non-nucleobase region. When referring to the compound of the invention consisting of a nucleobase sequence, the compound may comprise non-nucleobase components, such as a conjugate component.

Alternatively, the compound of the invention may consist entirely of a (contiguous) nucleobase region.

In one embodiment the nucleobase portion and/or subsequence is selected from at least 9, least 10, least 11, least 12, least 13, least 14 and least 15 consecutive nucleotides or nucleotide analogues, which preferably are complementary to the target nucleic acid(S)

In one embodiment, the compound according to the invention consists of no more than 22 nucleobases, such as no more than 20 nucleobases, such as no more than 18 nucleobases, such as 15, 16 or 17 nucleobases, optionally conjugated with one or more non-nucleobase entity.

### RNA antagonists

The nucleic acid which encodes a mammalian PCSK9 (target) may be in the sense or antisense orientation, preferably the sense orientation, such as the PCSK9 mRNA (of cDNA equivalent).

In one preferred embodiment, the compound may target a target nucleic acid which is an RNA transcript(s) of the gene(s) encoding the target proteins, such as mRNA or pre-mRNA,

The compound of the invention is an antisense oligonucleotide.

Suitably, when the antisense oligonucleotide is introduced into the cell which is expressing the PCSK9 gene, results in reduction of the PCSK9 mRNA level, resulting in reduction in the level of expression of the PCSK9 in the cell.

The oligomers which target the PCSK9 mRNA, may hybridize to any site along the target mRNA nucleic acid, such as the 5' untranslated leader, exons, introns and 3' untranslated tail. However, it is preferred that the oligomers which target the PCSK9 mRNA hybridise to the mature mRNA form of the target nucleic acid.

When designed as an antisense inhibitor, for example, the oligonucleotides of the invention bind to the target nucleic acid and modulate the expression of its cognate protein. Preferably, such modulation produces an inhibition of expression of at least 10% or 20% compared to the normal expression level, more preferably at least a 30%, 40%, 50%, 60%, 70%, 80%, 90% or 95% inhibition compared to the normal expression level. Suitably, such modulation is seen when using between 5 and 25nM concentrations of the compound of the invention. In the same of a different embodiment, the inhibition of expression is less than 100%, such as less than 98% inhibition, less than 95% inhibition, less than 90% inhibition, less than 80% inhibition, such as less than 70% inhibition. Modulation of expression level is determined by measuring protein levels, e.g. by the methods such as SDS-PAGE followed by western blotting using suitable antibodies raised against the target protein. Alternatively, modulation of expression levels can be determined by measuring levels of mRNA, eg. by northern blotting or quantitative RT-PCR. When measuring via mRNA levels, the level of down-regulation when using an appropriate dosage, such as between 5 and 25nM concentrations, is, in one embodiment, typically to a level of between 10-20% the normal levels in the absence of the compound of the invention.

The compound according to the invention is an antisense oligonucleotide.

The compound is not a siRNA.

In one embodiment, the compound of the invention does not comprise RNA (units).

The length of an oligomer (or contiguous nucleobase sequence) will be determined by that which will result in inhibition of the target. For a perfect match with the target, the contiguous nucleotide sequence or oligomer as low as 8 bases may suffice, but it will generally be more, e.g. 10 or 12, and preferably between 12-16. The maximum size of the oligomer will be determined by factors such as cost and convenience of production, ability to manipulate the oligomer and introduce it into a cell bearing the target mRNA, and also the desired binding affinity and target specificity. If too long, it may undesirably tolerate an increased number of mismatches, which may lead to unspecific binding.

The compound (oligomer or oligomeric compound) of the invention consists or comprises of between 10 and 25 nucleobases, such as 11, 12, 13, 14, 15, 16, 17, 18, 19, 20, 21, 22, 23, or 24 nucleobases.

Particularly preferred compounds are antisense oligonucleotides comprising from about 12 to 25 nucleobases and in one embodiment are antisense compounds comprising 13-18 nucleobases such as 13, 14, 15, 16 or 17 nucleobases. In one embodiment, the oligomer according to the invention consists of no more than 22 nucleobases. In one embodiment it is preferred that the compound of the invention comprises less than 20 nucleobases.

In one embodiment, the oligomer according to the invention consists of no more than 22 nucleobases, such as no more than 20 nucleobases, such as no more than 18 nucleobases, such as 15, 16 or 17 nucleobases, optionally conjugated with one or more non-nucleobase entity, such as a conjugate.

In one embodiment, the oligomer or contiguous nucleobase sequence has a length of between 10 - 22 nucleobases.

In one embodiment, the oligomer or contiguous nucleobase sequence has a length of between 10 - 18 nucleobases.

In one embodiment, the oligomer or contiguous nucleobase sequence has a length of between 10 - 16 nucleobases.

In one embodiment, the oligomer or contiguous nucleobase sequence has a length of between 12 - 16 nucleobases.

In one embodiment, the oligomer or contiguous nucleobase sequence has a length of between 12 - 14 nucleobases.

In one embodiment, the oligomer or contiguous nucleobase sequence has a length of between 14 - 16 nucleobases.

In one embodiment, the oligomer or contiguous nucleobase sequence has a length of between 14 - 18 nucleobases.

In one embodiment, the oligomer or contiguous nucleobase sequence has a length of 14, 15 or 16 nucleobases.

In one embodiment, the oligomer or contiguous nucleobase sequence has a length of between 10 - 14 nucleobases, such as 10, 11, 12, 13 or 13 nucleobases. As disclosed in US 60/977,409, such short oligonucleotides, *i.e.* "shortmers", are surprisingly effecting at target down-regulation *in vivo.*

### Preferred Sequences

Target sequences of the invention may, in one non limiting embodiment, be identified as follows. In a first step conserved regions in the target gene are identified. Amongst those conserved regions, any sequences with polymorphisms are normally excluded (unless required for a specific purpose) as these may affect the binding specificity and/or affinity of an oligomer designed to bind to a target sequence in this region. Any regions with palindromic or repeat sequences are normally excluded. The remaining regions are then analysed and candidate target sequences of suitable length (such as the lengths of the oligomer/contiguous nucleobase sequence referred to herein), e.g. 10-25 nucleobases, more preferably 10, 11, 12, 13, 14, 15 or 16 nucleobases are identified. Target sequences which are, based on computer analysis, likely to form structures such as dimers or hairpin structures are normally excluded.

Preferably these candidate target sequences show a high degree of sequence homology throughout the animal kingdom - or at least among animals likely to be required for pre-clinical testing. This allows the use of the identified oligomer sequences, and the corresponding oligomers such as antisense oligonucleotides, to be tested in animal models. Particularly useful are target sequences which are conserved in human, chimpanzee, dog, rat, mouse, and most preferred in human, and mouse (and/or rat).

Suitable nucleobase sequences, such as motif sequences of the oligomers of the invention, are provided in Table 3, herein.

In one embodiment the contiguous nucleobase sequence is a contiguous nucleotide sequence present in a nucleic acid sequence shown in table 3, such as a contiguous nucleotide sequence selected from the group consisting of SEQ ID NO 40 to SEQ ID NO 393; SEQ ID 30 to SEQ ID 39; SEQ ID NOs 3, 4 and 5.

Other preferred oligonucleotides include sequences of 10, 11, 12, 13, 14, 15 and 16 continuous (such as contiguous) nucleobases selected from a sequence from the group consisting of of SEQ ID NO 40 to SEQ ID NO 393; SEQ ID 30 to SEQ ID 39; SEQ ID NOs 3, 4 and 5.

Some preferred oligomers, and nucleobase sequences of the invention are shown in table 2.

In one embodiment the nucleobase portion (such as the contiguous nucleobase sequence) is selected from, or comprises, one of the following sequences: SEQ ID No 14, SEQ ID No 15, SEQ ID No 16, SEQ ID No 17, SEQ ID No 18 and SEQ ID No 19 or, in one embodiment a sub.sequence thereof, such as a sub.sequence of 10, 11, 12, 13, 14, 15 and 16 continuous (such as contiguous) nucleobases.

In one embodiment the contiguous nucleobase sequence is a contiguous nucleotide sequence present in a nucleic acid sequence selected from the group consisting of: SEQ ID NO 3, SEQ ID NO 4, SEQ ID NO 5, SEQ ID NO 6, SEQ ID NO 7, SEQ ID NO 8, SEQ ID 30, SEQ ID NO 31, SEQ ID NO 32, SEQ ID NO 33, SEQ ID NO 34, SEQ ID NO 35, SEQ ID NO 36, SEQ ID NO 37, SEQ ID NO 38, and SEQ ID NO 39, or, in one embodiment a sub.sequence thereof, such as a sub.sequence of 10, 11, 12, 13, 14, 15 and 16 continuous (such as contiguous) nucleobases.

In one embodiment the contiguous nucleobase or oligomer is selected from the group consisting of: SEQ ID NO 10, SEQ ID NO 20, SEQ ID NO 11, SEQ ID NO 9, SEQ ID NO 21, SEQ ID NO 22, SEQ ID NO 23, SEQ ID NO 24, SEQ ID NO 25, SEQ ID NO 26, SEQ ID NO 27, SEQ ID NO 28, and SEQ ID NO 29 or, in one embodiment a sub.sequence thereof, such as a sub.sequence of 10, 11, 12, 13, 14, 15 and 16 continuous (such as contiguous) nucleobases. In one embodiment the nucleobase portion is selected from, or comprises, one of the following sequences: SEQ ID NO 3, SEQ ID NO 4, SEQ ID NO 5, SEQ ID NO 6, SEQ ID NO 7, and SEQ ID NO 8, (preferably SEQ ID NO 3 and SEQ ID NO 4) or, in one embodiment a sub.sequence thereof, such as a sub.sequence of 10, 11, 12, 13, 14, 15 and 16 continuous (such as contiguous) nucleobases.

Other preferred oligonucleotides include sequences of 10, 11, 12, 13, 14, 15 and 16 continuous (such as contiguous) nucleobases selected from a sequence from the group consisting of SEQ ID NO 9, 10 and 11. Further preferred aspect of the invention is directed to compounds consisting or comprising of SEQ ID NO 9, 10 or 11.

It will be understood by the skilled person, that in one embodiment when referring to specific gapmer oligonucleotide sequences, such as those provided herein (e.g. SEQ ID NOS 9, 10 and 11) when the linkages are phosphorothioate linkages, alternative linkages, such as those disclosed herein may be used, for example phosphate linkages may be used, particularly for linkages between nucleotide analogues, such as LNA, units. Likewise, when referring to specific gapmer oligonucleotide sequences, such as those provided herein (e.g. SEQ ID NOS 9, 10 and 11), when the C residues are annotated as 5'methyl modified cytosine, in one embodiment, one or more of the Cs present in the oligonucleotide may be unmodified C residues.

In one embodiment the nucleobase sequence consists or comprises of a sequence which is, or corresponds to, a sequence selected from the group consisting of: SEQ ID NO 3, SEQ ID NO 4, SEQ ID NO 5, SEQ ID NO 6, SEQ ID NO 7 and SEQ ID NO 8, or a contiguous sequence of at least 12, 13, 14, 15, or 16 consecutive nucleobases present in said sequence, wherein the nucleotides present in the compound may be substituted with a corresponding nucleotide analogue and wherein said compound may comprise one, two, or three mismatches against said selected sequence.

In one embodiment the compound according to the invention consists or comprises of SEQ ID NO 3 or an equivalent nucleobase sequence.

In one embodiment the compound according to the invention consists or comprises of SEQ ID NO 4 or an equivalent nucleobase sequence.

In one embodiment the compound according to the invention consists or comprises of SEQ ID NO 5 or an equivalent nucleobase sequence.

In one embodiment the compound according to the invention consists or comprises of SEQ ID NO 6 or an equivalent nucleobase sequence.

In one embodiment the compound according to the invention consists or comprises of SEQ ID NO 7 or an equivalent nucleobase sequence.

In one embodiment the compound according to the invention consists or comprises of SEQ ID NO 8 or an equivalent nucleobase sequence.

In one embodiment the compound according to the invention consists or comprises of SEQ ID NO 9.

In one embodiment the compound according to the invention consists or comprises of SEQ ID NO 10.

In one embodiment the compound according to the invention consists or comprises of SEQ ID NO 11.

In one embodiment the compound according to the invention consists or comprises of SEQ ID NO 30, SEQ ID NO 31, SEQ ID NO 32, SEQ ID NO 33, SEQ ID NO 34, SEQ ID NO 35, SEQ ID NO 36, SEQ ID NO 37, SEQ ID NO 38, or SEQ ID NO 39.

Other oliogmers of the invention include sequences of 10, 11, 12, 13, 14, 15 and 16 continuous (such as contiguous) nucleobases selected from one of the above listed SEQ IDs or the compound IDs# as referred to in the examples.

Other oliogmers of the invention include sequences of 10, 11, 12, 13, 14, 15 and 16 continuous (such as contiguous) nucleobases selected from a sequence from the group consisting of SEQ ID No 14, SEQ ID No 15, SEQ ID No 16, SEQ ID No 17, SEQ ID No 18 and SEQ ID No 19, or a sequence selected from the group of (antisense) sequences shown in table 2 or table 3.

Other oligomers of the invention include sequences of 10, 11, 12, 13, 14, 15 and 16 continuous (such as contiguous) nucleobases selected from a sequence from the group consisting of SEQ ID NO 3, SEQ ID NO 4, SEQ ID NO 5, SEQ ID NO 6, SEQ ID NO 7, SEQ ID NO 8, SEQ ID NO 47, SEQ ID NO 49, SEQ ID NO 54, SEQ ID NO 56, SEQ ID NO 118, SEQ ID NO 136, and SEQ ID NO 139.

Preferred compounds consist of 10, 11, 12, 13, 14, 15 or 16 continuous (such as contiguous) nucleobases which correspond to a nucleotide sequence present in a sequence selected from the group consisting of SEQ ID NO 3, SEQ ID NO 4, SEQ ID NO 5, SEQ ID NO 6, SEQ ID NO 7, and SEQ ID NO 8, or preferably SEQ ID NO 3 and SEQ ID NO 4.

Further preferred compounds are shown in Tables 2 and table 3. of US 60/828,735 and table 4 of US 60/972,932, which are hereby specifically incorporated into this specification (as referred to in the specific list of embodiments listed herein).

Suitably, the oligomer according to the invention consists or comprises one of the above mentioned SEQ ID sequences.

### Complementarity and mismatches

The compound of the invention consists of a (contiguous) nucleobase sequence with is complementary to a corresponding (contiguous) region of the SEQ ID NO 2.

Referring to the principles by which the compound, can elicit its therapeutic action, the target of the present invention may be the mRNA derived from the corresponding sequence present in the nucleic acid which encodes the PCSK9 polypeptide, such as SEQ ID NO 2 or naturally occurring allelic variants thereof.

It will be recognised that when referring to a preferred nucleotide sequence motif or nucleotide sequence, which consists of only nucleotides, the compounds of the invention which are defined by that sequence may comprise a corresponding nucleotide analogues in place of one or more of the nucleotides present in said sequence, such as LNA units or other nucleotide analogues which raise the Tₘ of the oligonucleotide/target duplex - such as the nucleotide analogues described below, particularly LNA and/or 2' substited nucleotides (2' modified).

### Nucleotide Analogues

In one embodiment, at least one of the nucleobases present in the oligomeris a modified nucleobase selected from the group consisting of 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

It will be recognised that when referring to a preferred nucleotide sequence motif or nucleotide sequence, which consists of only nucleotides, the oligomers of the invention which are defined by that sequence may comprise a corresponding nucleotide analogues in place of one or more of the nucleotides present in said sequence, such as LNA units or other nucleotide analogues, which raise the duplex stability/Tₘ of the oligomer/target duplex *(i.e.* affinity enhancing nucleotide analogues).

Furthermore, the nucleotide analogues may enhance the stability of the oligomer *in vivo.*

Incorporation of affinity-enhancing nucleotide analogues in the oligomer nucleobase seqeunce, such as LNA or 2'-substituted sugars, preferably LNA, can allow the size of the specifically binding oligonucleotide to be reduced, and may also reduce the upper limit to the size of the oligonucleotide before non-specific or aberrant binding takes place. An affinity enhancing nucleotide analogue is one which, when inserted into the nucleobase sequence of the oligomer results in a increased Tₘ of the oligomer when formed in a duplex with a complementary RNA (such as the mRNA target), as compared to an equivalent oligomer which comprises a DNA nucleotide in place of the affinity enhancing nucleotide analogue Examples of suitable and preferred nucleotide analogues are provided by WO2007/031091 or are referenced therein.

In some embodiments at least one of said nucleotide analogues is 2'-MOE-RNA, such as 2, 3, 4, 5, 6, 7 or 8 2'-MOE-RNA nucleobase units.

In some embodiments at least one of said nucleotide analogues is 2'-fluoro DNA, such as 2, 3, 4, 5, 6, 7 or 8 2'-fluoro-DNA nucleobase units.

Specific examples of nucleoside analogues which may be utilised in the oligomers of the present invention are described by e.g. Freier & Altmann; Nucl. Acid Res., 1997, 25, 4429-4443 and Uhlmann; Curr. Opinion in Drug Development, 2000, 3(2), 293-213, and in Scheme 1: and 2.

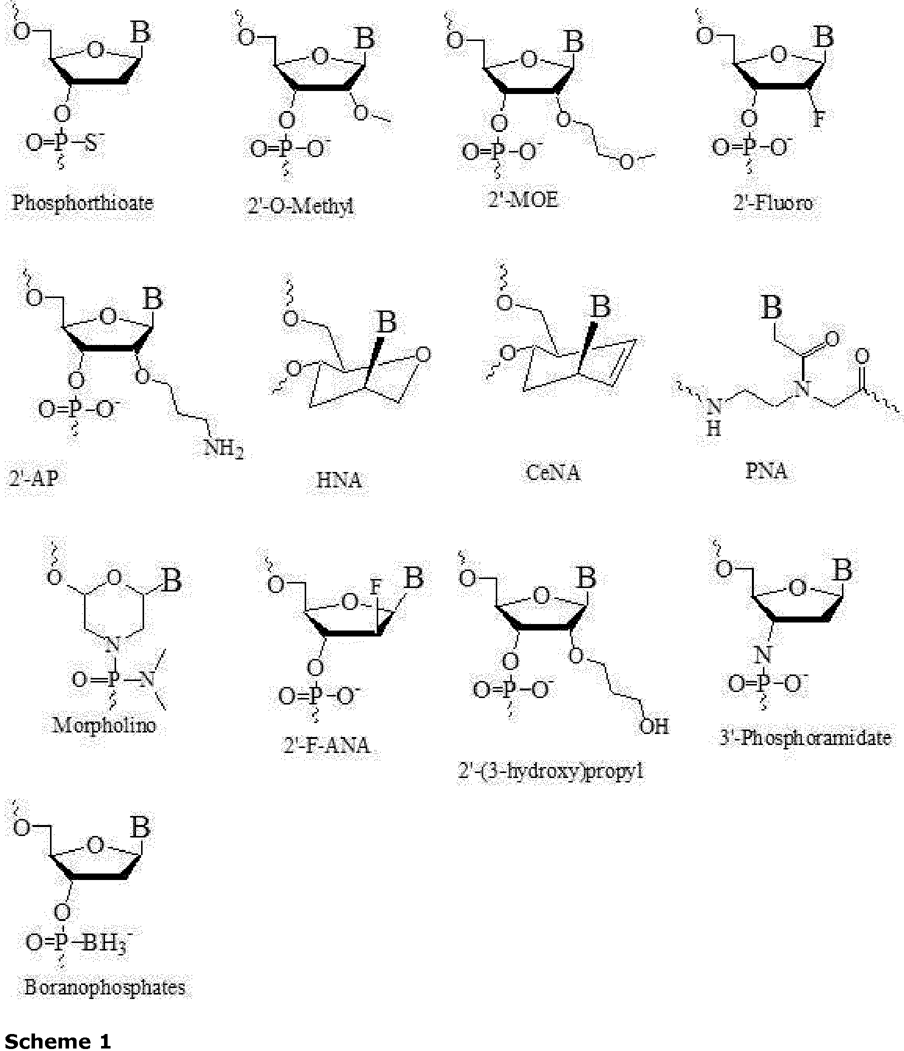

The term "LNA" refers to a bicyclic nucleotide analogue, known as "Locked Nucleic Acid". It may refer to an LNA monomer, or, when used in the context of an "ULNA oligonucleotide" refers to an oligonucleotide containing one or more such bicyclic nucleotide analogues. The LNA used in the oligonucleotide compounds of the invention preferably has the structure of the general formula 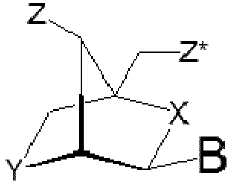 where X and Y are independently selected among the groups -O-,
-S-, -N(H)-, N(R)-, -CH₂- or -CH- (if part of a double bond), -CH₂-O-, -CH₂-S-, -CH₂-N(H)-, -CH₂-N(R)-, -CH₂-CH₂- or -CH₂-CH- (if part of a double bond), -CH=CH-, where R is selected from hydrogen and C₁₋₄-alkyl; Z and Z* are independently selected among an internucleoside linkage, a terminal group or a protecting group; B constitutes a natural or non-natural nucleotide base moiety; and the asymmetric groups may be found in either orientation.

Preferably, the LNA used in the oligomer of the invention comprises at least one LNA unit according any of the formulas 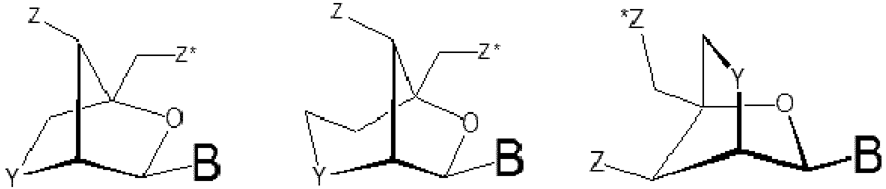 wherein Y is -O-, -S-, -NH-, or N(R^{H}); Z and Z* are independently selected among an internucleoside linkage, a terminal group or a protecting group; B constitutes a natural or non-natural nucleotide base moiety, and R^{H} is selected from hydrogen and C₁₋₄-alkyl.

Preferably, the Locked Nucleic Acid (LNA) used in the oligomeric compound, such as an antisense oligonucleotide, of the invention comprises at least one nucleotide comprises a Locked Nucleic Acid (LNA) unit according any of the formulas shown in Scheme 2 of WO2007/031091.

Preferably, the LNA used in the oligomer of the invention comprises internucleoside linkages selected from -O-P(O)₂-O-, -O-P(O,S)-O-, -O-P(S)₂-O-, -S-P(O)₂-O-, -S-P(O,S)-O-, -S-P(S)₂-O-, -O-P(O)₂-S-, -O-P(O,S)-S-, -S-P(O)₂-S-, -O-PO(R^{H})-O-, O-PO(OCH₃)-O-, -O-PO(NR^{H})-O-, -O-PO(OCH₂CH₂S-R)-O-, -O-PO(BH₃)-O-, -O-PO(NHR^{H})-O-, -O-P(O)₂-NR^{H}-, -NR^{H}-P(O)₂-O-, -NR^{H}-CO-O-, where R^{H} is selected form hydrogen and C₁₋₄-alkyl.

Specifically preferred LNA units are shown in scheme 2:

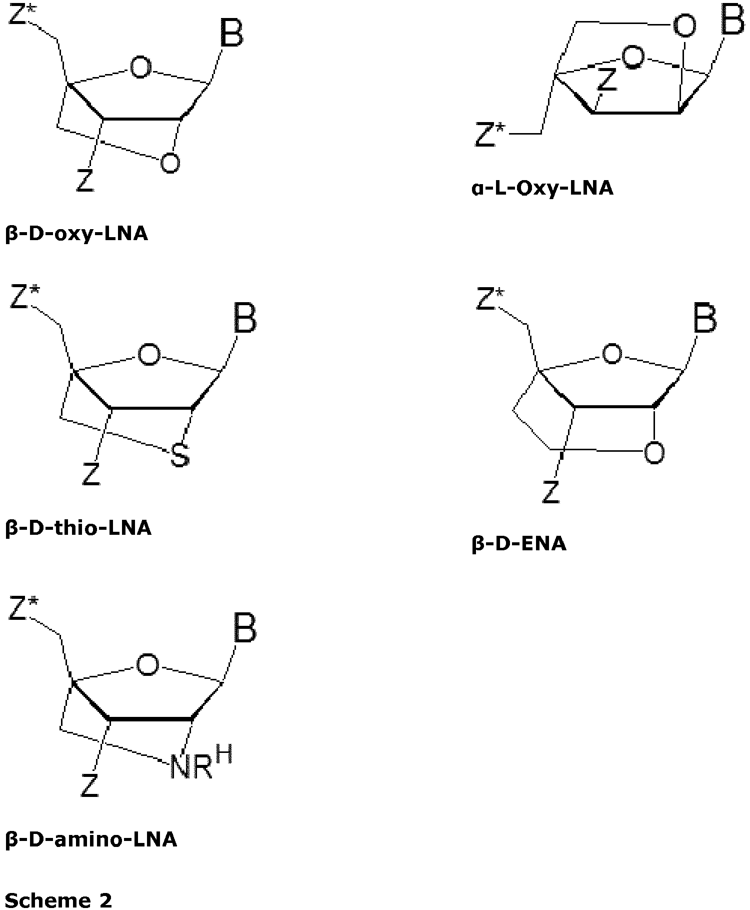

The term "thio-LNA" comprises a locked nucleotide in which at least one of X or Y in the general formula above is selected from S or -CH₂-S-. Thio-LNA can be in both beta-D and alpha-L-configuration.

The term "amino-LNA" comprises a locked nucleotide in which at least one of X or Y in the general formula above is selected from -N(H)-, N(R)-, CH₂-N(H)-, and -CH₂-N(R)- where R is selected from hydrogen and C₁₋₄-alkyl. Amino-LNA can be in both beta-D and alpha-L-configuration.

The term "oxy-LNA" comprises a locked nucleotide in which at least one of X or Y in the general formula above represents -O- or -CH₂-O-. Oxy-LNA can be in both beta-D and alpha-L-configuration.

The term "ena-LNA" comprises a locked nucleotide in which Y in the general formula above is -CH₂-O- (where the oxygen atom of -CH₂-O- is attached to the 2'-position relative to the base B).

In a preferred embodiment LNA is selected from beta-D-oxy-LNA, alpha-L-oxy-LNA, beta-D-amino-LNA and beta-D-thio-LNA, in particular beta-D-oxy-LNA.

Preferably, within the compound according to the invention, such as an antisense oligonucleotide, which comprises LNA, all LNA C residues are 5'methyl-Cytosine.

Preferably the LNA units of the compound, such as an antisense oligonucleotide, of the invention are selected from one or more of the following: thio-LNA, amino-LNA, oxy-LNA, ena-LNA and/or alpha-LNA in either the D-beta or L-alpha configurations or combinations thereof. Beta-D-oxy-LNA is a preferred LNA for use in the oligomeric compounds of the invention. Thio-LNA may also be preferred for use in the oligomeric compounds of the invention. Amino-LNA may also be preferred for use in the oligomeric compounds of the invention. Oxy-LNA may also be preferred for use in the oligomeric compounds of the invention. Ena-LNA may also be preferred for use in the oligomeric compounds of the invention. Alpha-LNA may also be preferred for use in the oligomeric compounds of the invention.

The Locked Nucleic Acid (LNA) used in the compound, such as an antisense oligonucleotide, of the invention has the structure of the general formula shown in scheme 1 of WO2007/031091. The terms "thio-LNA", "amino-LNA", "oxy-LNA", "ena-LNA", "alpha-L-LNA", "LNA derivatives", "locked nucleotide" and "locked nucleobase" are also used as defined in WO2007/031091.

Suitably, when the nucleobase sequence of the oligomer, or the contiguous nucleobase sequence, is not fully complementary to the corresponding region of the PCSK9 target sequence, in one embodiment, when the oligomer comprises affinity enhancing nucleotide analogues, such nucleotide analogues form a complement with their corresponding nucleotide in the PCSK9 target.

The oligomer may thus comprise or consist of a simple sequence of natural nucleotides - preferably 2'-deoxynucleotides (referred to here generally as "DNA"), but also possibly ribonucleotides (referred to here generally as "RNA") - or it could comprise one or more (and possibly consist completely of) nucleotide "analogues".

Nucleotide "analogues" are variants of natural DNA or RNA nucleotides by virtue of modifications in the sugar and/or base and/or phosphate portions. The term "nucleobase" will be used to encompass natural (DNA- or RNA-type) nucleotides as well as such "analogues" thereof. Analogues could in principle be merely "silent" or "equivalent" to the natural nucleotides in the context of the oligonucleotide, *i.e.* have no functional effect on the way the oligonucleotide works to PCSK9 expression. Such "equivalent" analogues may nevertheless be useful if, for example, they are easier or cheaper to manufacture, or are more stable to storage or manufacturing conditions, or represent a tag or label. Preferably, however, the analogues will have a functional effect on the way in which the oligomer works to inhibit expression; for example by producing increased binding affinity to the target and/or increased resistance to intracellular nucleases and/or increased ease of transport into the cell.

Examples of such modification of the nucleotide include modifying the sugar moiety to provide a 2'-substituent group or to produce a bridged (locked nucleic acid) structure which enhances binding affinity and probably also provides some increased nuclease resistance; modifying the internucleotide linkage from its normal phosphodiester to one that is more resistant to nuclease attack, such as phosphorothioate or boranophosphate - these two, being cleavable by RNase H, also allow that route of antisense inhibition in modulating the PCSK9 expression.

In some embodiments, the oligomer comprises from 3-8 nucleotide analogues, e.g. 6 or 7 nucleotide analogues. In the by far most preferred embodiments, at least one of said nucleotide analogues is a locked nucleic acid (LNA); for example at least 3 or at least 4, or at least 5, or at least 6, or at least 7, or 8, of the nucleotide analogues may be LNA. In some embodiments all the nucleotides analogues may be LNA.

In some embodiments the nucleotide analogues present within the oligomer of the invention in regions A and C mentioned herein are independently selected from, for example: 2'-O-alkyl-RNA units, 2'-amino-DNA units, 2'-fluoro-DNA units, LNA units, arabino nucleic acid (ANA) units, 2'-fluoro-ANA units, HNA units, INA (intercalating nucleic acid) units and 2'MOE units. It is also considered that the nucleotide analogues present in an oligomer of the invention are all the same, all be it, allowing for base variation.

2'-O-methoxyethyl-RNA (2'MOE), 2'-fluoro-DNA monomers and LNA are preferred nucleotide analogues, and as such the oligonucleotide of the invention may comprise nucleotide analogues which are independently selected from these three types of analogue, or may comprise only one type of analogue selected from the three types.

Compounds according to the invention, are, in one embodiment, those consisting or comprising a sequence selected from SEQ ID NO 3, SEQ ID NO 4, SEQ ID NO 5, SEQ ID NO 6, SEQ ID NO 7, and SEQ ID NO 8, or preferably SEQ ID NO 3 or SEQ ID NO 4.wherein, in one embodiment the nucleotides present in the compound may be substituted with a corresponding nucleotide analogue.

Preferred compounds according to the invention are those consisting or comprising of SEQ ID NOS 3 or 4, wherein they contain at least one nucleic acid analogue, wherein in one embodiment, the LNA units may be substituted with an alternative corresponding nucleotide analogue.

Nucleotide analogues which increase the Tₘ of the oligonucleotide/target nucleic acid target, as compared to the equivalent nucleotide are preferred.

Preferably, the compound according to the invention comprises at least one nucleotide analogue, such as Locked Nucleic Acid (LNA) unit, such as 4, 5, 6, 7, 8, 9, or 10 nucleotide analogues, such as Locked Nucleic Acid (LNA) units, preferably between 4 to 9 nucleotide analogues, such as LNA units, such as 6-9 nucleotide analogues, such as LNA units, most preferably 6, 7 or 8 nucleotide analogues, such as LNA units.

The term LNA is used as defined in PCT application WO2007/031091.

Preferably the LNA units comprise at least one beta-D-oxy-LNA unit(s) such as 2, 3, 4, 5, 6, 7, 8, 9, or 10 beta-D-oxy-LNA units. The compound of the invention, such as the antisense oligonucleotide, may comprise more than one type of LNA unit. Suitably, the compound may comprise both beta-D-oxy-LNA, and one or more of the following LNA units: thio-LNA, amino-LNA, oxy-LNA, ena-LNA and/or alpha-LNA in either the D-beta or L-alpha configurations or combinations thereof.

Preferably, the compound, such as an antisense oligonucleotide, may comprise both nucleotide analogues, such as LNA units, and DNA units. Preferably the combined total of nucleobases, such as, LNA and DNA units, is between 10 - 20, such as 14-20, such as between 15-18, such as 15, 16 or 17 nucleobase units, or is a shortmer as referred to herein. Preferably the ratio of nucleotide analogues to DNA present in the oligomeric compound of the invention is between 0.3 and 1, more preferably between 0.4 and 0.9, such as between 0.5 and 0.8.

Preferably, the compound of the invention, such as an antisense oligonucleotide, consists of a total of 10 - 25, or 12-25 nucleotides and/or nucleotide analogues, wherein said compound comprises a subsequence of at least 8 nucleotides or nucleotide analogues, said subsequence being located within *(i.e.* corresponding to) a sequence selected from the group consisting of SEQ ID No 14, SEQ ID No 15, SEQ ID No 16, SEQ ID No 17, SEQ ID No 18 and SEQ ID No 19.

In one aspect of the invention, the nucleotides (and/or nucleotide analogues) are linked to each other by means of a phosphorothioate group. An interesting embodiment of the invention is directed to compounds selected from the group consisting of SEQ ID NO 3, SEQ ID NO 4, SEQ ID NO 5, SEQ ID NO 6, SEQ ID NO 7, and SEQ ID NO 8, wherein each linkage group within each compound is a phosphorothioate group. Such modifications are denoted by the subscript S.

The tables referred to herein provide further nucleobase sequences of compounds of the invention.

In further embodiments, the compound of the invention, such as the antisense oligonucleotide of the invention may comprises or consist of 13, 14, 15, 16, 17, 18, 19, 20 or 21 nucleobases.

Preferably the compound according to the invention, such as an antisense oligonucleotide, comprises or consists of 2, 3, 4, 5, 6, 7, 8, 9, 10, 11, 12, 13, 14 or 15 nucleotide analogues, such as LNA units, in particular 4, 5, 6, 7, 8, 9 or 10 nucleotide analogues, such as LNA units, such as between 1 and 10 nucleotide analogues, such as LNA units such as between 2 and 8 nucleotide analogues such as LNA units.

### RNAseH recruitment

It is preferable that said subsequence or combined nucleobase sequence comprises a continuous (contiguous) sequence of at least 7 nucleobase residues, such as at least 8 or at least 9 nucleobase residues, including 7, 8 or 9 nucleobases, which, when formed in a duplex with the complementary target RNA corresponding to each of said polynucleotides which encode said mammalian PCSK9 are capable of recruiting RNaseH, such as DNA nucleotides.

The size of the contiguous sequence which is capable of recruiting RNAseH may be higher, such as 10, 11, 12, 13, 14, 15, 16, 17, 18 , 19 or 20 nucleobase units.

The contiguous sequence which is capable of recruiting RNAseH may be region B as referred to in the context of a gapmer as described herein.

EP 1 222 309 provides *in vitro* methods for determining RNaseH activity, which may be used to determine the ability to recruit RNaseH. A compound is deemed capable of recruiting RNase H if, when provided with the complementary RNA target, it has an initial rate, as measured in pmol/l/min, of at least 1 %, such as at least 5%, such as at least 10% or less than 20% of the equivalent DNA only oligonucleotide, with no 2' substitutions, with phosphorothioate linkage groups between all nucleotides in the oligonucleotide, using the methodology provided by Example 91 - 95 of EP 1 222 309.

A compound is deemed essentially incapable of recruiting RNaseH if, when provided with the complementary RNA target, and RNaseH, the RNaseH initial rate, as measured in pmol/l/min, is less than 1%, such as less than 5%,such as less than 10% or less than 20% of the initial rate determined using the equivalent DNA only oligonucleotide, with no 2' substitutions, with phosphiothioate linkage groups between all nucleotides in the oligonucleotide, using the methodology provided by Example 91 - 95 of EP 1 222 309.

However, it is also recognised that antisense oligonucleotides may function via non RNaseH mediated degradation of target mRNA, such as by steric hindrance of translation, or other methods.

The compound of the invention may comprise a nucleobase sequence which comprises both nucleotides and nucleotide analogues, and may be in the form of a gapmer, a headmer or a mixmer.

A headmer is defined by a contiguous stretch of nucleotide analogues at the 5'-end followed by a contiguous stretch of DNA or modified nucleobases units recognizable and cleavable by the RNaseH towards the 3'-end (such as at least 7 such nucleobases), and a tailmer is defined by a contiguous stretch of DNA or modified monomers recognizable and cleavable by the RNaseH at the 5'-end (such as at least 7 such nucleobases), followed by a contiguous stretch of nucleotide analogues towards the 3'-end. Other chimeras according to the invention, called mixmers consisting of an alternate composition of DNA or modified monomers recognizable and cleavable by RNaseH and nucleotide analogues. Some nucleotide analogues may also be able to mediate RNaseH binding and cleavage. Since α-L-LNA recruits RNaseH activity to a certain extent, smaller gaps of DNA or modified monomers recognizable and cleavable by the RNaseH for the gapmer construct might be required, and more flexibility in the mixmer construction might be introduced.

### Gapmers

Preferably, the compound of the invention is an antisense oligonucleotide which is a gapmer.

Preferably the gapmer comprises a (poly)nucleobase sequence of formula (5' to 3'), A-B-C (and optionally D), wherein; A (5' region) consists or comprises of at least one nucleotide analogue, such as at least one LNA unit, such as between 1-6 nucleotide analogues, such as LNA units, preferably between 2-5 nucleotide analogues, such as 2-5 LNA units, such as 3 or 4 nucleotide analogues, such as 3 or 4 LNA units and; B (central domain), preferably immediately 3' *(i.e.* contiguous) to A, consists or comprises at least one DNA sugar unit, such as 1-12 DNA units, preferably between 4-12 DNA units, more preferably between 6-10 DNA units, such as between 7-10 DNA units, most preferably 8, 9 or 10 DNA units, and; C(3' region) preferably immediately 3' to B, consists or comprises at of at least one nucleotide analogues, such as at least one LNA unit, such as between 1-6 nucleotide analogues, such between 2-5 nucleotide analogues, such as between 2-5 LNA units, most preferably 3 or 4 nucleotide analogues, such as 3 or 4 LNA units. Preferred gapmer designs are disclosed in WO2004/046160.

Preferred gapmer designs include, when:
A Consists of 3 or 4 consecutive nucleotide analogues
B Consists of 7 to 10 consecutive DNA nucleotides or equivalent nucleobases which are capable of recruiting RNAseH
C Consists of 3 or 4 consecutive nucleotide analogues
D Consists, where present, of one DNA nucleotide.

Or when
A Consists of 3 consecutive nucleotide analogues
B Consists of 9 consecutive DNA nucleotides or equivalent nucleobases which are capable of recruiting RNAseH
C Consists of 3 consecutive nucleotide analogues
D Consists, where present, of one DNA nucleotide.

Or when
A Consists of 4 consecutive nucleotide analogues
B Consists of 8 consecutive DNA nucleotides or equivalent nucleobases which are capable of recruiting RNAseH
C Consists of 4 consecutive nucleotide analogues
D Consists, where present, of one DNA nucleotide.

Or when
A Consists of 2 consecutive nucleotide analogues
B Consists of 8 consecutive DNA nucleotides or equivalent nucleobases which are capable of recruiting RNAseH
C Consists of 3 consecutive nucleotide analogues
D Consists, where present, of one DNA nucleotide.

Or when
A Consists of 3 consecutive nucleotide analogues
B Consists of 8 consecutive DNA nucleotides or equivalent nucleobases which are capable of recruiting RNAseH
C Consists of 2 consecutive nucleotide analogues
D Consists, where present, of one DNA nucleotide.

Or when
A Consists of 2 consecutive nucleotide analogues
B Consists of 8 consecutive DNA nucleotides or equivalent nucleobases which are capable of recruiting RNAseH
C Consists of 2 consecutive nucleotide analogues
D Consists, where present, of one DNA nucleotide.

The DNA nucleotides in the central domain (B) may be substituted with one or more, or even all the DNA nucleotides may be substituted with a nucleobase, including nucleotide analogues which are capable or recruiting RNAse H.

In the above embodiments referring to gapmer designs, the gap region 'B' may alternatively be 7, 8, 9 or 10 consecutive DNA nucleotides or equivalent nucleobases which are capable of recruiting RNAseH.

In a gapmer oligonucleotide, it is highly preferable that any mismatches are not within the central domain (B) above, are at least within a minimum stretch of 7 continuous nucleobases of the central domain, such as 7, 8 or 9 or 10 continuous nucleobases, which preferably comprises or consists of DNA units.

In a gapmer oligonucleotide, it is preferred that any mismatches are located towards the 5' or 3' termini of the gapmer. Therefore, it is preferred that in a gapmer oligonucleotide which comprises mismatches with the target mRNA, that such mismatches are located either in 5' (A) and/or 3' (C) regions, and/or said mismatches are between the 5' or 3' nucleotide unit of said gapmer oligonucleotide and target molecule.

Preferably, the gapmer, of formula A-B-C, further comprises a further region, D, which consists or comprises, preferably consists, of one or more DNA sugar residue terminal of the 3' region (C) of the oligomeric compound, such as between one and three DNA sugar residues, including between 1 and 2 DNA sugar residues, most preferably 1 DNA sugar residue.

### Shortmers

US provisional application, 60/977409, refers to 'shortmer' oligonucleotides, which, in one embodiment particularly, are preferred oligomeric compounds according to the present invention

In one embodiment oligomer consisting of a contiguous nucleobase sequence of a total of 10, 11, 12, 13 or 14 nucleobase units, wherein the contiguous nucleobase sequence is of formula (5' - 3'), A-B-C, or optionally A-B-C-D. wherein: A consists of 1, 2 or 3 LNA units; B consists of 7, 8 or 9 contiguous nucleobase units which are capable of recruiting RNAseH when formed in a duplex with a complementary RNA molecule (such as a mRNA target); and C consists of 1, 2 or 3 LNA units. When present, D consists of a single DNA unit. In one embodiment, there is no region D. In one embodiment A consists of 1 LNA unit. In one embodiment A consists of 2 LNA units. In one embodiment A consists of 3 LNA units. In one embodiment C consists of 1 LNA unit. In one embodiment C consists of 2 LNA units. In one embodiment C consists of 3 LNA units. In one embodiment B consists of 7 nucleobase units. In one embodiment B consists of 8 nucleobase units. In one embodiment B consists of 9 nucleobase units. In one embodiment B comprises of between 1 - 9 DNA units, such as 2, 3, 4, 5, 6, 7 or 8 DNA units. In one embodiment B consists of DNA units. In one embodiment B comprises of at least one LNA unit which is in the alpha-L configuration, such as 2, 3, 4, 5, 6, 7, 8 or 9 LNA units in the alpha-L-configuration. In one embodiment B comprises of at least one alpha-L-oxy LNA unit or wherein all the LNA units in the alpha-L- configuration are alpha-L-oxy LNA units. In one embodiment the number of nucleobases in A-B-C are selected from the group consisting of: 1-8-2, 2-8-1, 2-8-2, 3-8-3, 2-8-3, 3-8-2. In one embodiment the number of nucleobases in A-B-C are selected from the group consisting of: 1-9-1, 1-9-2, 2-9-1, 2-9-2, 3-9-2, and 2-9-3. In one embodiment the number of nucleobases in A-B-C are selected from the group consisting of: 2-7-1, 1-7-2, 2-7-2, 3-7-3, 2-7-3, 3-7-2, 3-7-4, and 4-7-3. In one embodiment both A and C both consist of two LNA units each, and B consists of 8 nucleobase units, preferably DNA units. In one embodiment the LNA units of A and C are independently selected from oxy-LNA, thio-LNA, and amino-LNA, in either of the beta-D and alpha-L configurations or combinations thereof. In one embodiment the LNA units of A and C are beta-D-oxy-LNA. In one embodiment the internucleoside linkages are independently selected from the group consisting of: phosphodiester, phosphorothioate and boranophosphate. In one embodiment the oligomer comprises at least one phosphorothioate internucleoside linkage. In one embodiment the internucleoside linkages adjacent to or between DNA units are phosphorothioate linkages. In one embodiment the linkages between at least one pair of consecutive LNA units, such as 2 LNA units in region A or C, is a phosphodiester linkage. In one embodiment the linkages between consecutive LNA units such as 2 LNA units in region A and C, are phosphodiester linkages. In one embodiment the all the internucleoside linkages are phosphorothioate linkages.

Suitable internucleoside linkages include those listed within WO2007/031091 , for example the internucleoside linkages listed on the first paragraph of page 34 of WO2007/031091.

Suitable sulphur (S) containing internucleoside linkages as provided above may be preferred. Phosphorothioate internucleotide linkages are also preferred, particularly for the gap region (B) of gapmers. Phosphorothioate linkages may also be used for the flanking regions (A and C, and for linking C to D, and D).

Regions A, B and C, may however comprise internucleoside linkages other than phosphorothioate, such as phosphodiester linkages, particularly, for instance when the use of nucleotide analogues protects the internucleoside linkages within regions A and C from endo-nuclease degradation - such as when regions A and C comprise LNA nucleobases.

The internucleobase linkages in the oligomer may be phosphodiester, phosphorothioate or boranophosphate so as to allow RNase H cleavage of targeted RNA. Phosphorothioate is preferred, for improved nuclease resistance and other reasons, such as ease of manufacture.

In one aspect of the oligomer of the invention, the nucleobases (nucleotides and/or nucleotide analogues) are linked to each other by means of phosphorothioate groups.

In some embodiments region A comprises at least one phosphodiester linkage between two nucleotide analogue units, or a nucleotide analogue unit and a nucleobase unit of Region B. In some embodiments region C comprises at least one phosphodiester linkage between two nucleotide analogue units, or a nucleotide analogue unit and a nucleobase unit of Region B.

In some embodiments, region C comprises at least one phosphodiester linkage between a nucleotide analogue unit and a nucleobase unit of Region D.

In some embodiments the internucleobase linkage between the 3' nucleotide analogue of region A and the 5' nucleobase of region B is a phosphodiester.

In some embodiments the internucleobase linkage between the 3' nucleobase of region B and the 5' nucleotide analogue of region C is a phosphodiester.

In some embodiments the internucleobase linkage between the two adjacent nucleotide analogues at the 5' end of region A are phosphodiester.

In some embodiments the internucleobase linkage between the two adjacent nucleotide analogues at the 3' end of region C is phosphodiester.

In some embodiments the internucleobase linkage between the two adjacent nucleotide analogues at the 3' end of region A is phosphodiester.

In some embodiments the internucleobase linkage between the two adjacent nucleotide analogues at the 5' end of region C is phosphodiester.

In some embodiments region A has a length of 4 nucleotide analogues and the internucleobase linkage between the two middle nucleotide analogues of region A is phosphodiester.

In some embodiments region C has a length of 4 nucleotide analogues and internucleobase linkage between the two middle nucleotide analogues of region C is phosphodiester.

In some embodiments all the internucleobase linkages between nucleotide analogues present in the compound of the invention are phosphodiester.

In some embodiments, such as the embodiments referred to above, where suitable and not specifically indicated, all remaining internucleobase linkages are either phosphodiester or phosphorothioate, or a mixture thereof.

In some embodiments all the internucleobase linkage groups are phosphorothioate.

When referring to specific gapmer oligonucleotide sequences, such as those provided herein it will be understood that, in one embodiment, when the linkages are phosphorothioate linkages, alternative linkages, such as those disclosed herein may be used, for example phosphate (phosphodiester) linkages may be used, particularly for linkages between nucleotide analogues, such as LNA, units. Likewise, when referring to specific gapmer oligonucleotide sequences, such as those provided herein, when the C residues are annotated as 5'methyl modified cytosine, in one embodiment, one or more of the Cs present in the oligonucleotide may be unmodified C residues.

### Method of identification and preparation of compounds of the invention:

The compounds of the invention, which modulate expression of the target, may be identified through experimentation or though rational design based on sequence information on the target and know-how on how best to design an oligomeric compound against a desired target. The sequences of these compounds are preferred embodiments of the invention. Likewise, the sequence motifs in the target to which these preferred oligomeric compounds are complementary (referred to as "hot spots") are preferred sites for targeting.

In many cases the identification of an oligomeric compound, such as an LNA oligonucleotide, effective in modulating PCSK9 expression or activity *in vivo* or clinically is based on sequence information on the target gene (such as SEQ ID NO 2). However, one of ordinary skill in the art will appreciate that such oligomeric compounds can also be identified by empirical testing. oligomeric compounds having, for example, less sequence homology, greater or fewer modified nucleotides, or longer or shorter lengths, compared to those of the preferred embodiments, but which nevertheless demonstrate responses in clinical treatments, are also within the scope of the invention. The Examples provide suitable methods for performing empirical testing.

In a preferred embodiment, the compound of the invention comprises a subsequence or a combined contiguous nucleobase sequence which has at least 10, such as at least 11, such as at least 12, such as at least 14, such as at least 16, such as at least 18, such as 12, 13, 14, 15, 16, 17 or 18 contiguous nucleobases which are 100% complementary to both the human and mouse, or both the human and rat, or both the human and monkey, or both the human, mouse and monkey, or both the human, rat and monkey nucleic acids that encode PCSK9. In one embodiment the polynucleobase sequence of the compound is 100% complementary to both the human and mouse, or both the human and rat, or both the human and monkey, or both the human, mouse and monkey, or both the human, rat and monkey nucleic acids that encode PCSK9. In one embodiment, when referring to compounds of the invention that are 100% complementary to more than one mammalian species as listed above, one or two mismatches between 1 or more of the sequence may exist, although it is preferred that there are no mismtaches. Figure 17 illustrates an alignment between the human and mouse nucleic acids that encode the respective human and mouse PCSK9 polypeptides. Table 1 provides suitable PCSK9 polynucleotides and the corresponding polypeptides provided by the NCBI Genbank Accession numbers - certain known allelic variants and known homologues from other mammalian species may be easily identified by performing BLAST searches using the sequences referenced in Table 1.

**Table 1**

| | Nucleic acid (mRNA/cDNA sequence) | Polypeptide (deduced) |
|---|---|---|
| Human | NM_174936 | NP_777596 |
| Mouse | NM_153565 | NP_705793.1 |
| Rat | NM_199253 | NP_954862.2 |
| Chimpanzee | NC_006468 (genomic - mRNA annotated) | XP_001154126 |
| Monkey (*Rhesus macaque*) | BV166576 | |

Amino acid and polynucleotide homology may be determined using ClustalW algorithm using standard settings: see http://www.ebi.ac.uk/emboss/align/index.html, Method: EMBOSS::water (local): Gap Open = 10.0, Gap extend = 0.5, using Blosum 62 (protein), or DNAfull for nucleotide sequences. As illustrated in Figure 17, such alignments can also be used to identify regions of the nucleic acids encoding PCSK9 from human and a different mammalian species, such as monkey, mouse and/or rat, where there are sufficient stretches of nucleic acid complementarity to allow the design of oligonucleotides which target both the human PCSK9 target nucleic acid, and the corresponding nucleic acids present in the different mammalian species, such as regions of at least 10, such as at least 12, such as at least 14, such as at least 16, such as at least 18, such as 12, 13, 14, 15, 16, 17 or 18 contiguous nucleobases which are 100% complementary to both the nucleic acid encoding PCSK9 from humans and the nucleic acid(s) encoding PCSK9 from the different mammalian species.

### Defintions

When determining "homology" between the oligomeric compounds of the invention (or sub-sequence or combined contiguous nucleobase sequence) and the nucleic acid which encodes the mammalian PCSK9, such as those disclosed herein (including SEQ ID No 2), the determination of homology may be made by a simple alignment with the corresponding nucleobase sequence of the compound of the invention and the corresponding region of the nucleic acid which encodes the mammalian PCSK9 (or target nucleic acid), and the homology is determined by counting the number of bases which align and dividing by the total number of contiguous bases in the compound of the invention, and multiplying by 100. In such a comparison, if gaps exist, it is preferable that such gaps are merely mismatches rather than areas where the number of nucleobases within the gap differ between the nucleobase sequence of the invention and the target nucleic acid.

The terms "located within" and "corresponding to"/ "corresponds to" refer to the comparison between the nucleobase sequence of the oligomer or contiguous nucleobase seqeunce and the equivalent nucleotide sequence of either the nucleic acid target such as the mRNA which encodes the PCSK9 target protein, such as SEQ ID NO 2, or the reverse complement of the nucleic acid target. Nucleotide analogues are compared directly to their equivalent or corresponding nucleotides.

The terms "corresponding nucleotide analogue" and "corresponding nucleotide" are intended to indicate that the nucleobase in the nucleotide analogue and the nucleotide are identical. For example, when the 2-deoxyribose unit of the nucleotide is linked to an adenine, the "corresponding nucleotide analogue" contains a pentose unit (different from 2-deoxyribose) linked to an adenine.

The term "continuous" in relation to a sequence of nucleobases, is interchangeable with the term "continuous".

The term "nucleobase" is used as a collective term which encompasses both nucleotides and nucleotide analogues. A nucleobase sequence is a sequence which comprises at least two nucleotides or nucleotide analogues. In one embodiment the nucleobase sequence may comprise of only nucleotides, such as DNA units, in an alternative embodiment, the nucleobase sequence may comprise of only nucleotide analogues, such as LNA units.

The term "nucleic acid" is defined as a molecule formed by covalent linkage of two or more nucleotides.

The terms "nucleic acid" and "polynucleotide" are used interchangeable herein.

The following terms are used as they are defined in WO2007/031091: "nucleotide", "nucleotide analogue", "located within", "corresponding to"/ "corresponds to", "corresponding nucleotide analogue" and "corresponding nucleotide", "nucleobase", "nucleic acid" and "polynucleotide", "compound" when used in the context of a "compound of the invention", "oligomeric compound", "oligonucleotide", "antisense oligonucleotide", and "oligo", "unit", "LNA" , "at least one", "linkage group", "conjugate", "pharmaceutically acceptable salts", "C₁₋₄-alkyl", "gene", "RNA antagonist", "mRNA", "complementary", "mismatche(s)",

The term "target nucleic acid", as used herein refers to the DNA encoding human PCSK9 polypeptide, and RNA nucleic acids derived therefrom, preferably mRNA, such as pre-mRNA, although preferably mature mRNA. In one embodiment, for example when used in research or diagnostics the "target nucleic acid" may be a cDNA or a synthetic oligonucleotide derived from the above DNA or RNA nucleic acid targets. The oligomeric compound according to the invention is preferably capable of hybridising to the target nucleic acid.

The term "naturally occurring variant thereof" refers to variants of the PCSK9 polypeptide of nucleic acid sequence which exist naturally within the defined taxonomic group, such as mammalian, such as mouse, rat, monkey, chimpanzee and preferably human. Typically, when referring to "naturally occurring variants" of a polynucleotide the term also may encompasses variants of the PCSK9 encoding genomic DNA which are found at the NARC1 locus, or a locus directly derived from the NARC-1 locus, e.g. by chromosomal translocation or duplication, and the RNA, such as mRNA derived therefrom. When referenced to a specific polypeptide sequence, e.g. SEQ ID NO 1, the term also includes naturally occurring forms of the protein which may therefore be processed, e.g. by co- or post-translational modifications, such as signal peptide cleavage, proteolytic cleavage, glycosylation, etc.

It is preferred that the compound according to the invention is a linear molecule or is synthesised as a linear molecule.

The term "linkage group" is intended to mean a group capable of covalently coupling together two nucleotides, two nucleotide analogues, and a nucleotide and a nucleotide analogue, etc. Specific and preferred examples include phosphate groups and phosphorothioate groups.

In the present context the term "conjugate" is intended to indicate a heterogenous molecule formed by the covalent attachment of a compound as described herein (i.e. a compound comprising a sequence of nucleotides analogues) to one or more non-nucleotide/non-nucleotide-analogue,or non-polynucleotide moieties. Examples of non-nucleotide or non-polynucleotide moieties include macromolecular agents such as proteins, fatty acid chains, sugar residues, glycoproteins, polymers, or combinations thereof. Typically proteins may be antibodies for a target protein. Typical polymers may be polyethylene glycol. When the compound of the invention consists of a nucleobase sequence, it may, in one embodiment further comprise a non-nucleobase portion, such as the above conjugates.

The term "at least one" comprises the integers larger than or equal to 1, such as 1, 2, 3, 4, 5, 6, 7, 8, 9, 10, 11, 12, 13, 14, 15, 16, 17, 18, 19, 20 and so forth.

In one embodiment, such as when referring to the nucleic acid or protein targets of the compounds of the invention, the term "at least one" includes the terms "at least two" and at "least three" and "at least four", likewise the term "at least two" may comprise the terms at "least three" and "at least four".

As used herein, the term "pharmaceutically acceptable salts" refers to salts that retain the desired biological activity of the herein identified compounds and exhibit minimal undesired toxicological effects. Non-limiting examples of such salts can be formed with organic amino acid and base addition salts formed with metal cations such as zinc, calcium, bismuth, barium, magnesium, aluminum, copper, cobalt, nickel, cadmium, sodium, potassium, and the like, or with a cation formed from ammonia, /V,/V-dibenzylethylene-diamine, D-glucosamine, tetraethylammonium, or ethylenediamine; or (c) combinations of (a) and (b); e.g., a zinc tannate salt or the like.

In the present context, the term "C1-4-alkyl" is intended to mean a linear or branched saturated hydrocarbon chain wherein the chain has from one to four carbon atoms, such as methyl, ethyl, n-propyl, isopropyl, n-butyl, isobutyl, sec-butyl and tert-butyl.

As used herein, the term "gene" means the gene including exons, introns, non-coding 5 'and 3 'regions and regulatory elements and all currently known variants thereof and any further variants, which may be elucidated.

As used herein, the terms "RNA antagonist" refers to an oligonucleotide which targets any form of RNA (including pre-mRNA, mRNA, miRNA, siRNA etc).

The term "related disorders" when referring to hypercholesterolemia refers to one or more of the conditions selected from the group consisting of: atherosclerosis, hyperlipidemia, HDL/LDL cholesterol imbalance, dyslipidemias, e.g., familial combined hyperlipidemia (FCHL), acquired hyperlipidemia, statin-resistant hypercholesterolemia, coronary artery disease (CAD), and coronary heart disease (CHD).

In one embodiment, the term "oligomeric compound" refers to an oligonucleotide which can induce a desired therapeutic effect in humans through for example binding by hydrogen bonding to a target nucleic acid. It is also envisaged that the oligomeric compounds disclosed herein may have non-therapeutic applications, such as diagnostic applications.

As used herein, the term "modulation" means either an increase (stimulation) or a decrease (inhibition) in the expression of a gene. In the present invention, inhibition is the preferred form of modulation of gene expression and mRNA is a preferred target.

As used herein, "hybridisation" means hydrogen bonding, which may be Watson-Crick, Holstein, reversed Holstein hydrogen bonding, etc. between complementary nucleotide bases. Watson and Crick showed approximately fifty years ago that deoxyribo nucleic acid (DNA) is composed of two strands which are held together in a helical configuration by hydrogen bonds formed between opposing complementary nucleobases in the two strands. The four nucleobases, commonly found in DNA are guanine (G), adenine (A), thymine (T) and cytosine (C) of which the G nucleobase pairs with C, and the A nucleobase pairs with T. In RNA the nucleobase thymine is replaced by the nucleobase uracil (U), which similarly to the T nucleobase pairs with A. The chemical groups in the nucleobases that participate in standard duplex formation constitute the Watson-Crick face. Hoogsteen showed a couple of years later that the purine nucleobases (G and A) in addition to their Watson-Crick face have a Hoogsteen face that can be recognised from the outside of a duplex, and used to bind pyrimidine oligonucleotides via hydrogen bonding, thereby forming a triple helix structure.

It is highly preferred that the compounds of the invention are capable of hybridizing to the target nucleic acid, such as the mRNA.

In a preferred embodiment, the oligonucleotides are capable of hybridising against the target nucleic acid(s), such as the corresponding PCSK9 mRNA(s), to form a duplex with a Tₘ of at least 37°C, such as at least 40 °C, at least 50 °C, at least 55 °C, or at least 60°C. In one aspect the Tₘ is between 37 °C and 80 °C , such as between 50 and 70 °C, or between 40 and 60 °C, or between 40 and 70 °C. In one embodiment the Tₘ is lower than 80 °C, such as lower than 70 °C or lower that 60 °C or lower than 50 °C

### Measurement of Tₘ

A 3 µM solution of the compound in 10 mM sodium phosphate/100 mM NaCl/ 0.1 nM EDTA, pH 7.0 is mixed with its complement DNA or RNA oligonucleotide at 3 µM concentration in 10 mM sodium phosphate/100 mM NaCl/ 0.1 nM EDTA, pH 7.0 at 90 °C for a minute and allowed to cool down to room temperature. The melting curve of the duplex is then determined by measuring the absorbance at 260 nm with a heating rate of 1 °C/min. in the range of 25 to 95 °C. The Tₘ is measured as the maximum of the first derivative of the melting curve.

### Conjugates

In one embodiment of the invention the oligomeric compound is linked to ligands/conjugates, which may be used, *e.g*. to increase the cellular uptake of antisense oligonucleotides. WO2007/031091 provides suitable ligands and conjugates.

The invention also provides for a conjugate comprising the compound according to the invention as herein described, and at least one non-nucleotide or non-polynucleotide moiety covalently attached to said compound. Therefore, in one embodiment where the compound of the invention consists of s specified nucleic acid, as herein disclosed, the compound may also comprise at least one non-nucleotide or non-polynucleotide moiety (e.g. not comprising one or more nucleotides or nucleotide analogues) covalently attached to said compound.

### Applications

The oligomeric compounds of the present invention can be utilized for, for example, as research reagents for diagnostics, therapeutics and prophylaxis.

Some of the benefits of utilising LNA, and methods of preparing and purifying LNA and LNA oligonucleotides are disclosed in WO2007/031091.

The oligomeric compounds of the invention, such as the LNA containing oligonucleotide compounds of the present invention, can also be utilized for as research reagents for diagnostics, therapeutics and prophylaxis.

In research, such antisense oligonucleotides may be used to specifically inhibit the synthesis of PCSK9 genes in cells and experimental animals thereby facilitating functional analysis of the target or an appraisal of its usefulness as a target for therapeutic intervention.

In diagnostics the antisense oligonucleotides may be used to detect and quantitate PCSK9 expression in cell and tissues by Northern blotting, in-situ hybridisation or similar techniques.

For therapeutics, an animal or a human, suspected of having a disease or disorder, which can be treated by modulating the expression of PCSK9 is treated by administering antisense compounds in accordance with this invention. Further provided are the use in treating an animal particular mouse and rat and treating a human, suspected of having or being prone to a disease or condition, associated with expression of PCSK9 by administering a therapeutically or prophylactically effective amount of one or more of the antisense compounds or compositions of the invention.

The pharmaceutical composition according to the invention can be used for the treatment of conditions associated with abnormal levels of PCSK9, such as hypercholesteromeia and releted disorders.

Suitable dosages, formulations, administration routes, compositions, dosage forms, combinations with other therapeutic agents, pro-drug formulations are also provided in WO2007/031091, although it should be recognised that the aspects of WO2007/031091 which are only specifically applicable to the treatment of cancer may not be appropriate in the therapeutic/pharmaceutical compositions and methods of the present invention.

The invention also provides for a pharmaceutical composition comprising a compound or a conjugate as herein described or a conjugate, and a pharmaceutically acceptable diluent, carrier or adjuvant. WO2007/031091 provides suitable and preferred pharmaceutically acceptable diluent, carrier and adjuvants.

### Pharmaceutical compositions comprising more than one active ingredient

The pharmaceutical composition according to the invention may further comprise other active ingredients, including those which are indicated as being useful for the treatment of hypercholesterolemia and/ore related disorders.

One such class of compounds are statins. The statins are HMG-CoA reductase inhibitors that form a class of hypolipidemic agents, used as pharmaceuticals to lower cholesterol levels in people at risk for cardiovascular disease because of hypercholesterolemia. They work by inhibiting the enzyme HMG-CoA reductase, the enzyme that determines the speed of cholesterol synthesis. Inhibition of this enzyme in the liver stimulates the LDL-receptors, which results in an increased clearance of LDL from the bloodstream and a decrease in blood cholesterol levels. Examples of statins include Atorvastatin^{™}, Cerivastatin^{™}, Fluvastatin^{™}, Lovastatin^{™}, Mevastatin^{™}, Pravastatin^{™}, Pravastatin^{™}, Rosuvastatin^{™}, and Simvastatin^{™}. The combined use of the compound of the invention and the statins may allow for a reduction in the dose of the statins, therefore overcoming side effects associated with usual dosage of statins, which include, for example, myalgias, muscle cramps, gastrointestinal symptoms, liver enzyme derangements, myositis, myopathy, rhabdomyolysis (the pathological breakdown of skeletal muscle) which may lead to acute renal failure when muscle breakdown products damage the kidney.

Fibrates, a class of amphipathic carboxylic acids is an alternative class of compound which are often combined with statin use, despite an increased frequency of rhabdomyolysis which has been reported with the combined use of statins and fribrates. The composition according to the invention may therefore further comprise firbrates, and optionally statins.

The composition according to the invention may further comprise modulators of Apolipoprotein B (Apo-B), particularly agents which are capable of lowering the expression of function of Apo-B. Suitably, the Apo-B modulators may be antisense oligonucleotides(e.g. oligomers), such as those disclosed in WO 00/97662, WO 03/11887 and WO 2004/44181. A preferred combination is with ISIS compound 301012 (illustrated as SEQ ID NO 13).

The composition according to the invention may further comprise modulators of FABP4 expression, such as antisense oligonucleotides (e.g. oligomers) which target FABP4, the composition may be used in concurrent down-regulation of both FABP4 and PSCK9 expression, resulting in a synergistic effect in terms of blood serum cholesterol and hence advantages when treating hypercholesterolemia and/or related disorders. Such compositions comprising both the compounds of the invention and FABP4 modulators, such as the antisense oligonucleotides referred to herein, may also further comprise statins. US provisional application 60/969,016 hereby incorporated by reference discloses suitable FABP4 modulators.

It is also envisaged that the composition may comprise antisense oligonucleotides which comprise nucleotide analogues, such as those disclosed in WO2007/031081 . Specific LNA oligonucleotides, as disclosed or highlighted are preferred in WO2007/031091 are especially suited for us in the pharmaceutical composition according to the present invention.

The invention also provides a kit of parts wherein a first part comprises the compound, the conjugate and/or the pharmaceutical composition according to the invention and a further part comprises an antisense oligonucleotide capable of lowering the expression of Apo-B or FABP4. It is therefore envisaged that the kit of parts may be used in a method of treatment, as referred to herein, where the method comprises administering both the first part and the further part, either simultaneously or one after the other.

### Medical methods and use

Further conditions which may be associated with abnormal levels of PCSK9, and which, therefore may be treated using the compositions, conjugates and compounds according to the invention include disorders selected form the group consisting of: hyperlipoproteinemia, familial type 3 hyperlipoprotienemia (familial dysbetalipoproteinemia), and familial hyperalphalipoprotienemia; hyperlipidemia, mixed hyperlipidemias, multiple lipoprotein-type hyperlipidemia, and familial combined hyperlipidemia; hypertriglyceridemia, familial hypertriglyceridemia, and familial lipoprotein lipase; hypercholesterolemia, statin-resistant hypercholesterolemia familial hypercholesterolemia, polygenic hypercholesterolemia, and familial defective apolipoprotein B; cardiovascular disorders including atherosclerosis and coronary artery disease; thrombosis; peripheral vascular disease, and obesity.

Further conditions which may be associated with abnormal levels of PCSK9, and which, therefore may be treated using the compositions, conjugates and compounds according to The invention include disorders selected form the group consisting of: von Gierke's disease (glycogen storage disease, type I); lipodystrophies (congenital and acquired forms); Cushing's syndrome; sexual ateloitic dwarfism (isolated growth hormone deficiency); diabetes mellitus; hyperthyroidism; hypertension; anorexia nervosa; Werner's syndrome; acute intermittent porphyria; primary biliary cirrhosis; extrahepatic biliary 5 obstruction; acute hepatitis; hepatoma; systemic lupus erythematosis; monoclonal gammopathies (including myeloma, multiple myeloma, macroglobulinemia, and lymphoma); endocrinopathies; obesity; nephrotic syndrome; metabolic syndrome; inflammation; hypothyroidism; uremia (hyperurecemia); impotence; obstructive liver disease; idiopathic hypercalcemia; dysqlobulinemia; elevated insulin levels; Syndrome X; Dupuytren's contracture; AIDS; and Alzheimer's disease and dementia.

The compounds of the invention may be used in methods of inhibiting cholesterol particle binding to vascular endothelium comprising the step of administering to an individual an amount of a compound of the invention sufficient to PCSK9 expression, and as a result, the invention also provides methods of reducing the risk of: (i) cholesterol particle oxidization; (ii) monocyte binding to vascular endothelium; (iii) monocyte differentiation into macrophage; (iv) macrophage ingestion of oxidized lipid 30 particles and release of cytokines (including, but limited to IL-I,TNF-alpha, TGF-beta); (v) platelet formation of fibrous fibrofatty lesions and inflammation; (vi) endothelium lesions leading to clots; and (vii) clots leading to myocardial infarction or stroke, also comprising the step of administering to an individual an amount of a compound of the invention sufficient to inhibit PCSK9 expression.

The compounds of the invention may be used in reducing hyperlipidemia associated with alcoholism, smoking, use of oral contraceptives, use of glucocorticoids, use of beta-adrenergic blocking agents, or use of isotretinion (13-cis retinoic acid) comprising the step of administering to an individual an amount of a compound of the invention sufficient to inhibit PCSK9 expression.

The invention further provides use of a compound of the invention in the manufacture of a medicament for the treatment of any and all conditions disclosed herein.

Generally stated, one aspect of the invention is directed to the use of the compounds in treating a mammal suffering from or susceptible to conditions associated with abnormal levels of PCSK9, comprising administering to the mammal and therapeutically effective amount of an oligonucleotide targeted to PCSK9 that comprises one or more LNA units.

An interesting aspect of the invention is directed to the use of a compound as defined herein or as conjugate as defined herein for the preparation of a medicament for the treatment of a condition according to above.

The methods of the invention are preferably employed for treatment or prophylaxis against diseases caused by abnormal levels of PCSK9.

Furthermore, the invention described herein encompasses the use of the compounds in preventing or treating a disease comprising a therapeutically effective amount of a PCSK9 modulating oligonucleotide compound, including but not limited to high doses of the oligomer, to a human in need of such therapy. The invention further encompasses the use of a short period of administration of a PCSK9 modulating oligonucleotide compound.

In one embodiment of the invention the oligonucleotide compound is linked to ligands/conjugates. It is way to increase the cellular uptake of antisense oligonucleotides.

Oligonucleotide compounds of the invention may also be conjugated to active drug substances, for example, aspirin, ibuprofen, a sulfa drug, an antidiabetic, an antibacterial or an antibiotic.

Alternatively stated, the invention is furthermore directed to the use of the compounds in treating abnormal levels of PCSK9, said method comprising administering a compound as defined herein, or a conjugate as defined herein or a pharmaceutical composition as defined herein to a patient in need thereof and further comprising the administration of a further chemotherapeutic agent. Said further administration may be such that the further chemotherapeutic agent is conjugated to the compound of the invention, is present in the pharmaceutical composition, or is administered in a separate formulation.

The invention also relates to a compound, composition or a conjugate as defined herein for use as a medicament.

The invention further relates to use of a compound, composition, or a conjugate as defined herein for the manufacture of a medicament for the treatment of abnormal levels of PCSK9. Typically, said abnormal levels of PCSK9 is in the form of, or causes, or is characterised by, hypercholesterolemia and related disorders, such as atherosclerosis or hyperlipidemia. Moreover, the invention relates to the use of the compounds in treating a subject suffering from a disease or condition selected from hypercholesterolemia and related disorders, such as atherosclerosis, and hyperlipidemia, the method comprising the step of administering a pharmaceutical composition as defined herein to the subject in need thereof. Preferably, the pharmaceutical composition is administered orally.

Examples of related diseases also include different types of HDL/LDL cholesterol imbalance; dyslipidemias, e.g., familial combined hyperlipidemia (FCHL), acquired hyperlipidemia, statin-resistant hypercholesterolemia; coronary artery disease (CAD) coronary heart disease (CHD), atherosclerosis.

It is recognised that when the composition according to the invention also comprises modulators of Apo-B100 or FABP4 expression, such as antisense oligonucleotides which target ApoB-100 or FABP4, the composition may be used in concurrent down-regulation of both PCSK9 and ApoB-100 (or FABP4) expression, resulting in a synergistic effect in terms of blood serum cholesterol and hence advantages when treating hypercholesterolemia and/or related disorders. Such compositions comprising both the compounds of the invention and ApoB or FABP4 modulators, such as the antisense oligonucleotides referred to herein, may also further comprise statins.

**Table 2: Designs of specific compounds / LNA antisense oligonucleotides. Note the numbers referred to in the Examples and the Figures refer to the compound ID# numbers. The above table provides both compound ID NO#, and the corresponding SEQ ID used in the sequence listing and the motif ID, also referred to in the sequence listing. Further oligomer sequence motifs according to the invention are shown in table 3.**

| **Comp' d ID#** | **Length** | **Sequence** | **SEQ ID** | **MOTIF SEQ ID** |
|---|---|---|---|---|
| 262 | 16 | 5'- **Gₛ ^{o m} cₛ ^{o m} cₛ ^{o}** tₛ gₛ tₛ cₛ tₛ gₛ tₛ gₛ gₛ aₛ **Aₛ ^{o}Gₛ^{om}C^{o} -3'** | 10 | 3 |
| 80 | 14 | 5'- **Gₛ^{o}Aₛ^{o} Gₛ^{o}**tₛ aₛ gₛ aₛ gₛ gₛ cₛ aₛ **Gₛ^{o}Gₛ^{om}C^{o}** -3' | 20 | 30 |
| 338 | 16 | 5'- **mcₛ^{o}Aₛ^{o}Aₛ^{o}** gₛ tₛ tₛ aₛ cₛ aₛ aₛ aₛ aₛ gₛ **^{m}cₛ^{o}Aₛ^{o}A^{o} -3'** | 11 | 4 |
| 341 | 16 | 5'- **Gₛ^{o}Aₛ^{o}Gₛ^{o}**aₛ tₛ aₛ cₛ aₛ cₛ cₛ tₛ cₛ cₛ **Aₛ^{om}Cₛ^{om}C^{o}** -3' | 9 | 5 |
| 301 | 16 | 5'-**Tₛ^{om}cₛ^{o m} cₛ^{o} tₛ** cₛ aₛ gₛ gₛ gₛ aₛ aₛ cₛ cₛ Aₛ **^{o} Gₛ^{o}G^{o}** -3' | 21 | 31 |
| 317 | 16 | 5'- **mcₛ^{o}Tₛ^{o}Gₛ^{o}** gₛ aₛ gₛ cₛ aₛ gₛ cₛ tₛ cₛ aₛ Gₛ^{om}Cₛ^{o}A^{o} -3' | 22 | 32 |
| 323 | 16 | 5'- **^{m}Cₛ^{o}Aₛ^{o}Tₛ^{o}**gₛ gₛ cₛ aₛ gₛ cₛ aₛ gₛ gₛ aₛ A**ₛ^{o}Gₛ^{om}C^{o}** -3', | 23 | 33 |
| 98 | 14 | 5'- **Gₛ^{o}Aₛ^{o}Tₛ^{o}** aₛ cₛ aₛ cₛ cₛ tₛ cₛ cₛ **Aₛ ^{o m} Cₛ ^{o m} C^{o}** -3' | 24 | 34 |
| 101 | 14 | 5'- **^{m}CₛoTₛ^{o}Gₛ^{o}** tₛ cₛ tₛ gₛ tₛ gₛ gₛ aₛ **Aₛ^{o}Gₛ^{o m}C^{o}** -3' | 25 | 35 |
| 9 | 13 | 5'- **Gₛ^{o}Tₛ^{o}**s^{o}cₛ tₛ gₛ tₛ gₛ gₛ aₛ aₛ **Gₛ^{o}Cₛ^{o}G^{o}** -3' | 26 | 36 |
| 11 | 13 | 5 **Aₛ^{o}Tₛ^{o}**gₛ aₛ gₛ gₛ gₛ tₛ gₛ cₛ cₛ **^{m}Gₛ^{om}C^{o}** -3' | 27 | 37 |
| 16 | 13 | 5'- **Aₛ^{o}Tₛ^{o}** aₛ aₛ aₛ cₛ tₛ cₛ cₛ aₛ **Gₛ^{o}Gₛ^{om}C^{o}** -3' | 28 | 38 |
| 18 | 13 | 5'-**Tₛ^{o}Aₛ^{o}** gₛ aₛ cₛ aₛ cₛ cₛ cₛ tₛ **^{m}Cₛ^{o}Aₛ^{om}C^{o}** -3' | 29 | 39 |

### EXAMPLES

### Example 1: Monomer synthesis

The LNA monomer building blocks and derivatives thereof were prepared following published procedures and references cited therein, see:
WO 03/095467 A1
   D. S. Pedersen, C. Rosenbohm, T. Koch (2002) Preparation of LNA Phosphoramidites, Synthesis 6, 802-808.
   M. D. Sørensen, L. Kværnø, T. Bryld, A. E. Håkansson, B. Verbeure, G. Gaubert, P. Herdewijn, J. Wengel (2002) α-L-ribo-configured Locked Nucleic Acid (α-I-LNA): Synthesis and Properties, J. Am. Chem. Soc., 124, 2164-2176.
   S. K. Singh, R. Kumar, J. Wengel (1998) Synthesis of Novel Bicyclo[2.2.1] Ribonucleosides: 2'-Amino- and 2'-Thio-LNA Monomeric Nucleosides, J. Org. Chem. 1998, 63, 6078-6079.
   C. Rosenbohm, S. M. Christensen, M. D. Sørensen, D. S. Pedersen, L. E. Larsen, J. Wengel, T. Koch (2003) Synthesis of 2'-amino-LNA: a new strategy, Org. Biomol. Chem. 1, 655-663.
   D. S. Pedersen, T. Koch (2003) Analogues of LNA (Locked Nucleic Acid). Synthesis of the 2'-Thio-LNA Thymine and 5-Methyl Cytosine Phosphoramidites, Synthesis 4, 578-582.

### Example 2: Oligonucleotide synthesis

Oligonucleotides were synthesized using the phosphoramidite approach on an Expedite 8900/MOSS synthesizer (Multiple Oligonucleotide Synthesis System) at 1 µmol or 15 µmol scale. For larger scale synthesis an Äkta Oligo Pilot was used. At the end of the synthesis (DMT-on), the oligonucleotides were cleaved from the solid support using aqueous ammonia for 1-2 h at room temperature, and further deprotected for 4 h at 65°C. The oligonucleotides were purified by reverse phase HPLC (RP-HPLC). After the removal of the DMT-group, the oligonucleotides were characterized by AE-HPLC, RP-HPLC, and CGE and the molecular mass was further confirmed by ESI-MS. See below for more details.

### Preparation of the LNA-solid support:

### Preparation of the LNA succinyl hemiester

5'-O-Dmt-3'-hydroxy-LNA monomer (500 mg), succinic anhydride (1.2 eq.) and DMAP (1.2 eq.) were dissolved in DCM (35 mL). The reaction was stirred at room temperature overnight. After extractions with NaH₂PO₄ 0.1 M pH 5.5 (2x) and brine (1x), the organic layer was further dried with anhydrous Na₂SO₄ filtered and evaporated. The hemiester derivative was obtained in 95% yield and was used without any further purification.

### Preparation of the LNA-support

The above prepared hemiester derivative (90 µmol) was dissolved in a minimum amount of DMF, DIEA and pyBOP (90 µmol) were added and mixed together for 1 min. This pre-activated mixture was combined with LCAA-CPG (500 Å, 80-120 mesh size, 300 mg) in a manual synthesizer and stirred. After 1.5 h at room temperature, the support was filtered off and washed with DMF, DCM and MeOH. After drying, the loading was determined to be 57 µmol/g (see Tom Brown, Dorcas J.S.Brown. Modern machine-aided methods of oligodeoxyribonucleotide synthesis. In: F.Eckstein, editor. Oligonucleotides and Analogues A Practical Approach. Oxford: IRL Press, 1991: 13-14).

### Elongation of the oligonucleotide

The coupling of phosphoramidites (A(bz), G(ibu), 5-methyl-C(bz)) or T-β-cyanoethyl-phosphoramidite) is performed by using a solution of 0.1 M of the 5'-O-DMT-protected amidite in acetonitrile and DCI (4,5-dicyanoimidazole) in acetonitrile (0.25 M) as activator. The thiolation is carried out by using xanthane chloride (0.01 M in acetonitrile:pyridine 10%). The rest of the reagents are the ones typically used for oligonucleotide synthesis. The protocol provided by the supplier was conveniently optimised.

### Purification by RP-HPLC:

- Column:: Xterra RP₁₈
- Flow rate:: 3 mL/min
- Buffers:: 0.1 M ammonium acetate pH 8 and acetonitrile

### Abbreviations

- DMT:: Dimethoxytrityl
- DCI:: 4,5-Dicyanoimidazole
- DMAP:: 4-Dimethylaminopyridine
- DCM:: Dichloromethane
- DMF:: Dimethylformamide
- THF:: Tetrahydrofurane
- DIEA:: *N,N*-diisopropylethylamine
- PyBOP:: Benzotriazole-1-yl-oxy-tris-pyrrolidino-phosphonium hexafluorophosphate
- Bz:: Benzoyl
- Ibu:: Isobutyryl

### Example 3: Design of the oligonucleotide compound

See table 2 and 3 (below) - Upper case letters indicates ribonucleotide units and subscript "s" represents 2'-O-methyl-modified ribonucleotide units.

In one embodiment of the invention, SEQ ID NOs: 3 and 4 contains at least 3 LNA nucleotides, such as 6 (7 or 8 LNAs) nucleotides like in SEQ ID NOs: 3 and 4.

### Example 4: Stability of LNA compounds in human or rat plasma

LNA oligonucleotide stability was tested in plasma from humans or rats (it could also be mouse, monkey or dog plasma). In 45 µl plasma 5 µl oligonucleotide is added (a final concentration of 20 µM). The oligos are incubated in plasma for times ranging from 0 h-96

h at 37°C (the plasma is tested for nuclease activity up to 96 h and shows no difference in nuclease cleavage-pattern). At the indicated time the sample were snap-frozen in liquid nitrogen. 2 µl (equals 40 pmol) oligonucleotide in plasma was diluted by adding 15 µl of water and 3 µl 6x loading dye (Invitrogen). As marker a 10 bp ladder (Invitrogen 10821-015) is used. To 1 µl ladder 1 µl 6x loading and 4 µl water was added. The samples were mixed, heated to 65°C for 10 min and loaded to a pre-run gel (16% acrylamide, 7 M UREA, 1x TBE, pre-run at 50 Watt for 1h) and run at 50-60 Watt for 2 ½ h. Subsequently the gel was stained with 1x SyBR gold (molecular probes) in 1x TBE for 15 min. The bands were visualised using a phosphoimager from Biorad.

### Example 5: In vitro model: Cell culture

The effect of antisense compounds on target nucleic acid expression can be tested in any of a variety of cell types provided that the target nucleic acid is present at measurable levels. Target can be expressed endogenously or by transient or stable transfection of a nucleic acid encoding said nucleic acid.

The expression level of target nucleic acid can be routinely determined using, for example, Northern blot analysis, Quantitative PCR, Ribonuclease protection assays. The following cell types are provided for illustrative purposes, but other cell types can be routinely used, provided that the target is expressed in the cell type chosen.

Cells were cultured in the appropriate medium as described below and maintained at 37°C at 95-98% humidity and 5% CO₂. Cells were routinely passaged 2-3 times weekly.

Huh-7: Human liver cell line Huh-7 was purchased from ATCC and cultured in Eagle MEM (Sigma) with 10% FBS + Glutamax I + non-essential amino acids + gentamicin.

### Example 6: In vitro model: Treatment with antisense oligonucleotide

Cell culturing and transfection: Huh-7 and Hepa 1-6 cells were seeded in 6-well plates at 37°C (5% CO₂) in growth media supplemented with 10% FBS, Glutamax I and Gentamicin. When the cells were 60-70% confluent, they were transfected in duplicates with different concentrations of oligonucleotides (0.04 - 25 nM) using Lipofectamine 2000 (5 µg/mL). Transfections were carried out essentially as described by Dean et al. (1994, JBC 269:16416-16424). In short, cells were incubated for 10 min. with Lipofectamine in OptiMEM followed by addition of oligonucleotide to a total volume of 0.5 mL transfection mix per well. After 4 hours, the transfection mix was removed, cells were washed and grown at 37°C for approximately 20 hours (mRNA analysis and protein analysis in the appropriate growth medium. Cells were then harvested for protein and RNA analysis.

### Example 7: in vitro model: Extraction of RNA and cDNA synthesis

### Total RNA Isolation

Total RNA was isolated using RNeasy mini kit (Qiagen). Cells were washed with PBS, and Cell Lysis Buffer (RTL, Qiagen) supplemented with 1% mercaptoethanol was added directly to the wells. After a few minutes, the samples were processed according to manufacturer's instructions.

### First strand synthesis

First strand synthesis was performed using either OmniScript Reverse Transcriptase kit or M-MLV Reverse transcriptase (essentially as described by manufacturer (Ambion)) according to the manufacturer's instructions (Qiagen). When using OmniScript Reverse Transcriptase 0.5 µg total RNA each sample, was adjusted to 12 µl and mixed with 0.2 µl poly (dT)₁₂₋₁₈ (0.5 µg/µl) (Life Technologies), 2 µl dNTP mix (5 mM each), 2 µl 10x RT buffer, 0.5 µl RNAguard^{™} RNase Inhibitor (33 units/mL, Amersham) and 1 µl OmniScript Reverse Transcriptase followed by incubation at 37°C for 60 min. and heat inactivation at 93°C for 5 min.

When first strand synthesis was performed using random decamers and M-MLV-Reverse Transcriptase (essentially as described by manufacturer (Ambion)) 0.25 µg total RNA of each sample was adjusted to 10.8 µl in H₂O. 2 µl decamers and 2 µl dNTP mix (2.5 mM each) was added. Samples were heated to 70°C for 3 min. and cooled immediately in ice water and added 3.25 µl of a mix containing (2 µl 10x RT bluffer; 1 µl M-MLV Reverse Transcriptase; 0.25 µl RNAase inhibitor). cDNA is synthesized at 42°C for 60 min followed by heating inactivation step at 95 °C for 10 min and finally cooled to 4 °C.

*Example 8: in vitro and in vivo model: Analysis of Oligonucleotide Inhibition of PCSK9*

### Expression by Real-time PCR

Antisense modulation of PCSK9 expression can be assayed in a variety of ways known in the art. For example, PCSK9 mRNA levels can be quantitated by, e.g., Northern blot analysis, competitive polymerase chain reaction (PCR), or real-time PCR. Real-time quantitative PCR is presently preferred. RNA analysis can be performed on total cellular RNA or mRNA.

Methods of RNA isolation and RNA analysis such as Northern blot analysis is routine in the art and is taught in, for example, Current Protocols in Molecular Biology, John Wiley and Sons.

Real-time quantitative (PCR) can be conveniently accomplished using the commercially iQ Multi-Color Real Time PCR Detection System available from BioRAD. Real-time Quantitative PCR is a technique well known in the art and is taught in for example Heid et al. Real time quantitative PCR, Genome Research (1996), 6: 986-994.

### Real-time Quantitative PCR Analysis of PCSK9 mRNA Levels

To determine the relative human PCSK9 mRNA level in treated and untreated samples, the generated cDNA was used in quantitative PCR analysis using an iCycler from Bio-Rad or 7500 Fast Real-Time PCR System from Applied Biosystems.

8 µl of 10-fold diluted cDNA was added 52 µl of a mix containing 29,5 µl Platinum qPCR Supermix-UDG (Invitrogen) 19,2 µl H₂O and 3,0 µl of a 20x human PCSK9 or GAPDH TaqMan gene expression assay (Applied Biosystems). Each sample was analysed in duplicates. PCR program: 95 °C for 20 seconds followed by 40 cycles of 95 °C, 3 seconds, 60 °C, 30 seconds.

Mouse PCSK9: Mouse PCSK9 expression is quantified using a mouse PCSK9 or GAPDH TaqMan gene expression assay (Applied Biosystems) 8 µl of 10-fold diluted cDNA is added 52 µl of a mix containing 29,5 µl Platinum qPCR Supermix-UDG (Invitrogen) 19,2 µl H₂O and 3,0 µl of a 20x mouse PCSK9 or GAPDH TaqMan gene expression assay (Applied Biosystems). Each sample is analysed in duplicates. PCR program: 95 °C for 20 seconds followed by 40 cycles of 95 °C, 3 seconds, 60 °C, 30 seconds.

PCSK9 mRNA expression is normalized to mouse Gapdh mRNA which was similarly quantified using Q-PCR.

2-fold dilutions of cDNA synthesised from untreated human Hepatocyte cell line (Huh-7) (diluted 5 fold and expressing both PCSK9 and Gapdh) is used to prepare standard curves for the assays. Relative quantities of PCSK9 mRNA were determined from the calculated Threshold cycle using the iCycler iQ Real Time Detection System software.

### Example 9 In vitro analysis: Dose response in cell culture (human hepatocyte Huh-7)/ Antisense Inhibition of Human PCSK9 Expression

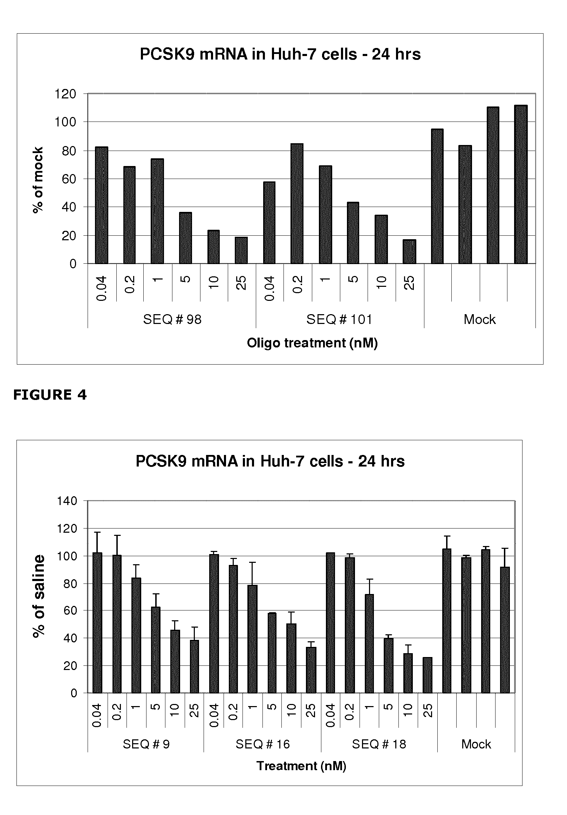

In accordance with the present invention, a series of oligonucleotides were designed to target different regions of the human PCSK9 mRNA. See Table 2 Oligonucleotide compounds were evaluated for their potential to knockdown PCSK9 mRNA in Human hepatocytes (Huh-7 cells) following lipid-assisted uptake of Compound ID NO#s: 9, 16, 18, 98, 101, 262, 301, 317, 323, 338, 341 (Figures 1-4). The experiment was performed as described in examples 5-8. The results showed very potent down regulation (60 to ≥80%) with 25 nM for all compounds.

### Example 10 In vitro analysis: Dose response in cell culture (murine hepatocyte Hepa 1-6)/ Antisense Inhibition of Murine PCSK9 Expression

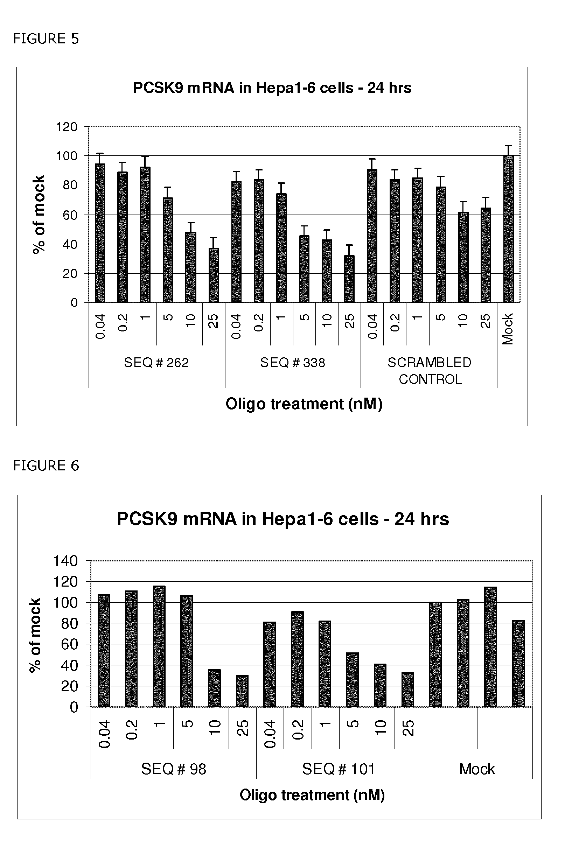

In accordance with the present invention, a series of oligonucleotides were designed to target different regions of the murine PCSK9 mRNA (See Table 2). Oligonucleotide compounds were evaluated for their potential to knockdown PCSK9 mRNA in Murine hepatocytes (Hepa 1-6) following lipid-assisted uptake of Compound ID NO#s: 98, 101, 262 and 338 (Figures 5-6). The experiment was performed as described in examples 5-8. The results showed very potent down regulation (≥60%) with 25 nM for all compounds

### Example 11 Cholesterol levels in mouse serum

Total cholesterol level was measured in serum using a colometric assay Cholesterol CP from ABX Pentra. The cholesterol is measured following enzymatic hydrolysis and oxidation. 20 µL water was added to 3 µL serum. 240 µL reagent is added and within 15 min the cholesterol content is measured at a wavelength of 500 nM. Measurements on each animal was made in duplicates. A standard curve was made using Multi Cal from ABX Diagnostics.

Cholesterol levels in the different lipoprotein classes (VLDL/LDL and HDL) was measured in serum by ultracentrifugation. The serum was adjusted to a density of 1.067 g/ml allowing to separate HDL from the other lipoproteins. Total cholesterol (ABX Pentra) is measured in each fraction (top and bottom) after centrifugation at approximately 400.000 g for 4 hours at 15 °C.

### Example 12 LDL-receptor protein level in mouse liver

### Western blotting

Liver samples were snap frozen in liquid nitrogen and stored at -80 °C until analysed. 30 mg tissue was defrosted and homogenised in 300 µl T-per Tissue Protein Extraction buffer (Pierce), supplemented with Halt Protease inhibitor cocktail (Pierce).

Total protein was measured by BCA protein assay kit (Pierce) using an albumin standard according to manufacturer's protocol.

25 µg total protein from each sample was loaded on a 4-12% Bis-Tris gel with 4x LDS sample buffer (NuPAGE, Invitrogen). The gel was run for two hours at 130 V in MOPS (Invitrogen). Protein bands were blotted on a PVDF membrane using a blotting module according to standard protocol (XCell II Blot Module, Invitrogen). The membrane was blocked in 5% skimmed milk powder in 1× PBS over night. For immunodetection, the membrane was incubated overnight in a blocking solution with primary antibodies of 1:1000 dilution of polyclonal goat-anti-mouse-LDLR antibody (R&D Systems) and 1:2000 dilution of monoclonal Mouse-anti-tubulin antibody (NeoMarkers). This was followed by two hours incubation in secondary antibody solution of 1:2000 dilution of HRP/anti-goat antibody and 1:2000 dilution of HRP/anti-mouse antibody (Dako). LDLR and tubulin bands were visualized using Chemiluminescence ECL+ detection kit (Amersham) and a VersaDoc5000 imaging system (Bio-Rad).

### Example 13 Lipoprotein class composition in serum measured using Sebia gels

Agarose gel electrophoresis in barbital buffer is used to separate lipoproteins according to charge and is one of the original methods for clinical analysis of lipoprotein profiles. Gels are usually stained with a lipophilic dye such as Sudan Black. The dye(s) will not distinguish between lipid species, hence the method is limited to providing a "general lipoproteins profile" as dyes cannot distinguish between cholesterol ester and triglycerides. However, the small sample volume, high reproducibility, and the possibility to follow changes in lipoprotein profiles (as percent lipid/band) in individual animals makes the agarose gels a useful tool for lipoprotein analysis. Analyses are made on high-quality gels and specialized electrophoresis equipment (Lipoprotein + Lp(a) agarose gel elctrophoresis, Sebia, France). Serum was isolated from mouse blood by centrifugation and the lipoproteins were separated on Sebia Gels and quantified using Sudan Black staining followed by scanning the gels (Molecular Imager FX) and analyzed by Quantity One software, using the Densiometry settings.

### Example 14 In vivo analysis: Dose response of different LNA oligonucleotides in C57BL/6 female mice.

In accordance with the present invention, a series of oligonucleotides were designed to target different regions of the murine PCSK9 mRNA. Three of these oligonucleotides were evaluated for their potential down regulation on PCSK9 mRNA in liver, reduction of serum cholesterol and increase in LDL-recptor protein in liver.

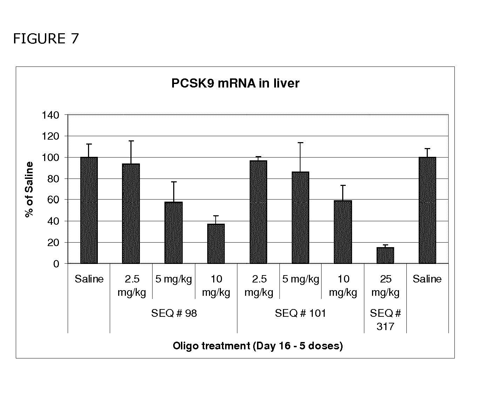

C57BL/6 female were were dosed 2.5, 5, or 10 mg/kg i.v. of the oligonucleotide or saline days 0, 3, 7, 10 and 14 and sacrificed day 16 after the first treatment dose. Liver was sampled for analysis of PCSK9 mRNA expression by qPCR (as described in example 8). PCSK9 mRNA expression was down regulated in a dose dependent manner after dosing Compound ID NO#s 98 and 101 (figure 7).

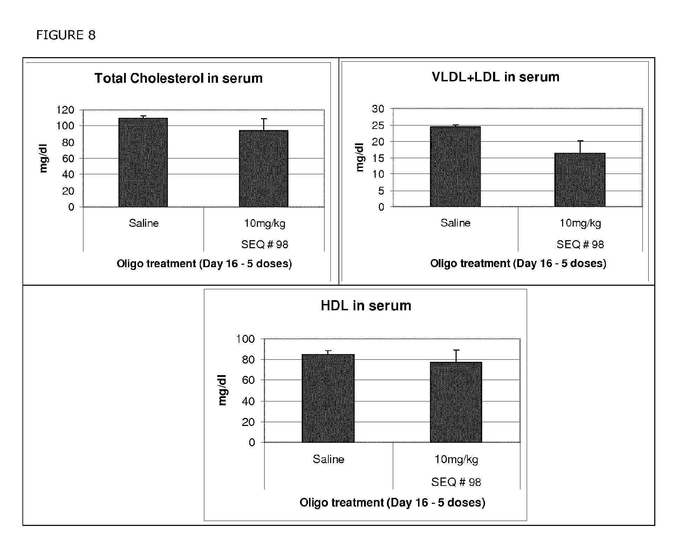

Blood was sampled at sacrifice for serum preparation and serum cholesterol was measured as described in example 11. Compound ID NO# 98 showed tendency of reduced serum total cholesterol and reduced level of VLDL+LDL cholesterol, about 30% and no effect on HDL-cholesterol (figure 8).

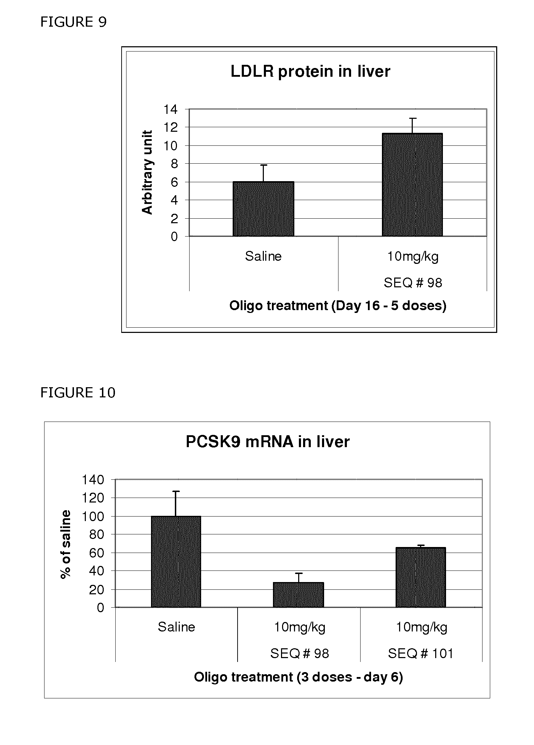

The down regulation of PCSK9 mRNA was expected to have an effect on the number of LDL-receptors presented on the surface of the hepatocytes. Western Blotting was used to examine the LDL-receptor protein in liver (example 12). The Compound ID NO# 98 resulted in an increase in LDL-receptor protein of about 80% compared to the saline group (figure 9).

### Example 15 In vivo analysis:Efficacy of LNA oligonucleotides of down regulating PCSK9 in female NMRI mice.

Two oligonucleotides targeting different refions of the murine PCSK9 mRNA was examined for potency to down regulate PCSK9 mRNA expression, reduce serum total cholesterol and increase LDL-receptor protein level.

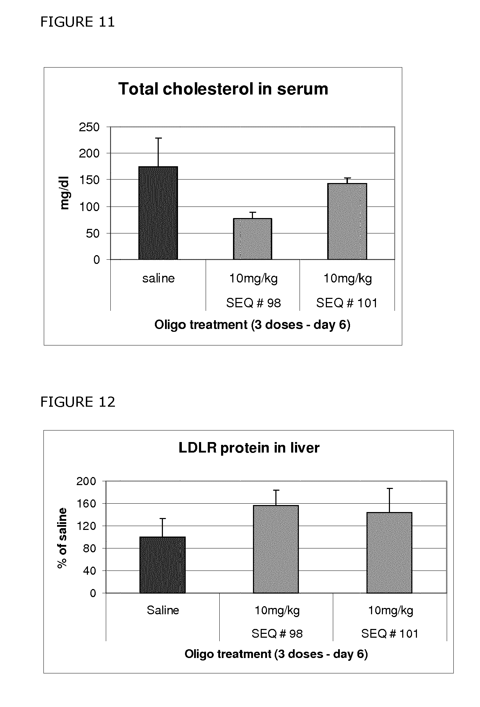

NMRI female mice were dosed i.v. 10 mg/kg/dose LNA oligonucleotide or saline at days 0, 2, 4 and sacrificed at day 6. Liver was sampled for analysis of PCSK9 mRNA expression by qPCR (as described in example 8). PCSK9 mRNA expression was reduced with about 70% after dosing Compound ID NO# 98 and about 30% dosing Compound ID NO# 101 (figure 10). The effect of this down regulation was observed on the LDL-receptor protein level in liver; about 50% and 40% increase after dosing Compound ID NO#s 98 and 101, respectively (figure 11. This increase in LDL-receptor resulted in decrease in serum cholesterol of 55% and 15% for Compound ID NO# 98 and 101, respectively (figure 12).

### Example 16 In vivo analysis: Efficacy of LNA oligonucleotides to reduce PCSK9 mRNA expression in C578L/6 fed a high fat diet (HFD) for 1 or 5 month before dosing.

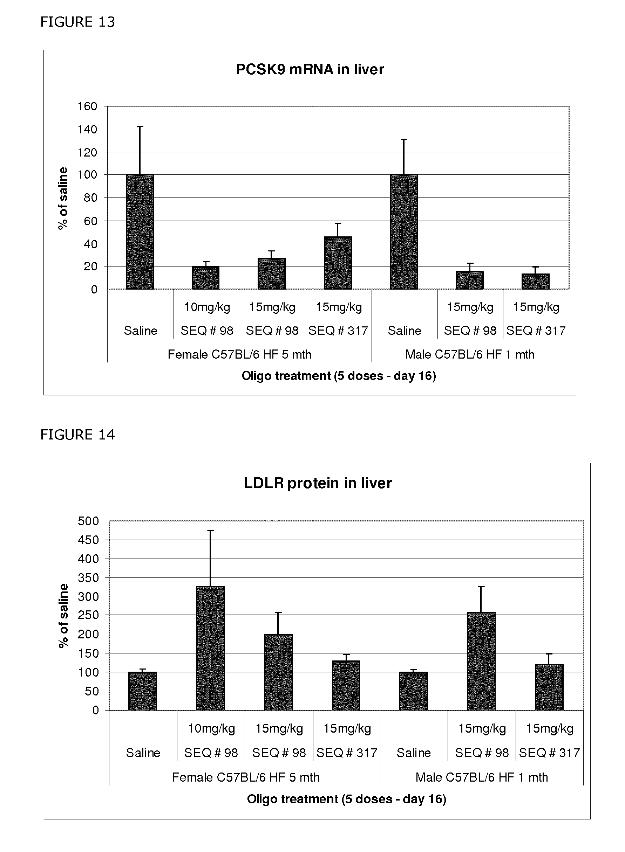

C57BL/6 female mice were fed a high fat diet (HFD) (60 energy% fat) for 5 month and male C57BL/6 were fed a HFD for 1 month before dosing LNA oligonucleotides at 10 or 15 mg/kg days 0, 3, 7, 10, 14 and sacrifice day 16. Liver was sampled for analysis of PCSK9 mRNA expression by qPCR (as described in example 8). Dosing Compound ID NO#s 98 and 317 resulted in a down regulation of PCSK9 mRNA expression (analysed by qPCR as described in example 8) of about 80 and 60%, respectively, in female mice and about 85% in male mice for both compounds (figure 13). The LDL-receptor protein level measured by Western blotting (described in example 12) was increased about 2-3 times after dosing Compound ID NO# 98 and 20% after dosing Compound ID NO# 317 to female HFD mice. In male mice 15 mg/kg/dose Compound ID NO# 98 resulted in an increased LDL-receptor protein level of 2.5 times whereas Compound ID NO# 317 had only minor effect on LDL-receptor protein level (figure 14).

### Example 17 In vivo analysis: Efficacy of 13-mer LNA oligonucleotides to reduce PCSK9 mRNA expression in NMRI female mice.

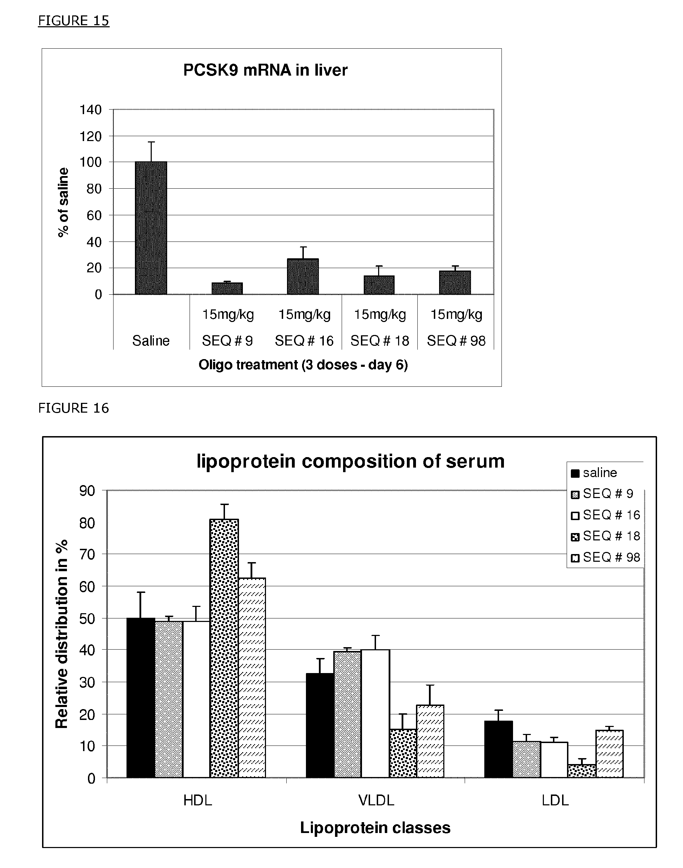

MNRI female mice were dosed 15 mg/kg days 0, 2 and 4 and sacrificed day 6. Liver was sampled for analysis of PCSK9 mRNA expression by qPCR as described in example 8. The 13-mer oligonucleotides; Compound ID NO#s 9, 16 and 18 resulted in reduction of PCSK9 mRNA expression of 90%, 70% and 85%, respectively and the 14-mer Compound ID NO# 98 gave 80% reduction in PCSK9 mRNA (figure 15). The distribution of the different lipoprotein classes in serum was determined after separation on Sebia gels as described in example 13. The distribution between the different classes (set to 100% for each group and presented relative to the other lipoprotins in that group) was examined for Compound ID NO#s 9, 16, 18 and 98. The highest effect was observed for the Compound ID NO# 18 for all lipipoproteins (50% and 65% reduction relative to saline for VLDL and LDL, respectively, as a result HDL was increased by 60%) and , Compound ID NO# 98 reduced VLDL by 30% and by about 10% for LDL relative to saline, and as a result HDL was increased by 20% (figure 16).

**TABLE 3: Further sequences of major interest for synthesis of antisense oligonucleotides targeting PCSK9, such as LNA antisense oligonucleotides.**

| **Target site Start** | **Target site End** | **Oligo length** | **Target sequence** | **Human mRNA targets** | **Murine mRNA targets** | **Oligo sequence** | **MotifS eq ID #** |
|---|---|---|---|---|---|---|---|
| 1082 | 1093 | 12 | CGCTTCCACAGA | 2 | 2 | TCTGTGGAAGCG | 40 |
| 1228 | 1239 | 12 | CACCCTCATAGG | 2 | 2 | CCTATGAGGGTG | 41 |
| 1244 | 1255 | 12 | GAGTTTATTCGG | 2 | 2 | CCGAATAAACTC | 42 |
| 1239 | 1250 | 12 | GCCTGGAGTTTA | 2 | 3 | TAAACTCCAGGC | 43 |
| 1138 | 1149 | 12 | GGTCAGCGGCCG | 2 | 4 | CGGCCGCTGACC | 44 |
| 1233 | 1244 | 12 | TCATAGGCCTGG | 2 | 4 | CCAGGCCTATGA | 45 |
| 1230 | 1241 | 12 | CCCTCATAGGCC | 3 | 1 | GGCCTATGAGGG | 46 |
| 1082 | 1094 | 13 | CGCTTCCACAGAC | 1 | 1 | GTCTGTGGAAGCG | 47 |
| 1139 | 1151 | 13 | GTCAGCGGCCGGG | 1 | 1 | CCCGGCCGCTGAC | 48 |
| 1224 | 1236 | 13 | GCGGCACCCTCAT | 1 | 1 | ATGAGGGTGCCGC | 49 |
| 1228 | 1240 | 13 | CACCCTCATAGGC | 1 | 1 | GCCTATGAGGGTG | 50 |
| 1232 | 1244 | 13 | CTCATAGGCCTGG | 1 | 1 | CCAGGCCTATGAG | 51 |
| 1235 | 1247 | 13 | ATAGGCCTGGAGT | 1 | 1 | ACTCCAGGCCTAT | 52 |
| 1238 | 1250 | 13 | GGCCTGGAGTTTA | 1 | 1 | TAAACTCCAGGCC | 53 |
| 1239 | 1251 | 13 | GCCTGGAGTTTAT | 1 | 1 | ATAAACTCCAGGC | 54 |
| 1826 | 1838 | 13 | GCACACTCGGGGC | 1 | 1 | GCCCCGAGTGTGC | 55 |
| 1989 | 2001 | 13 | GTGAGGGTGTCTA | 1 | 1 | TAGACACCCTCAC | 56 |
| 844 | 856 | 13 | GAAGTTGCCCCAT | 1 | 1 | ATGGGGCAACTTC | 57 |
| 979 | 991 | 13 | GGAGGTGTATCTC | 1 | 1 | GAGATACACCTCC | 58 |
| 1233 | 1245 | 13 | TCATAGGCCTGGA | 1 | 2 | TCCAGGCCTATGA | 59 |
| 1827 | 1839 | 13 | CACACTCGGGGCC | 1 | 2 | GGCCCCGAGTGTG | 60 |
| 1231 | 1243 | 13 | CCTCATAGGCCTG | 1 | 3 | CAGGCCTATGAGG | 61 |
| 978 | 990 | 13 | TGGAGGTGTATCT | 1 | 3 | AGATACACCTCCA | 62 |
| 1603 | 1615 | 13 | TGCTGCCCACGTG | 1 | 4 | CACGTGGGCAGCA | 63 |
| 1100 | 1112 | 13 | AGCAAGTGTGACA | 2 | 1 | TGTCACACTTGCT | 64 |
| 1140 | 1152 | 13 | TCAGCGGCCGGGA | 2 | 1 | TCCCGGCCGCTGA | 65 |
| 1225 | 1237 | 13 | CGGCACCCTCATA | 2 | 1 | TATGAGGGTGCCG | 66 |
| 1226 | 1238 | 13 | GGCACCCTCATAG | 2 | 1 | CTATGAGGGTGCC | 67 |
| 1227 | 1239 | 13 | GCACCCTCATAGG | 2 | 1 | CCTATGAGGGTGC | 68 |
| 1229 | 1241 | 13 | ACCCTCATAGGCC | 2 | 1 | GGCCTATGAGGGT | 69 |
| 1230 | 1242 | 13 | CCCTCATAGGCCT | 2 | 1 | AGGCCTATGAGGG | 70 |
| 1234 | 1246 | 13 | CATAGGCCTGGAG | 2 | 1 | CTCCAGGCCTATG | 71 |
| 1244 | 1256 | 13 | GAGTTTATTCGGA | 2 | 1 | TCCGAATAAACTC | 72 |
| 1388 | 1400 | 13 | AACTTCCGGGACG | 2 | 1 | CGTCCCGGAAGTT | 73 |
| 2929 | 2941 | 13 | GGCTCCCTGATTA | 2 | 1 | TAATCAGGGAGCC | 74 |
| 843 | 855 | 13 | TGAAGTTGCCCCA | 2 | 1 | TGGGGCAACTTCA | 75 |
| 845 | 857 | 13 | AAGTTGCCCCATG | 2 | 1 | CATGGGGCAACTT | 76 |
| 1137 | 1149 | 13 | TGGTCAGCGGCCG | 2 | 2 | CGGCCGCTGACCA | 77 |
| 1138 | 1150 | 13 | GGTCAGCGGCCGG | 2 | 2 | CCGGCCGCTGACC | 78 |
| 1243 | 1255 | 13 | GGAGTTTATTCGG | 2 | 2 | CCGAATAAACTCC | 79 |
| 1581 | 1593 | 13 | CACAGAGTGGGAC | 2 | 2 | GTCCCACTCTGTG | 80 |
| 1747 | 1759 | 13 | GACCCCCAACCTG | 2 | 2 | CAGGTTGGGGGTC | 81 |
| 2466 | 2478 | 13 | CCATCTGCTGCCG | 2 | 2 | CGGCAGCAGATGG | 82 |
| 1986 | 1998 | 13 | GGGGTGAGGGTGT | 2 | 3 | ACACCCTCACCCC | 83 |
| 2468 | 2480 | 13 | ATCTGCTGCCGGA | 2 | 3 | TCCGGCAGCAGAT | 84 |
| 976 | 988 | 13 | GGTGGAGGTGTAT | 2 | 3 | ATACACCTCCACC | 85 |
| 1085 | 1097 | 13 | TTCCACAGACAGG | 2 | 4 | CCTGTCTGTGGAA | 86 |
| 1086 | 1098 | 13 | TCCACAGACAGGC | 2 | 4 | GCCTGTCTGTGGA | 87 |
| 1245 | 1257 | 13 | AGTTTATTCGGAA | 2 | 4 | TTCCGAATAAACT | 88 |
| 1434 | 1446 | 13 | AGGTCATCACAGT | 2 | 4 | ACTGTGATGACCT | 89 |
| 1389 | 1401 | 13 | ACTTCCGGGACGA | 2 | 5 | TCGTCCCGGAAGT | 90 |
| 1580 | 1592 | 13 | TCACAGAGTGGGA | 2 | 6 | TCCCACTCTGTGA | 91 |
| 1240 | 1252 | 13 | CCTGGAGTTTATT | 3 | 1 | AATAAACTCCAGG | 92 |
| 1410 | 1422 | 13 | TCTACTCCCCAGC | 3 | 1 | GCTGGGGAGTAGA | 93 |
| 2930 | 2942 | 13 | GCTCCCTGATTAA | 3 | 1 | TTAATCAGGGAGC | 94 |
| 1082 | 1095 | 14 | CGCTTCCACAGACA | 1 | 1 | TGTCTGTGGAAGCG | 95 |
| 1084 | 1097 | 14 | CTTCCACAGACAGG | 1 | 1 | CCTGTCTGTGGAAG | 96 |
| 1100 | 1113 | 14 | AGCAAGTGTGACAG | 1 | 1 | CTGTCACACTTGCT | 97 |
| 1136 | 1149 | 14 | GTGGTCAGCGGCCG | 1 | 1 | CGGCCGCTGACCAC | 98 |
| 1138 | 1151 | 14 | GGTCAGCGGCCGGG | 1 | 1 | CCCGGCCGCTGACC | 99 |
| 1139 | 1152 | 14 | GTCAGCGGCCGGGA | 1 | 1 | TCCCGGCCGCTGAC | 100 |
| 1140 | 1153 | 14 | TCAGCGGCCGGGAT | 1 | 1 | ATCCCGGCCGCTGA | 101 |
| 1223 | 1236 | 14 | AGCGGCACCCTCAT | 1 | 1 | ATGAGGGTGCCGCT | 102 |
| 1224 | 1237 | 14 | GCGGCACCCTCATA | 1 | 1 | TATGAGGGTGCCGC | 103 |
| 1227 | 1240 | 14 | GCACCCTCATAGGC | 1 | 1 | GCCTATGAGGGTGC | 104 |
| 1228 | 1241 | 14 | CACCCTCATAGGCC | 1 | 1 | GGCCTATGAGGGTG | 105 |
| 1230 | 1243 | 14 | CCCTCATAGGCCTG | 1 | 1 | CAGGCCTATGAGGG | 106 |
| 1231 | 1244 | 14 | CCTCATAGGCCTGG | 1 | 1 | CCAGGCCTATGAGG | 107 |
| 1232 | 1245 | 14 | CTCATAGGCCTGGA | 1 | 1 | TCCAGGCCTATGAG | 108 |
| 1233 | 1246 | 14 | TCATAGGCCTGGAG | 1 | 1 | CTCCAGGCCTATGA | 109 |
| 1234 | 1247 | 14 | CATAGGCCTGGAGT | 1 | 1 | ACTCCAGGCCTATG | 110 |
| 1235 | 1248 | 14 | ATAGGCCTGGAGTT | 1 | 1 | AACTCCAGGCCTAT | 111 |
| 1236 | 1249 | 14 | TAGGCCTGGAGTTT | 1 | 1 | AAACTCCAGGCCTA | 112 |
| 1237 | 1250 | 14 | AGGCCTGGAGTTTA | 1 | 1 | TAAACTCCAGGCCT | 113 |
| 1238 | 1251 | 14 | GGCCTGGAGTTTAT | 1 | 1 | ATAAACTCCAGGCC | 114 |
| 1239 | 1252 | 14 | GCCTGGAGTTTATT | 1 | 1 | AATAAACTCCAGGC | 115 |
| 1244 | 1257 | 14 | GAGTTTATTCGGAA | 1 | 1 | TTCCGAATAAACTC | 116 |
| 1388 | 1401 | 14 | AACTTCCGGGACGA | 1 | 1 | TCGTCCCGGAAGTT | 117 |
| 1403 | 1416 | 14 | GCCTGCCTCTACTC | 1 | 1 | GAGTAGAGGCAGGC | 118 |
| 1406 | 1419 | 14 | TGCCTCTACTCCCC | 1 | 1 | GGGGAGTAGAGGCA | 119 |
| 1409 | 1422 | 14 | CTCTACTCCCCAGC | 1 | 1 | GCTGGGGAGTAGAG | 120 |
| 1433 | 1446 | 14 | GAGGTCATCACAGT | 1 | 1 | ACTGTGATGACCTC | 121 |
| 1580 | 1593 | 14 | TCACAGAGTGGGAC | 1 | 1 | GTCCCACTCTGTGA | 122 |
| 1747 | 1760 | 14 | GACCCCCAACCTGG | 1 | 1 | CCAGGTTGGGGGTC | 123 |
| 1826 | 1839 | 14 | GCACACTCGGGGCC | 1 | 1 | GGCCCCGAGTGTGC | 124 |
| 1985 | 1998 | 14 | GGGGGTGAGGGTGT | 1 | 1 | ACACCCTCACCCCC | 125 |
| 1986 | 1999 | 14 | GGGGTGAGGGTGTC | 1 | 1 | GACACCCTCACCCC | 126 |
| 1988 | 2001 | 14 | GGTGAGGGTGTCTA | 1 | 1 | TAGACACCCTCACC | 127 |
| 2237 | 2250 | 14 | TGCTGCCATGCCCC | 1 | 1 | GGGGCATGGCAGCA | 128 |
| 2465 | 2478 | 14 | GCCATCTGCTGCCG | 1 | 1 | CGGCAGCAGATGGC | 129 |
| 2466 | 2479 | 14 | CCATCTGCTGCCGG | 1 | 1 | CCGGCAGCAGATGG | 130 |
| 2469 | 2482 | 14 | TCTGCTGCCGGAGC | 1 | 1 | GCTCCGGCAGCAGA | 131 |
| 2928 | 2941 | 14 | GGGCTCCCTGATTA | 1 | 1 | TAATCAGGGAGCCC | 132 |
| 2929 | 2942 | 14 | GGCTCCCTGATTAA | 1 | 1 | TTAATCAGGGAGCC | 133 |
| 843 | 856 | 14 | TGAAGTTGCCCCAT | 1 | 1 | ATGGGGCAACTTCA | 134 |
| 844 | 857 | 14 | GAAGTTGCCCCATG | 1 | 1 | CATGGGGCAACTTC | 135 |
| 976 | 989 | 14 | GGTGGAGGTGTATC | 1 | 1 | GATACACCTCCACC | 136 |
| 977 | 990 | 14 | GTGGAGGTGTATCT | 1 | 1 | AGATACACCTCCAC | 137 |
| 978 | 991 | 14 | TGGAGGTGTATCTC | 1 | 1 | GAGATACACCTCCA | 138 |
| 1083 | 1096 | 14 | GCTTCCACAGACAG | 1 | 2 | CTGTCTGTGGAAGC | 139 |
| 1085 | 1098 | 14 | TTCCACAGACAGGC | 1 | 2 | GCCTGTCTGTGGAA | 140 |
| 1601 | 1614 | 14 | GCTGCTGCCCACGT | 1 | 2 | ACGTGGGCAGCAGC | 141 |
| 1721 | 1734 | 14 | TGGTTCCCTGAGGA | 1 | 2 | TCCTCAGGGAACCA | 142 |
| 2234 | 2247 | 14 | TCCTGCTGCCATGC | 1 | 2 | GCATGGCAGCAGGA | 143 |
| 2468 | 2481 | 14 | ATCTGCTGCCGGAG | 1 | 2 | CTCCGGCAGCAGAT | 144 |
| 1602 | 1615 | 14 | CTGCTGCCCACGTG | 1 | 3 | CACGTGGGCAGCAG | 145 |
| 1603 | 1616 | 14 | TGCTGCCCACGTGG | 1 | 3 | CCACGTGGGCAGCA | 146 |
| 1887 | 1900 | 14 | TGCTGAGCTGCTCC | 1 | 3 | GGAGCAGCTCAGCA | 147 |
| 1886 | 1899 | 14 | CTGCTGAGCTGCTC | 1 | 4 | GAGCAGCTCAGCAG | 148 |
| 1773 | 1786 | 14 | CCCCCAGCACCCAT | 1 | 5 | ATGGGTGCTGGGGG | 149 |
| 1137 | 1150 | 14 | TGGTCAGCGGCCGG | 2 | 1 | CCGGCCGCTGACCA | 150 |
| 1141 | 1154 | 14 | CAGCGGCCGGGATG | 2 | 1 | CATCCCGGCCGCTG | 151 |
| 1225 | 1238 | 14 | CGGCACCCTCATAG | 2 | 1 | CTATGAGGGTGCCG | 152 |
| 1226 | 1239 | 14 | GGCACCCTCATAGG | 2 | 1 | CCTATGAGGGTGCC | 153 |
| 1229 | 1242 | 14 | ACCCTCATAGGCCT | 2 | 1 | AGGCCTATGAGGGT | 154 |
| 1240 | 1253 | 14 | CCTGGAGTTTATTC | 2 | 1 | GAATAAACTCCAGG | 155 |
| 1241 | 1254 | 14 | CTGGAGTTTATTCG | 2 | 1 | CGAATAAACTCCAG | 156 |
| 1243 | 1256 | 14 | GGAGTTTATTCGGA | 2 | 1 | TCCGAATAAACTCC | 157 |
| 1483 | 1496 | 14 | GGGGACTTTGGGGA | 2 | 1 | TCCCCAAAGTCCCC | 158 |
| 1578 | 1591 | 14 | TGTCACAGAGTGGG | 2 | 1 | CCCACTCTGTGACA | 159 |
| 1683 | 1696 | 14 | TGATCCACTTCTCT | 2 | 1 | AGAGAAGTGGATCA | 160 |
| 1718 | 1731 | 14 | GCCTGGTTCCCTGA | 2 | 1 | TCAGGGAACCAGGC | 161 |
| 1748 | 1761 | 14 | ACCCCCAACCTGGT | 2 | 1 | ACCAGGTTGGGGGT | 162 |
| 1983 | 1996 | 14 | TTGGGGGTGAGGGT | 2 | 1 | ACCCTCACCCCCAA | 163 |
| 2086 | 2099 | 14 | TGTCCACTGCCACC | 2 | 1 | GGTGGCAGTGGACA | 164 |
| 2087 | 2100 | 14 | GTCCACTGCCACCA | 2 | 1 | TGGTGGCAGTGGAC | 165 |
| 2240 | 2253 | 14 | TGCCATGCCCCAGG | 2 | 1 | CCTGGGGCATGGCA | 166 |
| 3435 | 3448 | 14 | CTTTTGTAACTTGA | 2 | 1 | TCAAGTTACAAAAG | 167 |
| 742 | 755 | 14 | GGCTGCCCGCCGGG | 2 | 1 | CCCGGCGGGCAGCC | 168 |
| 845 | 858 | 14 | AAGTTGCCCCATGT | 2 | 1 | ACATGGGGCAACTT | 169 |
| 1205 | 1218 | 14 | CAAGGGAAGGGCAC | 2 | 2 | GTGCCCTTCCCTTG | 170 |
| 1242 | 1255 | 14 | TGGAGTTTATTCGG | 2 | 2 | CCGAATAAACTCCA | 171 |
| 1408 | 1421 | 14 | CCTCTACTCCCCAG | 2 | 2 | CTGGGGAGTAGAGG | 172 |
| 1579 | 1592 | 14 | GTCACAGAGTGGGA | 2 | 2 | TCCCACTCTGTGAC | 173 |
| 1599 | 1612 | 14 | AGGCTGCTGCCCAC | 2 | 2 | GTGGGCAGCAGCCT | 174 |
| 1682 | 1695 | 14 | CTGATCCACTTCTC | 2 | 2 | GAGAAGTGGATCAG | 175 |
| 1722 | 1735 | 14 | GGTTCCCTGAGGAC | 2 | 2 | GTCCTCAGGGAACC | 176 |
| 1746 | 1759 | 14 | TGACCCCCAACCTG | 2 | 2 | CAGGTTGGGGGTCA | 177 |
| 1982 | 1995 | 14 | TTTGGGGGTGAGGG | 2 | 2 | CCCTCACCCCCAAA | 178 |
| 2235 | 2248 | 14 | CCTGCTGCCATGCC | 2 | 2 | GGCATGGCAGCAGG | 179 |
| 2238 | 2251 | 14 | GCTGCCATGCCCCA | 2 | 2 | TGGGGCATGGCAGC | 180 |
| 2467 | 2480 | 14 | CATCTGCTGCCGGA | 2 | 2 | TCCGGCAGCAGATG | 181 |
| 3434 | 3447 | 14 | GCTTTTGTAACTTG | 2 | 2 | CAAGTTACAAAAGC | 182 |
| 890 | 903 | 14 | GCCCAGAGCATCCC | 2 | 2 | GGGATGCTCTGGGC | 183 |
| 905 | 918 | 14 | TGGAACCTGGAGCG | 2 | 2 | CGCTCCAGGTTCCA | 184 |
| 1597 | 1610 | 14 | ACAGGCTGCTGCCC | 2 | 3 | GGGCAGCAGCCTGT | 185 |
| 2233 | 2246 | 14 | TTCCTGCTGCCATG | 2 | 3 | CATGGCAGCAGGAA | 186 |
| 1774 | 1787 | 14 | CCCCAGCACCCATG | 2 | 7 | CATGGGTGCTGGGG | 187 |
| 1142 | 1155 | 14 | AGCGGCCGGGATGC | 3 | 1 | GCATCCCGGCCGCT | 188 |
| 1604 | 1617 | 14 | GCTGCCCACGTGGC | 3 | 1 | GCCACGTGGGCAGC | 189 |
| 1987 | 2000 | 14 | GGGTGAGGGTGTCT | 3 | 1 | AGACACCCTCACCC | 190 |
| 1082 | 1096 | 15 | CGCTTCCACAGACAG | 1 | 1 | CTGTCTGTGGAAGCG | 191 |
| 1083 | 1097 | 15 | GCTTCCACAGACAGG | 1 | 1 | CCTGTCTGTGGAAGC | 192 |
| 1084 | 1098 | 15 | CTTCCACAGACAGGC | 1 | 1 | GCCTGTCTGTGGAAG | 193 |
| 1136 | 1150 | 15 | GTGGTCAGCGGCCGG | 1 | 1 | CCGGCCGCTGACCAC | 194 |
| 1137 | 1151 | 15 | TGGTCAGCGGCCGGG | 1 | 1 | CCCGGCCGCTGACCA | 195 |
| 1138 | 1152 | 15 | GGTCAGCGGCCGGGA | 1 | 1 | TCCCGGCCGCTGACC | 196 |
| 1139 | 1153 | 15 | GTCAGCGGCCGGGAT | 1 | 1 | ATCCCGGCCGCTGAC | 197 |
| 1140 | 1154 | 15 | TCAGCGGCCGGGATG | 1 | 1 | CATCCCGGCCGCTGA | 198 |
| 1223 | 1237 | 15 | AGCGGCACCCTCATA | 1 | 1 | TATGAGGGTGCCGCT | 199 |
| 1224 | 1238 | 15 | GCGGCACCCTCATAG | 1 | 1 | CTATGAGGGTGCCGC | 200 |
| 1226 | 1240 | 15 | GGCACCCTCATAGGC | 1 | 1 | GCCTATGAGGGTGCC | 201 |
| 1227 | 1241 | 15 | GCACCCTCATAGGCC | 1 | 1 | GGCCTATGAGGGTGC | 202 |
| 1228 | 1242 | 15 | CACCCTCATAGGCCT | 1 | 1 | AGGCCTATGAGGGTG | 203 |
| 1229 | 1243 | 15 | ACCCTCATAGGCCTG | 1 | 1 | CAGGCCTATGAGGGT | 204 |
| 1230 | 1244 | 15 | CCCTCATAGGCCTGG | 1 | 1 | CCAGGCCTATGAGGG | 205 |
| 1231 | 1245 | 15 | CCTCATAGGCCTGGA | 1 | 1 | TCCAGGCCTATGAGG | 206 |
| 1232 | 1246 | 15 | CTCATAGGCCTGGAG | 1 | 1 | CTCCAGGCCTATGAG | 207 |
| 1233 | 1247 | 15 | TCATAGGCCTGGAGT | 1 | 1 | ACTCCAGGCCTATGA | 208 |
| 1234 | 1248 | 15 | CATAGGCCTGGAGTT | 1 | 1 | AACTCCAGGCCTATG | 209 |
| 1235 | 1249 | 15 | ATAGGCCTGGAGTTT | 1 | 1 | AAACTCCAGGCCTAT | 210 |
| 1236 | 1250 | 15 | TAGGCCTGGAGTTTA | 1 | 1 | TAAACTCCAGGCCTA | 211 |
| 1237 | 1251 | 15 | AGGCCTGGAGTTTAT | 1 | 1 | ATAAACTCCAGGCCT | 212 |
| 1238 | 1252 | 15 | GGCCTGGAGTTTATT | 1 | 1 | AATAAACTCCAGGCC | 213 |
| 1239 | 1253 | 15 | GCCTGGAGTTTATTC | 1 | 1 | GAATAAACTCCAGGC | 214 |
| 1240 | 1254 | 15 | CCTGGAGTTTATTCG | 1 | 1 | CGAATAAACTCCAGG | 215 |
| 1243 | 1257 | 15 | GGAGTTTATTCGGAA | 1 | 1 | TTCCGAATAAACTCC | 216 |
| 1403 | 1417 | 15 | GCCTGCCTCTACTCC | 1 | 1 | GGAGTAGAGGCAGGC | 217 |
| 1405 | 1419 | 15 | CTGCCTCTACTCCCC | 1 | 1 | GGGGAGTAGAGGCAG | 218 |
| 1406 | 1420 | 15 | TGCCTCTACTCCCCA | 1 | 1 | TGGGGAGTAGAGGCA | 219 |
| 1407 | 1421 | 15 | GCCTCTACTCCCCAG | 1 | 1 | CTGGGGAGTAGAGGC | 220 |
| 1408 | 1422 | 15 | CCTCTACTCCCCAGC | 1 | 1 | GCTGGGGAGTAGAGG | 221 |
| 1483 | 1497 | 15 | GGGGACTTTGGGGAC | 1 | 1 | GTCCCCAAAGTCCCC | 222 |
| 1579 | 1593 | 15 | GTCACAGAGTGGGAC | 1 | 1 | GTCCCACTCTGTGAC | 223 |
| 1603 | 1617 | 15 | TGCTGCCCACGTGGC | 1 | 1 | GCCACGTGGGCAGCA | 224 |
| 1682 | 1696 | 15 | CTGATCCACTTCTCT | 1 | 1 | AGAGAAGTGGATCAG | 225 |
| 1718 | 1732 | 15 | GCCTGGTTCCCTGAG | 1 | 1 | CTCAGGGAACCAGGC | 226 |
| 1721 | 1735 | 15 | TGGTTCCCTGAGGAC | 1 | 1 | GTCCTCAGGGAACCA | 227 |
| 1745 | 1759 | 15 | CTGACCCCCAACCTG | 1 | 1 | CAGGTTGGGGGTCAG | 228 |
| 1746 | 1760 | 15 | TGACCCCCAACCTGG | 1 | 1 | CCAGGTTGGGGGTCA | 229 |
| 1747 | 1761 | 15 | GACCCCCAACCTGGT | 1 | 1 | ACCAGGTTGGGGGTC | 230 |
| 1772 | 1786 | 15 | CCCCCCAGCACCCAT | 1 | 1 | ATGGGTGCTGGGGGG | 231 |
| 1887 | 1901 | 15 | TGCTGAGCTGCTCCA | 1 | 1 | TGGAGCAGCTCAGCA | 232 |
| 1982 | 1996 | 15 | TTTGGGGGTGAGGGT | 1 | 1 | ACCCTCACCCCCAAA | 233 |
| 1983 | 1997 | 15 | TTGGGGGTGAGGGTG | 1 | 1 | CACCCTCACCCCCAA | 234 |
| 1984 | 1998 | 15 | TGGGGGTGAGGGTGT | 1 | 1 | ACACCCTCACCCCCA | 235 |
| 1985 | 1999 | 15 | GGGGGTGAGGGTGTC | 1 | 1 | GACACCCTCACCCCC | 236 |
| 1986 | 2000 | 15 | GGGGTGAGGGTGTCT | 1 | 1 | AGACACCCTCACCCC | 237 |
| 1987 | 2001 | 15 | GGGTGAGGGTGTCTA | 1 | 1 | TAGACACCCTCACCC | 238 |
| 2233 | 2247 | 15 | TTCCTGCTGCCATGC | 1 | 1 | GCATGGCAGCAGGAA | 239 |
| 2234 | 2248 | 15 | TCCTGCTGCCATGCC | 1 | 1 | GGCATGGCAGCAGGA | 240 |
| 2236 | 2250 | 15 | CTGCTGCCATGCCCC | 1 | 1 | GGGGCATGGCAGCAG | 241 |
| 2237 | 2251 | 15 | TGCTGCCATGCCCCA | 1 | 1 | TGGGGCATGGCAGCA | 242 |
| 2238 | 2252 | 15 | GCTGCCATGCCCCAG | 1 | 1 | CTGGGGCATGGCAGC | 243 |
| 2464 | 2478 | 15 | TGCCATCTGCTGCCG | 1 | 1 | CGGCAGCAGATGGCA | 244 |
| 2465 | 2479 | 15 | GCCATCTGCTGCCGG | 1 | 1 | CCGGCAGCAGATGGC | 245 |
| 2466 | 2480 | 15 | CCATCTGCTGCCGGA | 1 | 1 | TCCGGCAGCAGATGG | 246 |
| 2467 | 2481 | 15 | CATCTGCTGCCGGAG | 1 | 1 | CTCCGGCAGCAGATG | 247 |
| 2468 | 2482 | 15 | ATCTGCTGCCGGAGC | 1 | 1 | GCTCCGGCAGCAGAT | 248 |
| 2469 | 2483 | 15 | TCTGCTGCCGGAGCC | 1 | 1 | GGCTCCGGCAGCAGA | 249 |
| 2928 | 2942 | 15 | GGGCTCCCTGATTAA | 1 | 1 | TTAATCAGGGAGCCC | 250 |
| 3434 | 3448 | 15 | GCTTTTGTAACTTGA | 1 | 1 | TCAAGTTACAAAAGC | 251 |
| 843 | 857 | 15 | TGAAGTTGCCCCATG | 1 | 1 | CATGGGGCAACTTCA | 252 |
| 844 | 858 | 15 | GAAGTTGCCCCATGT | 1 | 1 | ACATGGGGCAACTTC | 253 |
| 976 | 990 | 15 | GGTGGAGGTGTATCT | 1 | 1 | AGATACACCTCCACC | 254 |
| 977 | 991 | 15 | GTGGAGGTGTATCTC | 1 | 1 | GAGATACACCTCCAC | 255 |
| 1597 | 1611 | 15 | ACAGGCTGCTGCCCA | 1 | 2 | TGGGCAGCAGCCTGT | 256 |
| 1600 | 1614 | 15 | GGCTGCTGCCCACGT | 1 | 2 | ACGTGGGCAGCAGCC | 257 |
| 1601 | 1615 | 15 | GCTGCTGCCCACGTG | 1 | 2 | CACGTGGGCAGCAGC | 258 |
| 1720 | 1734 | 15 | CTGGTTCCCTGAGGA | 1 | 2 | TCCTCAGGGAACCAG | 259 |
| 1773 | 1787 | 15 | CCCCCAGCACCCATG | 1 | 2 | CATGGGTGCTGGGGG | 260 |
| 1883 | 1897 | 15 | GAGCTGCTGAGCTGC | 1 | 2 | GCAGCTCAGCAGCTC | 261 |
| 1885 | 1899 | 15 | GCTGCTGAGCTGCTC | 1 | 2 | GAGCAGCTCAGCAGC | 262 |
| 1886 | 1900 | 15 | CTGCTGAGCTGCTCC | 1 | 2 | GGAGCAGCTCAGCAG | 263 |
| 1602 | 1616 | 15 | CTGCTGCCCACGTGG | 1 | 3 | CCACGTGGGCAGCAG | 264 |
| 1725 | 1739 | 15 | TCCCTGAGGACCAGC | 1 | 3 | GCTGGTCCTCAGGGA | 265 |
| 1771 | 1785 | 15 | GCCCCCCAGCACCCA | 1 | 3 | TGGGTGCTGGGGGGC | 266 |
| 1120 | 1134 | 15 | CACCCACCTGGCAGG | 2 | 1 | CCTGCCAGGTGGGTG | 267 |
| 1141 | 1155 | 15 | CAGCGGCCGGGATGC | 2 | 1 | GCATCCCGGCCGCTG | 268 |
| 1225 | 1239 | 15 | CGGCACCCTCATAGG | 2 | 1 | CCTATGAGGGTGCCG | 269 |
| 1241 | 1255 | 15 | CTGGAGTTTATTCGG | 2 | 1 | CCGAATAAACTCCAG | 270 |
| 1242 | 1256 | 15 | TGGAGTTTATTCGGA | 2 | 1 | TCCGAATAAACTCCA | 271 |
| 1482 | 1496 | 15 | TGGGGACTTTGGGGA | 2 | 1 | TCCCCAAAGTCCCCA | 272 |
| 1578 | 1592 | 15 | TGTCACAGAGTGGGA | 2 | 1 | TCCCACTCTGTGACA | 273 |
| 1595 | 1609 | 15 | TCACAGGCTGCTGCC | 2 | 1 | GGCAGCAGCCTGTGA | 274 |
| 1599 | 1613 | 15 | AGGCTGCTGCCCACG | 2 | 1 | CGTGGGCAGCAGCCT | 275 |
| 1717 | 1731 | 15 | GGCCTGGTTCCCTGA | 2 | 1 | TCAGGGAACCAGGCC | 276 |
| 1719 | 1733 | 15 | CCTGGTTCCCTGAGG | 2 | 1 | CCTCAGGGAACCAGG | 277 |
| 1748 | 1762 | 15 | ACCCCCAACCTGGTG | 2 | 1 | CACCAGGTTGGGGGT | 278 |
| 2086 | 2100 | 15 | TGTCCACTGCCACCA | 2 | 1 | TGGTGGCAGTGGACA | 279 |
| 2232 | 2246 | 15 | CTTCCTGCTGCCATG | 2 | 1 | CATGGCAGCAGGAAG | 280 |
| 2235 | 2249 | 15 | CCTGCTGCCATGCCC | 2 | 1 | GGGCATGGCAGCAGG | 281 |
| 2239 | 2253 | 15 | CTGCCATGCCCCAGG | 2 | 1 | CCTGGGGCATGGCAG | 282 |
| 2472 | 2486 | 15 | GCTGCCGGAGCCGGC | 2 | 1 | GCCGGCTCCGGCAGC | 283 |
| 742 | 756 | 15 | GGCTGCCCGCCGGGG | 2 | 1 | CCCCGGCGGGCAGCC | 284 |
| 1119 | 1133 | 15 | GCACCCACCTGGCAG | 2 | 2 | CTGCCAGGTGGGTGC | 285 |
| 1596 | 1610 | 15 | CACAGGCTGCTGCCC | 2 | 2 | GGGCAGCAGCCTGTG | 286 |
| 1598 | 1612 | 15 | CAGGCTGCTGCCCAC | 2 | 2 | GTGGGCAGCAGCCTG | 287 |
| 1722 | 1736 | 15 | GGTTCCCTGAGGACC | 2 | 2 | GGTCCTCAGGGAACC | 288 |
| 1723 | 1737 | 15 | GTTCCCTGAGGACCA | 2 | 2 | TGGTCCTCAGGGAAC | 289 |
| 1750 | 1764 | 15 | CCCCAACCTGGTGGC | 2 | 2 | GCCACCAGGTTGGGG | 290 |
| 1882 | 1896 | 15 | GGAGCTGCTGAGCTG | 2 | 2 | CAGCTCAGCAGCTCC | 291 |
| 2115 | 2129 | 15 | TCACAGGCTGCAGCT | 2 | 2 | AGCTGCAGCCTGTGA | 292 |
| 2471 | 2485 | 15 | TGCTGCCGGAGCCGG | 2 | 2 | CCGGCTCCGGCAGCA | 293 |
| 3433 | 3447 | 15 | TGCTTTTGTAACTTG | 2 | 2 | CAAGTTACAAAAGCA | 294 |
| 1404 | 1418 | 15 | CCTGCCTCTACTCCC | 2 | 3 | GGGAGTAGAGGCAGG | 295 |
| 1884 | 1898 | 15 | AGCTGCTGAGCTGCT | 2 | 3 | AGCAGCTCAGCAGCT | 296 |
| 2231 | 2245 | 15 | GCTTCCTGCTGCCAT | 2 | 3 | ATGGCAGCAGGAAGC | 297 |
| 1118 | 1132 | 15 | GGCACCCACCTGGCA | 3 | 1 | TGCCAGGTGGGTGCC | 298 |
| 1082 | 1097 | 16 | CGCTTCCACAGACAGG | 1 | 1 | CCTGTCTGTGGAAGCG | 299 |
| 1083 | 1098 | 16 | GCTTCCACAGACAGGC | 1 | 1 | GCCTGTCTGTGGAAGC | 300 |
| 1136 | 1151 | 16 | GTGGTCAGCGGCCGGG | 1 | 1 | CCCGGCCGCTGACCAC | 301 |
| 1137 | 1152 | 16 | TGGTCAGCGGCCGGGA | 1 | 1 | TCCCGGCCGCTGACCA | 302 |
| 1138 | 1153 | 16 | GGTCAGCGGCCGGGAT | 1 | 1 | ATCCCGGCCGCTGACC | 303 |
| 1139 | 1154 | 16 | GTCAGCGGCCGGGATG | 1 | 1 | CATCCCGGCCGCTGAC | 304 |
| 1140 | 1155 | 16 | TCAGCGGCCGGGATGC | 1 | 1 | GCATCCCGGCCGCTGA | 305 |
| 1223 | 1238 | 16 | AGCGGCACCCTCATAG | 1 | 1 | CTATGAGGGTGCCGCT | 306 |
| 1224 | 1239 | 16 | GCGGCACCCTCATAGG | 1 | 1 | CCTATGAGGGTGCCGC | 307 |
| 1225 | 1240 | 16 | CGGCACCCTCATAGGC | 1 | 1 | GCCTATGAGGGTGCCG | 308 |
| 1226 | 1241 | 16 | GGCACCCTCATAGGCC | 1 | 1 | GGCCTATGAGGGTGCC | 309 |
| 1227 | 1242 | 16 | GCACCCTCATAGGCCT | 1 | 1 | AGGCCTATGAGGGTGC | 310 |
| 1228 | 1243 | 16 | CACCCTCATAGGCCTG | 1 | 1 | CAGGCCTATGAGGGTG | 311 |
| 1229 | 1244 | 16 | ACCCTCATAGGCCTGG | 1 | 1 | CCAGGCCTATGAGGGT | 312 |
| 1230 | 1245 | 16 | CCCTCATAGGCCTGGA | 1 | 1 | TCCAGGCCTATGAGGG | 313 |
| 1231 | 1246 | 16 | CCTCATAGGCCTGGAG | 1 | 1 | CTCCAGGCCTATGAGG | 314 |
| 1232 | 1247 | 16 | CTCATAGGCCTGGAGT | 1 | 1 | ACTCCAGGCCTATGAG | 315 |
| 1233 | 1248 | 16 | TCATAGGCCTGGAGTT | 1 | 1 | AACTCCAGGCCTATGA | 316 |
| 1234 | 1249 | 16 | CATAGGCCTGGAGTTT | 1 | 1 | AAACTCCAGGCCTATG | 317 |
| 1235 | 1250 | 16 | ATAGGCCTGGAGTTTA | 1 | 1 | TAAACTCCAGGCCTAT | 318 |
| 1236 | 1251 | 16 | TAGGCCTGGAGTTTAT | 1 | 1 | ATAAACTCCAGGCCTA | 319 |
| 1237 | 1252 | 16 | AGGCCTGGAGTTTATT | 1 | 1 | AATAAACTCCAGGCCT | 320 |
| 1238 | 1253 | 16 | GGCCTGGAGTTTATTC | 1 | 1 | GAATAAACTCCAGGCC | 321 |
| 1239 | 1254 | 16 | GCCTGGAGTTTATTCG | 1 | 1 | CGAATAAACTCCAGGC | 322 |
| 1240 | 1255 | 16 | CCTGGAGTTTATTCGG | 1 | 1 | CCGAATAAACTCCAGG | 323 |
| 1242 | 1257 | 16 | TGGAGTTTATTCGGAA | 1 | 1 | TTCCGAATAAACTCCA | 324 |
| 1403 | 1418 | 16 | GCCTGCCTCTACTCCC | 1 | 1 | GGGAGTAGAGGCAGGC | 325 |
| 1404 | 1419 | 16 | CCTGCCTCTACTCCCC | 1 | 1 | GGGGAGTAGAGGCAGG | 326 |
| 1405 | 1420 | 16 | CTGCCTCTACTCCCCA | 1 | 1 | TGGGGAGTAGAGGCAG | 327 |
| 1406 | 1421 | 16 | TGCCTCTACTCCCCAG | 1 | 1 | CTGGGGAGTAGAGGCA | 328 |
| 1407 | 1422 | 16 | GCCTCTACTCCCCAGC | 1 | 1 | GCTGGGGAGTAGAGGC | 329 |
| 1482 | 1497 | 16 | TGGGGACTTTGGGGAC | 1 | 1 | GTCCCCAAAGTCCCCA | 330 |
| 1578 | 1593 | 16 | TGTCACAGAGTGGGAC | 1 | 1 | GTCCCACTCTGTGACA | 331 |
| 1595 | 1610 | 16 | TCACAGGCTGCTGCCC | 1 | 1 | GGGCAGCAGCCTGTGA | 332 |
| 1596 | 1611 | 16 | CACAGGCTGCTGCCCA | 1 | 1 | TGGGCAGCAGCCTGTG | 333 |
| 1597 | 1612 | 16 | ACAGGCTGCTGCCCAC | 1 | 1 | GTGGGCAGCAGCCTGT | 334 |
| 1599 | 1614 | 16 | AGGCTGCTGCCCACGT | 1 | 1 | ACGTGGGCAGCAGCCT | 335 |
| 1602 | 1617 | 16 | CTGCTGCCCACGTGGC | 1 | 1 | GCCACGTGGGCAGCAG | 336 |
| 1717 | 1732 | 16 | GGCCTGGTTCCCTGAG | 1 | 1 | CTCAGGGAACCAGGCC | 337 |
| 1718 | 1733 | 16 | GCCTGGTTCCCTGAGG | 1 | 1 | CCTCAGGGAACCAGGC | 338 |
| 1719 | 1734 | 16 | CCTGGTTCCCTGAGGA | 1 | 1 | TCCTCAGGGAACCAGG | 339 |
| 1720 | 1735 | 16 | CTGGTTCCCTGAGGAC | 1 | 1 | GTCCTCAGGGAACCAG | 340 |
| 1721 | 1736 | 16 | TGGTTCCCTGAGGACC | 1 | 1 | GGTCCTCAGGGAACCA | 341 |
| 1724 | 1739 | 16 | TTCCCTGAGGACCAGC | 1 | 1 | GCTGGTCCTCAGGGAA | 342 |
| 1745 | 1760 | 16 | CTGACCCCCAACCTGG | 1 | 1 | CCAGGTTGGGGGTCAG | 343 |
| 1746 | 1761 | 16 | TGACCCCCAACCTGGT | 1 | 1 | ACCAGGTTGGGGGTCA | 344 |
| 1747 | 1762 | 16 | GACCCCCAACCTGGTG | 1 | 1 | CACCAGGTTGGGGGTC | 345 |
| 1748 | 1763 | 16 | ACCCCCAACCTGGTGG | 1 | 1 | CCACCAGGTTGGGGGT | 346 |
| 1770 | 1785 | 16 | TGCCCCCCAGCACCCA | 1 | 1 | TGGGTGCTGGGGGGCA | 347 |
| 1771 | 1786 | 16 | GCCCCCCAGCACCCAT | 1 | 1 | ATGGGTGCTGGGGGGC | 348 |
| 1772 | 1787 | 16 | CCCCCCAGCACCCATG | 1 | 1 | CATGGGTGCTGGGGGG | 349 |
| 1882 | 1897 | 16 | GGAGCTGCTGAGCTGC | 1 | 1 | GCAGCTCAGCAGCTCC | 350 |
| 1883 | 1898 | 16 | GAGCTGCTGAGCTGCT | 1 | 1 | AGCAGCTCAGCAGCTC | 351 |
| 1884 | 1899 | 16 | AGCTGCTGAGCTGCTC | 1 | 1 | GAGCAGCTCAGCAGCT | 352 |
| 1885 | 1900 | 16 | GCTGCTGAGCTGCTCC | 1 | 1 | GGAGCAGCTCAGCAGC | 353 |
| 1886 | 1901 | 16 | CTGCTGAGCTGCTCCA | 1 | 1 | TGGAGCAGCTCAGCAG | 354 |
| 887 | 1902 | 16 | TGCTGAGCTGCTCCAG | 1 | 1 | CTGGAGCAGCTCAGCA | 355 |
| 1982 | 1997 | 16 | TTTGGGGGTGAGGGTG | 1 | 1 | CACCCTCACCCCCAAA | 356 |
| 1983 | 1998 | 16 | TTGGGGGTGAGGGTGT | 1 | 1 | ACACCCTCACCCCCAA | 357 |
| 1984 | 1999 | 16 | TGGGGGTGAGGGTGTC | 1 | 1 | GACACCCTCACCCCCA | 358 |
| 1985 | 2000 | 16 | GGGGGTGAGGGTGTCT | 1 | 1 | AGACACCCTCACCCCC | 359 |
| 1986 | 2001 | 16 | GGGGTGAGGGTGTCTA | 1 | 1 | TAGACACCCTCACCCC | 360 |
| 2231 | 2246 | 16 | GCTTCCTGCTGCCATG | 1 | 1 | CATGGCAGCAGGAAGC | 361 |
| 2232 | 2247 | 16 | CTTCCTGCTGCCATGC | 1 | 1 | GCATGGCAGCAGGAAG | 362 |
| 2233 | 2248 | 16 | TTCCTGCTGCCATGCC | 1 | 1 | GGCATGGCAGCAGGAA | 363 |
| 2234 | 2249 | 16 | TCCTGCTGCCATGCCC | 1 | 1 | GGGCATGGCAGCAGGA | 364 |
| 2235 | 2250 | 16 | CCTGCTGCCATGCCCC | 1 | 1 | GGGGCATGGCAGCAGG | 365 |
| 2236 | 2251 | 16 | CTGCTGCCATGCCCCA | 1 | 1 | TGGGGCATGGCAGCAG | 366 |
| 2237 | 2252 | 16 | TGCTGCCATGCCCCAG | 1 | 1 | CTGGGGCATGGCAGCA | 367 |
| 2238 | 2253 | 16 | GCTGCCATGCCCCAGG | 1 | 1 | CCTGGGGCATGGCAGC | 368 |
| 2464 | 2479 | 16 | TGCCATCTGCTGCCGG | 1 | 1 | CCGGCAGCAGATGGCA | 369 |
| 2465 | 2480 | 16 | GCCATCTGCTGCCGGA | 1 | 1 | TCCGGCAGCAGATGGC | 370 |
| 2466 | 2481 | 16 | CCATCTGCTGCCGGAG | 1 | 1 | CTCCGGCAGCAGATGG | 371 |
| 2467 | 2482 | 16 | CATCTGCTGCCGGAGC | 1 | 1 | GCTCCGGCAGCAGATG | 372 |
| 2468 | 2483 | 16 | ATCTGCTGCCGGAGCC | 1 | 1 | GGCTCCGGCAGCAGAT | 373 |
| 2469 | 2484 | 16 | TCTGCTGCCGGAGCCG | 1 | 1 | CGGCTCCGGCAGCAGA | 374 |
| 2471 | 2486 | 16 | TGCTGCCGGAGCCGGC | 1 | 1 | GCCGGCTCCGGCAGCA | 375 |
| 3432 | 3447 | 16 | TTGCTTTTGTAACTTG | 1 | 1 | CAAGTTACAAAAGCAA | 376 |
| 3433 | 3448 | 16 | TGCTTTTGTAACTTGA | 1 | 1 | TCAAGTTACAAAAGCA | 377 |
| 843 | 858 | 16 | TGAAGTTGCCCCATGT | 1 | 1 | ACATGGGGCAACTTCA | 378 |
| 976 | 991 | 16 | GGTGGAGGTGTATCTC | 1 | 1 | GAGATACACCTCCACC | 379 |
| 1600 | 1615 | 16 | GGCTGCTGCCCACGTG | 1 | 2 | CACGTGGGCAGCAGCC | 380 |
| 1601 | 1616 | 16 | GCTGCTGCCCACGTGG | 1 | 2 | CCACGTGGGCAGCAGC | 381 |
| 1722 | 1737 | 16 | GGTTCCCTGAGGACCA | 1 | 2 | TGGTCCTCAGGGAACC | 382 |
| 1118 | 1133 | 16 | GGCACCCACCTGGCAG | 2 | 1 | CTGCCAGGTGGGTGCC | 383 |
| 1119 | 1134 | 16 | GCACCCACCTGGCAGG | 2 | 1 | CCTGCCAGGTGGGTGC | 384 |
| 1241 | 1256 | 16 | CTGGAGTTTATTCGGA | 2 | 1 | TCCGAATAAACTCCAG | 385 |
| 1598 | 1613 | 16 | CAGGCTGCTGCCCACG | 2 | 1 | CGTGGGCAGCAGCCTG | 386 |
| 2114 | 2129 | 16 | CTCACAGGCTGCAGCT | 2 | 1 | AGCTGCAGCCTGTGAG | 387 |
| 2470 | 2485 | 16 | CTGCTGCCGGAGCCGG | 2 | 1 | CCGGCTCCGGCAGCAG | 388 |
| 1723 | 1738 | 16 | GTTCCCTGAGGACCAG | 2 | 2 | CTGGTCCTCAGGGAAC | 389 |
| 1749 | 1764 | 16 | CCCCCAACCTGGTGGC | 2 | 2 | GCCACCAGGTTGGGGG | 390 |
| 1750 | 1765 | 16 | CCCCAACCTGGTGGCC | 2 | 2 | GGCCACCAGGTTGGGG | 391 |
| 1880 | 1895 | 16 | GAGGAGCTGCTGAGCT | 2 | 2 | AGCTCAGCAGCTCCTC | 392 |
| 1881 | 1896 | 16 | AGGAGCTGCTGAGCTG | 2 | 2 | CAGCTCAGCAGCTCCT | 393 |

## Claims

1. An antisense oligonucleotide, capable of inhibiting the expression of human PCSK9, wherein said antisense oligonucleotide is 10-25 nucleobases in length and is complementary to a corresponding region of SEQ ID NO 2; wherein the antisense oligonucleotide is a gapmer oligonucleotide of formula A-B-C, wherein;
A consists or comprises of 1-6 nucleotide analogues and;
B consists or comprises between 4-12 consecutive DNA nucleobases, and;
C consists or comprises of 1-6 nucleotide analogues; wherein the nucleotide analogues of A and C raise the Tₘ of the oligonucleotide/target duplex.

2. The antisense oligonucleotide according to claim 1, wherein region B comprises of 6 - 12 consecutive DNA nucleotides.

3. The antisense oligonucleotide according to claim 1 or 2, wherein the nucleotide analogues of A and C are LNA units.

4. The antisense oligonucleotide according to claim 3 wherein the LNA units are beta-D-oxy LNA units.

5. The antisense oligonucleotide according to claim 3 or 4 which comprises 3 - 8 nucleotide LNA units.

6. The antisense oligonucleotide according to any one of claims 3 -5, wherein regions A and C consist of 3 LNA nucleotides and region B consists of 7, 8, 9 or 10 consecutive DNA nucleotides.

7. The antisense oligonucleotide according to any one of the preceding claims, wherein said antisense oligonucleotide is 10 - 16 nucleobases in length.

8. The antisense oligonucleotide according to any one of the preceding claims, wherein said antisense oligonucleotide is 12 - 14 nucleobases in length.

9. The antisense oligonucleotide according to any one of the preceding claims, wherein each internucleoside linkage group is a phosphorothioate group.

10. The antisense oligonucleotide, according to claim 1, wherein A (5' region) consists of 1, 2 or 3 LNA units; B (central domain) consists of 6-12 DNA units, and; C (3' region) consists of 1, 2 or 3 LNA units, and wherein each internucleoside linkage group is a phosphorothioate group.

11. The antisense oligonucleotide according to claim 10, wherein said antisense oligonucleotide is 12 - 14 nucleobases in length.

12. A conjugate comprising the antisense oligonucleotide according to any one of the preceding claims and at least one non-nucleotide or non-polynucleotide moiety covalently attached to said antisense oligonucleotide.

13. The conjugate according to claim 12, wherein the antisense oligonucleotide is as defined in any one of claims 3 - 11.

14. The conjugate according to claim 12, wherein the antisense oligonucleotide is as defined claim 11.

15. An antisense oligonucleotide, or conjugate thereof, capable of inhibiting the expression of human PCSK9, wherein said antisense oligonucleotide is 10-25 nucleobases in length and is complementary to a corresponding region of SEQ ID NO 2, for use as a medicament, wherein the antisense oligonucleotide is not a siRNA.

16. The antisense oligonucleotide for use according to claim 15, wherein the antisense oligonucleotide is as defined in any one of claims 1- 11, or a conjugate according to claims 12-14.

17. An antisense oligonucleotide, or conjugate thereof, capable of inhibiting the expression of human PCSK9, wherein said antisense oligonucleotide is 10-25 nucleobases in length and is complementary to a corresponding region of SEQ ID NO 2, for use in the treatment of hypercholesterolemia, or a hypercholesterolemia related disorder selected from the group consisting of atherosclerosis, hyperlipidemia, HDL/LDL cholesterol imbalance, dyslipidemias, e.g., familial combined hyperlipidemia (FCHL), acquired hyperlipidemia, statin-resistant hypercholesterolemia, coronary artery disease (CAD), and coronary heart disease (CHD), and; wherein the antisense oligonucleotide is not a siRNA.

18. The antisense oligonucleotide for use according to claim 17, wherein the antisense oligonucleotide is as defined in any one of claims 1- 11, or a conjugate according to claims 12 - 14.

19. The antisense oligonucleotide for use according to claim 17 or 18, wherein the antisense oligonucleotide is for use in the treatment of hypercholesterolemia.

20. The antisense oligonucleotide for use according to claim 17 or 18, wherein the antisense oligonucleotide is for use in the treatment of statin-resistant hypercholesterolemia.

21. The antisense oligonucleotide for use according to claim 17 or 18, wherein the antisense oligonucleotide is for use in the treatment of atherosclerosis.

22. The antisense oligonucleotide for use according to any one of claims 15 - 21, wherein the antisense oligonucleotide is for use in combination with statins.

23. A pharmaceutical composition comprising the antisense oligonucleotide or conjugate according to any one of claims 1- 22, and a pharmaceutically acceptable diluent, carrier or adjuvant.

24. Use of an antisense oligonucleotide, or conjugate thereof, capable of inhibiting the expression of human PCSK9, wherein said antisense oligonucleotide is 10- 25 nucleobases in length and is complementary to a corresponding region of SEQ ID NO 2, for the manufacture of a medicament for the treatment of hypercholesterolemia or a related disorder selected from the group consisting of atherosclerosis, hyperlipidemia, HDL/LDL cholesterol imbalance, dyslipidemias, e.g., familial combined hyperlipidemia (FCHL), acquired hyperlipidemia, statin-resistant hypercholesterolemia, coronary artery disease (CAD), and coronary heart disease (CHD), wherein the antisense oligonucleotide is not a siRNA.

25. The use according to claim 24, wherein the medicament is for use in combination with statins.

## Patentansprüche

1. Antisense-Oligonukleotid, das in der Lage ist, die Expression von humanem PCSK9 zu hemmen, wobei das Antisense-Oligonukleotid eine Länge von 10-25 Nukleobase aufweist und komplementär zu einer entsprechenden Region von SEQ ID NR. 2 ist; wobei das Antisense-Oligonukleotid ein Gapmer-Oligonukleotid der Formel A-B-C ist, wobei;
A aus 1-6 Nukleotidanalogen besteht oder dieser umfasst und
B aus 4-12 aufeinanderfolgenden DNA-Nukleobasen besteht oder dieser umfasst und
C aus 1-6 Nukleotidanalogen besteht oder dieser umfasst; wobei die Nukleotidanaloge von A und C die Tₘ des Oligonukleotid:Zielduplex erhöhen.

2. Antisense-Oligonukleotid nach Anspruch 1, wobei Region B 6-12 aufeinanderfolgenden DNA-Nukleotide umfasst.

3. Antisense-Oligonukleotid nach Anspruch 1 oder 2, wobei die Nukleotidanaloge von A und C LNA-Einheiten sind.

4. Antisense-Oligonukleotid nach Anspruch 3, wobei die LNA-Einheiten beta-D-Oxy-LNA-Einheiten sind.

5. Antisense-Oligonukleotid nach Anspruch 3 oder 4, das 3-8 Nukleotid-LNA-Einheiten umfasst.

6. Antisense-Oligonukleotid nach einem der Ansprüche 3-5, wobei Region A und C aus 3 LNA-Nukleotiden und Region B aus 7, 8, 9 oder 10 aufeinanderfolgenden DNA-Nukleotiden besteht.

7. Antisense-Oligonukleotid nach einem der vorhergehenden Ansprüche, wobei das Antisense-Oligonukleotid eine Länge von 10-16 Nukleobasen aufweist.

8. Antisense-Oligonukleotid nach einem der vorhergehenden Ansprüche, wobei das Antisense-Oligonukleotid eine Länge von 12-14 Nukleobasen aufweist.

9. Antisense-Oligonukleotid nach einem der vorhergehenden Ansprüche, wobei jede Inter-Nukleosid-Verbindungsgruppe eine Phosphorthioat-Gruppe ist.

10. Antisense-Oligonukleotid nach Anspruch 1, wobei A (5'-Region) aus 1, 2 oder 3 LNA-Einheiten besteht; B (zentrale Domäne) aus 6-12 DNA-Einheiten besteht und; C (3'-Region) aus 1, 2 oder 3 LNA-Einheiten besteht und wobei jede Inter-Nukleosid-Verbindungsgruppe eine Phosphorthioat-Gruppe ist.

11. Antisense-Oligonukleotid nach Anspruch 10, wobei das Antisense-Oligonukleotid eine Länge von 12-14 Nukleobasen aufweist.

12. Konjugat, umfassend das Antisense-Oligonukleotid nach einem der vorhergehenden Ansprüche und mindestens eine Nicht-Nukleotid- oder Nicht-Polynukleotid-Gruppe, die kovalent an das Antisense-Oligonukleotid gebunden ist.

13. Konjugat nach Anspruch 12, wobei das Antisense-Oligonukleotid wie in einem der Ansprüche 3-11 definiert ist.

14. Konjugat nach Anspruch 12, wobei das Antisense-Oligonukleotid wie in Anspruch11 definiert ist.

15. Antisense-Oligonukleotid oder Konjugat davon, das in der Lage ist, die Expression von humanem PCSK9 zu hemmen, wobei das Antisense-Oligonukleotid eine Länge von 10-25 Nukleobase aufweist und komplementär zu einer entsprechenden Region von SEQ ID NR. 2 ist, zur Verwendung als Arzneimittel, wobei das Antisense-Oligonukleotid kein siRNA ist.

16. Antisense-Oligonukleotid zur Verwendung nach Anspruch 15, wobei das Antisense-Oligonukleotid wie in einem der Ansprüche 1-11 definiert oder ein Konjugat nach Anspruch 12-14 ist.

17. Antisense-Oligonukleotid oder Konjugat davon, das in der Lage ist, die Expression von humanem PCSK9 zu hemmen, wobei das Antisense-Oligonukleotid eine Länge von 10-25 Nukleobase aufweist und komplementär zu einer entsprechenden Region von SEQ ID NR. 2 ist, zur Verwendung bei der Behandlung von Hypercholesterinämie oder einer mit Hypercholesterinämie verbundenen Krankheit, ausgewählt aus der Gruppe, bestehend aus Arteriosklerose, Hyperlipidämie, HDL/LDL-Cholesterin-Ungleichgewicht, Dyslipidämien, z. B. familiär kombinierte Hyperlipidämie (FCHL), erworbene Hyperlipidämie, Statin-resistente Hypercholesterinämie, Koronararterienerkrankung (CAD) und koronare Herzkrankheit (KHK) und wobei das Antisense-Oligonukleotid kein siRNA ist.

18. Antisense-Oligonukleotid zur Verwendung nach Anspruch 17, wobei das Antisense-Oligonukleotid wie in einem der Ansprüche 1-11 definiert oder ein Konjugat nach Anspruch 12-14 ist.

19. Antisense-Oligonukleotid zur Verwendung nach Anspruch 17 oder 18, wobei das Antisense-Oligonukleotid zur Verwendung bei der Behandlung von Hypercholesterinämie vorgesehen ist.

20. Antisense-Oligonukleotid zur Verwendung nach Anspruch 17 oder 18, wobei das Antisense-Oligonukleotid zur Verwendung bei der Behandlung von Statin-resistenter Hypercholesterinämie vorgesehen ist.

21. Antisense-Oligonukleotid zur Verwendung nach Anspruch 17 oder 18, wobei das Antisense-Oligonukleotid zur Verwendung bei der Behandlung von Arteriosklerose vorgesehen ist.

22. Antisense-Oligonukleotid zur Verwendung nach einem der Ansprüche 15-21, wobei das Antisense-Oligonukleotid zur Verwendung in Kombination mit Statinen vorgesehen ist.

23. Pharmazeutische Zusammensetzung, umfassend das Antisense-Oligonukleotid oder Konjugat nach einem der Ansprüche 1-22 und ein pharmazeutisch verträgliches Verdünnungsmittel, ein solcher Träger oder ein solches Hilfsmittel.

24. Verwendung eines Antisense-Oligonukleotids oder Konjugats davon, das in der Lage ist, die Expression von humanem PCSK9 zu hemmen, wobei das Antisense-Oligonukleotid eine Länge von 10-25 Nukleobase aufweist und komplementär zu einer entsprechenden Region von SEQ ID NR. 2 ist, zur Herstellung eines Arzneimittels zur Behandlung von Hypercholesterinämie oder einer mit Hypercholesterinämie verbundenen Krankheit, ausgewählt aus der Gruppe, bestehend aus Arteriosklerose, Hyperlipidämie, HDL/LDL-Cholesterin-Ungleichgewicht, Dyslipidämien, z. B. familiär kombinierte Hyperlipidämie (FCHL), erworbene Hyperlipidämie, Statin-resistente Hypercholesterinämie, Koronararterienerkrankung (CAD) und koronare Herzkrankheit (KHK) und wobei das Antisense-Oligonukleotid kein siRNA ist.

25. Verwendung nach Anspruch 24, wobei das Arzneimittel zur Verwendung in Kombination mit Statinen vorgesehen ist.

## Revendications

1. Oligonucléotide antisens, capable d'inhiber l'expression de la PCSK9 humaine, dans lequel ledit oligonucléotide antisens présente une longueur de 10 à 25 nucléobases et est complémentaire d'une région correspondante de SEQ ID NO 2, dans lequel l'oligonucléotide antisens est un oligonucléotide gapmère de formule A-B-C, dans lequel :
A se compose de ou comprend 1 à 6 analogues de nucléotides ; et
B se compose de ou comprend 4 à 12 nucléobases d'ADN consécutives ; et
C se compose de ou comprend 1 à 6 analogues de nucléotides ; dans lequel les analogues de nucléotides de A et de C augmentent la Tₘ du duplex oligonucléotide/cible.

2. L'oligonucléotide antisens selon la revendication 1, dans lequel la région B comprend 6 à 12 nucléotides d'ADN consécutifs.

3. L'oligonucléotide antisens selon la revendication 1 ou 2, dans lequel les analogues de nucléotides de A et de C sont des unités de LNA.

4. L'oligonucléotide antisens selon la revendication 3, dans lequel les unités de LNA sont des unités de bêta-D-oxy LNA.

5. L'oligonucléotide antisens selon la revendication 3 ou 4 qui comprend des unités de LNA de 3 à 8 nucléotides.

6. L'oligonucléotide antisens selon l'une quelconque des revendications 3 à 5, dans lequel les régions A et C se composent de 3 nucléotides de LNA et la région B se compose de 7, 8, 9 ou 10 nucléotides d'ADN consécutifs.

7. L'oligonucléotide antisens selon l'une quelconque des revendications précédentes, dans lequel ledit oligonucléotide antisens présente une longueur de 10 à 16 nucléobases.

8. L'oligonucléotide antisens selon l'une quelconque des revendications précédentes, dans lequel ledit oligonucléotide antisens présente une longueur de 12 à 14 nucléobases.

9. L'oligonucléotide antisens selon l'une quelconque des revendications précédentes, dans lequel chaque groupe de liaison internucléosidique est un groupe phosphorothioate.

10. L'oligonucléotide antisens selon la revendication 1, dans lequel A (région 5') se compose de 1, 2 ou 3 unités de LNA ; B (domaine central) se compose de 6 à 12 unités de LNA ; et C (région 3') se compose de 1, 2 ou 3 unités de LNA, et dans lequel chaque groupe de liaison internucléosidique est un groupe phosphorothioate.

11. L'oligonucléotide antisens selon la revendication 10, dans lequel ledit oligonucléotide antisens présente une longueur de 12 à 14 nucléobases.

12. Conjugué comprenant l'oligonucléotide antisens selon l'une quelconque des revendications précédentes et au moins une fraction non nucléotidique ou non polynucléotidique reliée par liaison covalente au dit oligonucléotide antisens.

13. Le conjugué selon la revendication 12, dans lequel l'oligonucléotide antisens est tel que défini dans l'une quelconque des revendications 3 à 11.

14. Le conjugué selon la revendication 12, dans lequel l'oligonucléotide antisens est tel que défini dans la revendication 11.

15. Oligonucléotide antisens, ou conjugué de celui-ci, capable d'inhiber l'expression de la PCSK9 humaine, dans lequel ledit oligonucléotide antisens présente une longueur de 10 à 25 nucléobases et est complémentaire d'une région correspondante de SEQ ID NO 2, pour utilisation comme médicament, dans lequel l'oligonucléotide antisens n'est pas un siARN.

16. L'oligonucléotide antisens pour utilisation selon la revendication 15, dans lequel l'oligonucléotide antisens est tel que défini dans l'une quelconque des revendications 1 à 11, ou un conjugué selon les revendications 12 à 14.

17. Oligonucléotide antisens, ou conjugué de celui-ci, capable d'inhiber l'expression de la PCSK9 humaine, dans lequel ledit oligonucléotide antisens présente une longueur de 10 à 25 nucléobases et est complémentaire d'une région correspondante de SEQ ID NO 2, pour utilisation dans le traitement de l'hypercholestérolémie ou d'un trouble associé à l'hypercholestérolémie choisi dans le groupe consistant en : athérosclérose, hyperlipidémie, déséquilibre du cholestérol HDL/LDL, dyslipidémies, par exemple hyperlipidémie mixte familiale (HLMF), hyperlipidémie acquise, hypercholestérolémie résistante aux statines, maladie des artères coronaires (MAC) et maladie cardiaque coronarienne (MCC) ; et dans lequel l'oligonucléotide antisens n'est pas un siARN.

18. L'oligonucléotide antisens pour utilisation selon la revendication 17, dans lequel l'oligonucléotide antisens est tel que défini dans l'une quelconque des revendications 1 à 11, ou un conjugué selon les revendications 12 à 14.

19. L'oligonucléotide antisens pour utilisation selon la revendication 17 ou 18, dans lequel l'oligonucléotide antisens est pour utilisation dans le traitement de l'hypercholestérolémie.

20. L'oligonucléotide antisens pour utilisation selon la revendication 17 ou 18, dans lequel l'oligonucléotide antisens est pour utilisation dans le traitement de l'hypercholestérolémie résistante aux statines.

21. L'oligonucléotide antisens pour utilisation selon la revendication 17 ou 18, dans lequel l'oligonucléotide antisens est pour utilisation dans le traitement de l'athérosclérose.

22. L'oligonucléotide antisens pour utilisation selon l'une quelconque des revendications 15 à 21, dans lequel l'oligonucléotide antisens est pour utilisation en association avec des statines.

23. Composition pharmaceutique comprenant l'oligonucléotide antisens ou le conjugué selon l'une quelconque des revendications 1 à 22, et un solvant, un véhicule ou un adjuvant pharmaceutiquement acceptable.

24. Utilisation d'un oligonucléotide antisens, ou d'un conjugué de celui-ci, capable d'inhiber l'expression de la PCSK9 humaine, dans laquelle ledit oligonucléotide antisens présente une longueur de 10 à 25 nucléobases et est complémentaire d'une région correspondante de SEQ ID NO 2, pour la fabrication d'un médicament pour le traitement de l'hypercholestérolémie ou d'un trouble associé choisi dans le groupe consistant en : athérosclérose, hyperlipidémie, déséquilibre du cholestérol HDL/LDL, dyslipidémies, par exemple hyperlipidémie mixte familiale (HLMF), hyperlipidémie acquise, hypercholestérolémie résistante aux statines, maladie des artères coronaires (MAC) et maladie cardiaque coronarienne (MCC), dans laquelle l'oligonucléotide antisens n'est pas un siARN.

25. L'utilisation selon la revendication 24, dans laquelle le médicament est pour utilisation en association avec des statines.


SEQUENCE LISTING
<110> Santaris A/S
<120> RNA Antagonist Compounds for the Modulation of *PCSK9*
<130> 1034EP2
<160> 393
<170> PatentIn version 3.3
<210> 1
   <211> 692
   <212> PRT
   <213> homo sapiens
<400> 1
<210> 2
   <211> 3636
   <212> DNA
   <213> homo sapiens
<400> 2
   cagcgacgtc gaggcgctca tggttgcagg cgggcgccgc cgttcagttc agggtctgag      60
   cctggaggag tgagccaggc agtgagactg gctcgggcgg gccgggacgc gtcgttgcag     120
   cagcggctcc cagctcccag ccaggattcc gcgcgcccct tcacgcgccc tgctcctgaa     180
   cttcagctcc tgcacagtcc tccccaccgc aaggctcaag gcgccgccgg cgtggaccgc     240
   gcacggcctc taggtctcct cgccaggaca gcaacctctc ccctggccct catgggcacc     300
   gtcagctcca ggcggtcctg gtggccgctg ccactgctgc tgctgctgct gctgctcctg     360
   ggtcccgcgg gcgcccgtgc gcaggaggac gaggacggcg actacgagga gctggtgcta     420
   gccttgcgtt ccgaggagga cggcctggcc gaagcacccg agcacggaac cacagccacc     480
   ttccaccgct gcgccaagga tccgtggagg ttgcctggca cctacgtggt ggtgctgaag     540
   gaggagaccc acctctcgca gtcagagcgc actgcccgcc gcctgcaggc ccaggctgcc     600
   cgccggggat acctcaccaa gatcctgcat gtcttccatg gccttcttcc tggcttcctg     660
   gtgaagatga gtggcgacct gctggagctg gccttgaagt tgccccatgt cgactacatc     720
   gaggaggact cctctgtctt tgcccagagc atcccgtgga acctggagcg gattacccct     780
   ccacggtacc gggcggatga ataccagccc cccgacggag gcagcctggt ggaggtgtat     840
   ctcctagaca ccagcataca gagtgaccac cgggaaatcg agggcagggt catggtcacc     900
   gacttcgaga atgtgcccga ggaggacggg acccgcttcc acagacaggc cagcaagtgt     960
   gacagtcatg gcacccacct ggcaggggtg gtcagcggcc gggatgccgg cgtggccaag    1020
   ggtgccagca tgcgcagcct gcgcgtgctc aactgccaag ggaagggcac ggttagcggc    1080
   accctcatag gcctggagtt tattcggaaa agccagctgg tccagcctgt ggggccactg    1140
   gtggtgctgc tgcccctggc gggtgggtac agccgcgtcc tcaacgccgc ctgccagcgc    1200
   ctggcgaggg ctggggtcgt gctggtcacc gctgccggca acttccggga cgatgcctgc   1260
   ctctactccc cagcctcagc tcccgaggtc atcacagttg gggccaccaa tgcccaagac   1320
   cagccggtga ccctggggac tttggggacc aactttggcc gctgtgtgga cctctttgcc   1380
   ccaggggagg acatcattgg tgcctccagc gactgcagca cctgctttgt gtcacagagt   1440
   gggacatcac aggctgctgc ccacgtggct ggcattgcag ccatgatgct gtctgccgag   1500
   ccggagctca ccctggccga gttgaggcag agactgatcc acttctctgc caaagatgtc   1560
   atcaatgagg cctggttccc tgaggaccag cgggtactga cccccaacct ggtggccgcc   1620
   ctgcccccca gcacccatgg ggcaggttgg cagctgtttt gcaggactgt atggtcagca   1680
   cactcggggc ctacacggat ggccacagcc gtcgcccgct gcgccccaga tgaggagctg   1740
   ctgagctgct ccagtttctc caggagtggg aagcggcggg gcgagcgcat ggaggcccaa   1800
   gggggcaagc tggtctgccg ggcccacaac gcttttgggg gtgagggtgt ctacgccatt   1860
   gccaggtgct gcctgctacc ccaggccaac tgcagcgtcc acacagctcc accagctgag   1920
   gccagcatgg ggacccgtgt ccactgccac caacagggcc acgtcctcac aggctgcagc   1980
   tcccactggg aggtggagga ccttggcacc cacaagccgc ctgtgctgag gccacgaggt   2040
   cagcccaacc agtgcgtggg ccacagggag gccagcatcc acgcttcctg ctgccatgcc   2100
   ccaggtctgg aatgcaaagt caaggagcat ggaatcccgg cccctcagga gcaggtgacc   2160
   gtggcctgcg aggagggctg gaccctgact ggctgcagtg ccctccctgg gacctcccac   2220
   gtcctggggg cctacgccgt agacaacacg tgtgtagtca ggagccggga cgtcagcact   2280
   acaggcagca ccagcgaagg ggccgtgaca gccgttgcca tctgctgccg gagccggcac   2340
   ctggcgcagg cctcccagga gctccagtga cagccccatc ccaggatggg tgtctgggga   2400
   gggtcaaggg ctggggctga gctttaaaat ggttccgact tgtccctctc tcagccctcc   2460
   atggcctggc acgaggggat ggggatgctt ccgcctttcc ggggctgctg gcctggccct   2520
   tgagtggggc agcctccttg cctggaactc actcactctg ggtgcctcct ccccaggtgg   2580
   aggtgccagg aagctccctc cctcactgtg gggcatttca ccattcaaac aggtcgagct   2640
   gtgctcgggt gctgccagct gctcccaatg tgccgatgtc cgtgggcaga atgactttta   2700
   ttgagctctt gttccgtgcc aggcattcaa tcctcaggtc tccaccaagg aggcaggatt   2760
   cttcccatgg ataggggagg gggcggtagg ggctgcaggg acaaacatcg ttggggggtg    2820
   agtgtgaaag gtgctgatgg ccctcatctc cagctaactg tggagaagcc cctgggggct   2880
   ccctgattaa tggaggctta gctttctgga tggcatctag ccagaggctg gagacaggtg   2940
   cgcccctggt ggtcacaggc tgtgccttgg tttcctgagc cacctttact ctgctctatg   3000
   ccaggctgtg ctagcaacac ccaaaggtgg cctgcgggga gccatcacct aggactgact 3060
   cggcagtgtg cagtggtgca tgcactgtct cagccaaccc gctccactac ccggcagggt 3120
   acacattcgc acccctactt cacagaggaa gaaacctgga accagagggg gcgtgcctgc 3180
   caagctcaca cagcaggaac tgagccagaa acgcagattg ggctggctct gaagccaagc 3240
   ctcttcttac ttcacccggc tgggctcctc atttttacgg gtaacagtga ggctgggaag 3300
   gggaacacag accaggaagc tcggtgagtg atggcagaac gatgcctgca ggcatggaac 3360
   tttttccgtt atcacccagg cctgattcac tggcctggcg gagatgcttc taaggcatgg 3420
   tcgggggaga gggccaacaa ctgtccctcc ttgagcacca gccccaccca agcaagcaga 3480
   catttatctt ttgggtctgt cctctctgtt gcctttttac agccaacttt tctagacctg 3540
   ttttgctttt gtaacttgaa gatatttatt ctgggttttg tagcattttt attaatatgg 3600
   tgacttttta aaataaaaac aaacaaacgt tgtcct 3636
<210> 3
   <211> 16
   <212> DNA
   <213> artificial
<220>
   <223> Preferred seqeunce motif
<400> 3
   gcctgtctgt ggaagc 16
<210> 4
   <211> 16
   <212> DNA
   <213> Artificial
<220>
   <223> Preferred seqeunce motif
<400> 4
   caagttacaa aagcaa 16
<210> 5
   <211> 16
   <212> DNA
   <213> Artificial
<220>
   <223> Preferred seqeunce motif
<400> 5
   gagatacacc tccacc 16
<210> 6
   <211> 16
   <212> DNA
   <213> Artificial
<220>
   <223> Preferred seqeunce motif
<400> 6
   tcctcaggga accagg 16
<210> 7
   <211> 16
   <212> DNA
   <213> Artificial
<220>
   <223> Preferred seqeunce motif
<400> 7
   ctggagcagc tcagca 16
<210> 8
   <211> 16
   <212> DNA
   <213> Artificial
<220>
   <223> Preferred seqeunce motif
<400> 8
   catggcagca ggaagc 16
<210> 9
   <211> 16
   <212> DNA
   <213> Artificial
<220>
   <223> LNA Oligomer
<220>
   <221> phosphotohioate linkage
   <222> (1) .. (15)
<220>
   <221> LNA Nucleobase
   <222> (1) .. (3)
<220>
   <221> LNA Nucleobase
   <222> (14) .. (16)
<220>
   <221> 5'-methyl modified cytosine
   <222> (15) .. (16)
<400> 9
   gagatacacc tccacc 16
<210> 10
   <211> 16
   <212> DNA
   <213> Artificial
<220>
   <223> LNA Oligomer
<220>
   <221> Phosphorothioate Linkage
   <222> (1) .. (15)
<220>
   <221> LNA Nucleobase
   <222> (1) .. (3)
<220>
   <221> 5'-methyl modified cytosine
   <222> (2)..(3)
<220>
   <221> LNA Nucleobase
   <222> (14) .. (16)
<220>
   <221> 5'-methyl modified cytosine
   <222> (16) .. (16)
<400> 10
   gcctgtctgt ggaagc 16
<210> 11
   <211> 16
   <212> DNA
   <213> Artificial
<220>
   <223> LNA Oligomer
<220>
   <221> phosphothioate Linkage
   <222> (1) .. (15)
<220>
   <221> LNA nucleobase
   <222> (1) .. (3)
<220>
   <221> 5'-methyl modified cytosine
   <222> (1) .. (1)
<220>
   <221> LNA nucleobase
   <222> (14) .. (16)
<220>
   <221> 5'-methyl modified cytosine
   <222> (14)..(14)
<400> 11
   caagttacaa aagcaa 16
<210> 12
   <211> 15
   <212> DNA
   <213> artificial
<220>
   <223> Control Oligomer
<220>
   <221> Phosphorothioate Linkage
   <222> (1) .. (15)
<220>
   <221> LNA Nucleobase
   <222> (1) .. (3)
<220>
   <221> 5'-methyl modified cytosine
   <222> (1) .. (1)
<220>
   <221> LNA Nucleobase
   <222> (13)..(15)
<400> 12
   cgtcagtatg cgaat 15
<210> 13
   <211> 20
   <212> DNA
   <213> Artificial
<220>
   <223> ISIS Antisense oligonucloeitde compound
<220>
   <221> phosphorothioate Linkage
   <222> (1) .. (19)
<220>
   <221> 2'-O-methyl DNA
   <222> (1) .. (5)
<220>
   <221> 5'-methyl modified cytosine
   <222> (2)..(3)
<220>
   <221> 5'-methyl modified cytosine
   <222> (5) .. (5)
<220>
   <221> 5'-methyl modified cytosine
   <222> (12)..(12)
<220>
   <221> 5'-methyl modified cytosine
   <222> (15)..(15)
<220>
   <221> 2'-O-methyl DNA
   <222> (16)..(20)
<220>
   <221> 5'-methyl modified cytosine
   <222> (17)..(17)
<220>
   <221> 5'-methyl modified cytosine
   <222> (19) .. (20)
<400> 13
   gcctcagtct gcttcgcacc 20
<210> 14
   <211> 16
   <212> DNA
   <213> homo sapiens
<400> 14
   ggtggaggtg tatctc 16
<210> 15
   <211> 17
   <212> DNA
   <213> Homo sapiens
<400> 15
   cgcttccaca gacaggc 17
<210> 16
   <211> 23
   <212> DNA
   <213> homo sapiens
<400> 16
   ggcctggttc cctgaggacc agc 23
<210> 17
   <211> 23
   <212> DNA
   <213> homo sapiens
<400> 17
   gaggagctgc tgagctgctc cag 23
<210> 18
   <211> 23
   <212> DNA
   <213> homo sapiens
<400> 18
   gcttcctgct gccatgcccc agg 23
<210> 19
   <211> 15
   <212> DNA
   <213> homo sapiens
<400> 19
   ttgcttttgt aactt 15
<210> 20
   <211> 14
   <212> DNA
   <213> Artificial
<220>
   <223> LNA Oligomer
<220>
   <221> Phosphorothioate Linkage
   <222> (1) .. (14)
<220>
   <221> LNA Nucleobase
   <222> (1) .. (3)
<220>
   <221> LNA Nucleobase
   <222> (12)..(14)
<220>
   <221> 5'-methyl modified cytosine
   <222> (14)..(14)
<400> 20
   gagtagaggc aggc 14
<210> 21
   <211> 16
   <212> DNA
   <213> Artificial
<220>
   <223> LNA Oligomer
<220>
   <221> Phosphorothioate Linkage
   <222> (1) .. (15)
<220>
   <221> LNA Nucleobase
   <222> (1) .. (3)
<220>
   <221> 5'-methyl modified cytosine
   <222> (2)..(3)
<220>
   <221> LNA Nucleobase
   <222> (14) .. (16)
<400> 21
   tcctcaggga accagg 16
<210> 22
   <211> 16
   <212> DNA
   <213> Artificial
<220>
   <223> LNA Oligomer
<220>
   <221> Phosphorothioate Linkage
   <222> (1) .. (15)
<220>
   <221> LNA Nucleobase
   <222> (1) .. (3)
<220>
   <221> 5'-methyl modified cytosine
   <222> (1) .. (1)
<220>
   <221> LNA Nucleobase
   <222> (14) .. (16)
<220>
   <221> 5'-methyl modified cytosine
   <222> (15)..(15)
<400> 22
   ctggagcagc tcagca 16
<210> 23
   <211> 16
   <212> DNA
   <213> Artificial
<220>
   <223> LNA oligomer
<220>
   <221> Phosphorothioate Linkage
   <222> (1) .. (15)
<220>
   <221> LNA Nucleobase
   <222> (1) .. (3)
<220>
   <221> 5'-methyl modified cytosine
   <222> (1) .. (1)
<220>
   <221> LNA Nucleobase
   <222> (14) .. (16)
<220>
   <221> 5'-methyl modified cytosine
   <222> (16) .. (16)
<400> 23
   catggcagca ggaagc 16
<210> 24
   <211> 14
   <212> DNA
   <213> Artificial
<220>
   <223> LNA Oligomer
<220>
   <221> Phosphorothioate Linkage
   <222> (1) .. (13)
<220>
   <221> LNA Nucleobase
   <222> (1) .. (3)
<220>
   <221> LNA Nucleobase
   <222> (12)..(14)
<220>
   <221> 5'-methyl modified cytosine
   <222> (13)..(14)
<400> 24
   gatacacctc cacc 14
<210> 25
   <211> 14
   <212> DNA
   <213> Artificial
<220>
   <223> LNA Oligomer
<220>
   <221> Phosphorothioate Linkage
   <222> (1) .. (13)
<220>
   <221> LNA Nucleobase
   <222> (1) .. (3)
<220>
   <221> 5'-methyl modified cytosine
   <222> (1) .. (1)
<220>
   <221> LNA Nucleobase
   <222> (12)..(14)
<220>
   <221> 5'-methyl modified cytosine
   <222> (14)..(14)
<400> 25
   ctgtctgtgg aagc 14
<210> 26
   <211> 13
   <212> DNA
   <213> Artificial
<220>
   <223> LNA Oligomer
<220>
   <221> Phosphorothioate Linkage
   <222> (1) .. (12)
<220>
   <221> LNA Nucleobase
   <222> (1) .. (2)
<220>
   <221> LNA Nucleobase
   <222> (11) .. (13)
<220>
   <221> 5'-methyl modified cytosine
   <222> (12)..(12)
<400> 26
   gtctgtggaa gcg 13
<210> 27
   <211> 13
   <212> DNA
   <213> Artificial
<220>
   <223> LNA Oligomer
<220>
   <221> Phosphorothioate Linkage
   <222> (1) .. (12)
<220>
   <221> LNA Nucleobase
   <222> (1) .. (12)
<220>
   <221> LNA Nucleobase
   <222> (11) .. (13)
<220>
   <221> 5'-methyl modified cytosine
   <222> (11) .. (11)
<220>
   <221> 5'-methyl modified cytosine
   <222> (13)..(13)
<400> 27
   atgagggtgc cgc 13
<210> 28
   <211> 13
   <212> DNA
   <213> Artificial
<220>
   <223> LNA Oligomer
<220>
   <221> Phosphorothioate Linkage
   <222> (1) .. (12)
<220>
   <221> LNA Nucleobase
   <222> (1) .. (2)
<220>
   <221> LNA Nucleobase
   <222> (11) .. (13)
<220>
   <221> 5'-methyl modified cytosine
   <222> (13)..(13)
<400> 28
   ataaactcca ggc 13
<210> 29
   <211> 13
   <212> DNA
   <213> artificial
<220>
   <223> LNA oligomer
<220>
   <221> Phosphorothioate Linkage
   <222> (1) .. (12)
<220>
   <221> LNA Nucleobase
   <222> (1) .. (2)
<220>
   <221> LNA Nucleobase
   <222> (11) .. (13)
<220>
   <221> 5'-methyl modified cytosine
   <222> (11) .. (11)
<220>
   <221> 5'-methyl modified cytosine
   <222> (13)..(13)
<400> 29
   tagacaccct cac 13
<210> 30
   <211> 14
   <212> DNA
   <213> Artificial
<220>
   <223> Oligomer motif
<400> 30
   gagtagaggc aggc 14
<210> 31
   <211> 16
   <212> DNA
   <213> Artificial
<220>
   <223> Oligomer motif
<400> 31
   tcctcaggga accagg 16
<210> 32
   <211> 16
   <212> DNA
   <213> Artificial
<220>
   <223> Oligomer motif
<400> 32
   ctggagcagc tcagca 16
<210> 33
   <211> 16
   <212> DNA
   <213> Artificial
<220>
   <223> Oligomer motif
<400> 33
   catggcagca ggaagc 16
<210> 34
   <211> 14
   <212> DNA
   <213> Artificial
<220>
   <223> Oligomer motif
<400> 34
   gatacacctc cacc 14
<210> 35
   <211> 14
   <212> DNA
   <213> Oligomer motif
<400> 35
   ctgtctgtgg aagc 14
<210> 36
   <211> 13
   <212> DNA
   <213> Artificial
<220>
   <223> Oligomer motif
<400> 36
   gtctgtggaa gcg 13
<210> 37
   <211> 13
   <212> DNA
   <213> Artificial
<220>
   <223> Oligomer motif
<400> 37
   atgagggtgc cgc 13
<210> 38
   <211> 13
   <212> DNA
   <213> Artificial
<220>
   <223> Oligomer motif
<400> 38
   ataaactcca ggc 13
<210> 39
   <211> 13
   <212> DNA
   <213> Artificial
<220>
   <223> Oligomer motif
<400> 39
   tagacaccct cac 13
<210> 40
   <211> 12
   <212> DNA
   <213> Artificial
<220>
   <223> Oligo motif
<400> 40
   tctgtggaag cg 12
<210> 41
   <211> 12
   <212> DNA
   <213> Artificial
<220>
   <223> Oligo motif
<400> 41
   cctatgaggg tg 12
<210> 42
   <211> 12
   <212> DNA
   <213> Artificial
<220>
   <223> Oligo motif
<400> 42
   ccgaataaac tc 12
<210> 43
   <211> 12
   <212> DNA
   <213> Artificial
<220>
   <223> Oligo motif
<400> 43
   taaactccag gc 12
<210> 44
   <211> 12
   <212> DNA
   <213> Artificial
<220>
   <223> Oligo motif
<400> 44
   cggccgctga cc 12
<210> 45
   <211> 12
   <212> DNA
   <213> Artificial
<220>
   <223> Oligo motif
<400> 45
   ccaggcctat ga 12
<210> 46
   <211> 12
   <212> DNA
   <213> Artificial
<220>
   <223> Oligo motif
<400> 46
   ggcctatgag gg 12
<210> 47
   <211> 13
   <212> DNA
   <213> Artificial
<220>
   <223> Oligo motif
<400> 47
   gtctgtggaa gcg 13
<210> 48
   <211> 13
   <212> DNA
   <213> Artificial
<220>
   <223> Oligo motif
<400> 48
   cccggccgct gac 13
<210> 49
   <211> 13
   <212> DNA
   <213> Artificial
<220>
   <223> Oligo motif
<400> 49
   atgagggtgc cgc 13
<210> 50
   <211> 13
   <212> DNA
   <213> Artificial
<220>
   <223> Oligo motif
<400> 50
   gcctatgagg gtg 13
<210> 51
   <211> 13
   <212> DNA
   <213> Artificial
<220>
   <223> Oligo motif
<400> 51
   ccaggcctat gag 13
<210> 52
   <211> 13
   <212> DNA
   <213> Artificial
<220>
   <223> Oligo motif
<400> 52
   actccaggcc tat 13
<210> 53
   <211> 13
   <212> DNA
   <213> Artificial
<220>
   <223> Oligo motif
<400> 53
   taaactccag gcc 13
<210> 54
   <211> 13
   <212> DNA
   <213> Artificial
<220>
   <223> Oligo motif
<400> 54
   ataaactcca ggc 13
<210> 55
   <211> 13
   <212> DNA
   <213> Artificial
<220>
   <223> Oligo motif
<400> 55
   gccccgagtg tgc 13
<210> 56
   <211> 13
   <212> DNA
   <213> Artificial
<220>
   <223> Oligo motif
<400> 56
   tagacaccct cac 13
<210> 57
   <211> 13
   <212> DNA
   <213> Artificial
<220>
   <223> Oligo motif
<400> 57
   atggggcaac ttc 13
<210> 58
   <211> 13
   <212> DNA
   <213> Artificial
<220>
   <223> Oligo motif
<400> 58
   gagatacacc tcc 13
<210> 59
   <211> 13
   <212> DNA
   <213> Artificial
<220>
   <223> Oligo motif
<400> 59
   tccaggccta tga 13
<210> 60
   <211> 13
   <212> DNA
   <213> Artificial
<220>
   <223> Oligo motif
<400> 60
   ggccccgagt gtg 13
<210> 61
   <211> 13
   <212> DNA
   <213> Artificial
<220>
   <223> Oligo motif
<400> 61
   caggcctatg agg 13
<210> 62
   <211> 13
   <212> DNA
   <213> Artificial
<220>
   <223> Oligo motif
<400> 62
   agatacacct cca 13
<210> 63
   <211> 13
   <212> DNA
   <213> Artificial
<220>
   <223> Oligo motif
<400> 63
   cacgtgggca gca 13
<210> 64
   <211> 13
   <212> DNA
   <213> Artificial
<220>
   <223> Oligo motif
<400> 64
   tgtcacactt gct 13
<210> 65
   <211> 13
   <212> DNA
   <213> Artificial
<220>
   <223> Oligo motif
<400> 65
   tcccggccgc tga 13
<210> 66
   <211> 13
   <212> DNA
   <213> Artificial
<220>
   <223> Oligo motif
<400> 66
   tatgagggtg ccg 13
<210> 67
   <211> 13
   <212> DNA
   <213> Artificial
<220>
   <223> Oligo motif
<400> 67
   ctatgagggt gcc 13
<210> 68
   <211> 13
   <212> DNA
   <213> Artificial
<220>
   <223> Oligo motif
<400> 68
   cctatgaggg tgc 13
<210> 69
   <211> 13
   <212> DNA
   <213> Artificial
<220>
   <223> Oligo motif
<400> 69
   ggcctatgag ggt 13
<210> 70
   <211> 13
   <212> DNA
   <213> Artificial
<220>
   <223> Oligo motif
<400> 70
   aggcctatga ggg 13
<210> 71
   <211> 13
   <212> DNA
   <213> Artificial
<220>
   <223> Oligo motif
<400> 71
   ctccaggcct atg 13
<210> 72
   <211> 13
   <212> DNA
   <213> Artificial
<220>
   <223> Oligo motif
<400> 72
   tccgaataaa ctc 13
<210> 73
   <211> 13
   <212> DNA
   <213> Artificial
<220>
   <223> Oligo motif
<400> 73
   cgtcccggaa gtt 13
<210> 74
   <211> 13
   <212> DNA
   <213> Artificial
<220>
   <223> Oligo motif
<400> 74
   taatcaggga gcc 13
<210> 75
   <211> 13
   <212> DNA
   <213> Artificial
<220>
   <223> Oligo motif
<400> 75
   tggggcaact tca 13
<210> 76
   <211> 13
   <212> DNA
   <213> Artificial
<220>
   <223> Oligo motif
<400> 76
   catggggcaa ctt 13
<210> 77
   <211> 13
   <212> DNA
   <213> Artificial
<220>
   <223> Oligo motif
<400> 77
   cggccgctga cca 13
<210> 78
   <211> 13
   <212> DNA
   <213> Artificial
<220>
   <223> Oligo motif
<400> 78
   ccggccgctg acc 13
<210> 79
   <211> 13
   <212> DNA
   <213> Artificial
<220>
   <223> Oligo motif
<400> 79
   ccgaataaac tcc 13
<210> 80
   <211> 13
   <212> DNA
   <213> Artificial
<220>
   <223> Oligo motif
<400> 80
   gtcccactct gtg 13
<210> 81
   <211> 13
   <212> DNA
   <213> Artificial
<220>
   <223> Oligo motif
<400> 81
   caggttgggg gtc 13
<210> 82
   <211> 13
   <212> DNA
   <213> Artificial
<220>
   <223> Oligo motif
<400> 82
   cggcagcaga tgg 13
<210> 83
   <211> 13
   <212> DNA
   <213> Artificial
<220>
   <223> Oligo motif
<400> 83
   acaccctcac ccc 13
<210> 84
   <211> 13
   <212> DNA
   <213> Artificial
<220>
   <223> Oligo motif
<400> 84
   tccggcagca gat 13
<210> 85
   <211> 13
   <212> DNA
   <213> Artificial
<220>
   <223> Oligo motif
<400> 85
   atacacctcc acc 13
<210> 86
   <211> 13
   <212> DNA
   <213> Artificial
<220>
   <223> Oligo motif
<400> 86
   cctgtctgtg gaa 13
<210> 87
   <211> 13
   <212> DNA
   <213> Artificial
<220>
   <223> Oligo motif
<400> 87
   gcctgtctgt gga 13
<210> 88
   <211> 13
   <212> DNA
   <213> Artificial
<220>
   <223> Oligo motif
<400> 88
   ttccgaataa act 13
<210> 89
   <211> 13
   <212> DNA
   <213> Artificial
<220>
   <223> Oligo motif
<400> 89
   actgtgatga cct 13
<210> 90
   <211> 13
   <212> DNA
   <213> Artificial
<220>
   <223> Oligo motif
<400> 90
   tcgtcccgga agt 13
<210> 91
   <211> 13
   <212> DNA
   <213> Artificial
<220>
   <223> Oligo motif
<400> 91
   tcccactctg tga 13
<210> 92
   <211> 13
   <212> DNA
   <213> Artificial
<220>
   <223> Oligo motif
<400> 92
   aataaactcc agg 13
<210> 93
   <211> 13
   <212> DNA
   <213> Artificial
<220>
   <223> Oligo motif
<400> 93
   gctggggagt aga 13
<210> 94
   <211> 13
   <212> DNA
   <213> Artificial
<220>
   <223> Oligo motif
<400> 94
   ttaatcaggg agc 13
<210> 95
   <211> 14
   <212> DNA
   <213> Artificial
<220>
   <223> Oligo motif
<400> 95
   tgtctgtgga agcg 14
<210> 96
   <211> 14
   <212> DNA
   <213> Artificial
<220>
   <223> Oligo motif
<400> 96
   cctgtctgtg gaag 14
<210> 97
   <211> 14
   <212> DNA
   <213> Artificial
<220>
   <223> Oligo motif
<400> 97
   ctgtcacact tgct 14
<210> 98
   <211> 14
   <212> DNA
   <213> Artificial
<220>
   <223> Oligo motif
<400> 98
   cggccgctga ccac 14
<210> 99
   <211> 14
   <212> DNA
   <213> Artificial
<220>
   <223> Oligo motif
<400> 99
   cccggccgct gacc 14
<210> 100
   <211> 14
   <212> DNA
   <213> Artificial
<220>
   <223> Oligo motif
<400> 100
   tcccggccgc tgac 14
<210> 101
   <211> 14
   <212> DNA
   <213> Artificial
<220>
   <223> Oligo motif
<400> 101
   atcccggccg ctga 14
<210> 102
   <211> 14
   <212> DNA
   <213> Artificial
<220>
   <223> Oligo motif
<400> 102
   atgagggtgc cgct 14
<210> 103
   <211> 14
   <212> DNA
   <213> Artificial
<220>
   <223> Oligo motif
<400> 103
   tatgagggtg ccgc 14
<210> 104
   <211> 14
   <212> DNA
   <213> Artificial
<220>
   <223> Oligo motif
<400> 104
   gcctatgagg gtgc 14
<210> 105
   <211> 14
   <212> DNA
   <213> Artificial
<220>
   <223> Oligo motif
<400> 105
   ggcctatgag ggtg 14
<210> 106
   <211> 14
   <212> DNA
   <213> Artificial
<220>
   <223> Oligo motif
<400> 106
   caggcctatg aggg 14
<210> 107
   <211> 14
   <212> DNA
   <213> Artificial
<220>
   <223> Oligo motif
<400> 107
   ccaggcctat gagg 14
<210> 108
   <211> 14
   <212> DNA
   <213> Artificial
<220>
   <223> Oligo motif
<400> 108
   tccaggccta tgag 14
<210> 109
   <211> 14
   <212> DNA
   <213> Artificial
<220>
   <223> Oligo motif
<400> 109
   ctccaggcct atga 14
<210> 110
   <211> 14
   <212> DNA
   <213> Artificial
<220>
   <223> Oligo motif
<400> 110
   actccaggcc tatg 14
<210> 111
   <211> 14
   <212> DNA
   <213> Artificial
<220>
   <223> Oligo motif
<400> 111
   aactccaggc ctat 14
<210> 112
   <211> 14
   <212> DNA
   <213> Artificial
<220>
   <223> Oligo motif
<400> 112
   aaactccagg ccta 14
<210> 113
   <211> 14
   <212> DNA
   <213> Artificial
<220>
   <223> Oligo motif
<400> 113
   taaactccag gcct 14
<210> 114
   <211> 14
   <212> DNA
   <213> Artificial
<220>
   <223> Oligo motif
<400> 114
   ataaactcca ggcc 14
<210> 115
   <211> 14
   <212> DNA
   <213> Artificial
<220>
   <223> Oligo motif
<400> 115
   aataaactcc aggc 14
<210> 116
   <211> 14
   <212> DNA
   <213> Artificial
<220>
   <223> Oligo motif
<400> 116
   ttccgaataa actc 14
<210> 117
   <211> 14
   <212> DNA
   <213> Artificial
<220>
   <223> Oligo motif
<400> 117
   tcgtcccgga agtt 14
<210> 118
   <211> 14
   <212> DNA
   <213> Artificial
<220>
   <223> Oligo motif
<400> 118
   gagtagaggc aggc 14
<210> 119
   <211> 14
   <212> DNA
   <213> Artificial
<220>
   <223> Oligo motif
<400> 119
   ggggagtaga ggca 14
<210> 120
   <211> 14
   <212> DNA
   <213> Artificial
<220>
   <223> Oligo motif
<400> 120
   gctggggagt agag 14
<210> 121
   <211> 14
   <212> DNA
   <213> Artificial
<220>
   <223> Oligo motif
<400> 121
   actgtgatga cctc 14
<210> 122
   <211> 14
   <212> DNA
   <213> Artificial
<220>
   <223> Oligo motif
<400> 122
   gtcccactct gtga 14
<210> 123
   <211> 14
   <212> DNA
   <213> Artificial
<220>
   <223> Oligo motif
<400> 123
   ccaggttggg ggtc 14
<210> 124
   <211> 14
   <212> DNA
   <213> Artificial
<220>
   <223> Oligo motif
<400> 124
   ggccccgagt gtgc 14
<210> 125
   <211> 14
   <212> DNA
   <213> Artificial
<220>
   <223> Oligo motif
<400> 125
   acaccctcac cccc 14
<210> 126
   <211> 14
   <212> DNA
   <213> Artificial
<220>
   <223> Oligo motif
<400> 126
   gacaccctca cccc 14
<210> 127
   <211> 14
   <212> DNA
   <213> Artificial
<220>
   <223> Oligo motif
<400> 127
   tagacaccct cacc 14
<210> 128
   <211> 14
   <212> DNA
   <213> Artificial
<220>
   <223> Oligo motif
<400> 128
   ggggcatggc agca 14
<210> 129
   <211> 14
   <212> DNA
   <213> Artificial
<220>
   <223> Oligo motif
<400> 129
   cggcagcaga tggc 14
<210> 130
   <211> 14
   <212> DNA
   <213> Artificial
<220>
   <223> Oligo motif
<400> 130
   ccggcagcag atgg 14
<210> 131
   <211> 14
   <212> DNA
   <213> Artificial
<220>
   <223> Oligo motif
<400> 131
   gctccggcag caga 14
<210> 132
   <211> 14
   <212> DNA
   <213> Artificial
<220>
   <223> Oligo motif
<400> 132
   taatcaggga gccc 14
<210> 133
   <211> 14
   <212> DNA
   <213> Artificial
<220>
   <223> Oligo motif
<400> 133
   ttaatcaggg agcc 14
<210> 134
   <211> 14
   <212> DNA
   <213> Artificial
<220>
   <223> Oligo motif
<400> 134
   atggggcaac ttca 14
<210> 135
   <211> 14
   <212> DNA
   <213> Artificial
<220>
   <223> Oligo motif
<400> 135
   catggggcaa cttc 14
<210> 136
   <211> 14
   <212> DNA
   <213> Artificial
<220>
   <223> Oligo motif
<400> 136
   gatacacctc cacc 14
<210> 137
   <211> 14
   <212> DNA
   <213> Artificial
<220>
   <223> Oligo motif
<400> 137
   agatacacct ccac 14
<210> 138
   <211> 14
   <212> DNA
   <213> Artificial
<220>
   <223> Oligo motif
<400> 138
   gagatacacc tcca 14
<210> 139
   <211> 14
   <212> DNA
   <213> Artificial
<220>
   <223> Oligo motif
<400> 139
   ctgtctgtgg aagc 14
<210> 140
   <211> 14
   <212> DNA
   <213> Artificial
<220>
   <223> Oligo motif
<400> 140
   gcctgtctgt ggaa 14
<210> 141
   <211> 14
   <212> DNA
   <213> Artificial
<220>
   <223> Oligo motif
<400> 141
   acgtgggcag cagc 14
<210> 142
   <211> 14
   <212> DNA
   <213> Artificial
<220>
   <223> Oligo motif
<400> 142
   tcctcaggga acca 14
<210> 143
   <211> 14
   <212> DNA
   <213> Artificial
<220>
   <223> Oligo motif
<400> 143
   gcatggcagc agga 14
<210> 144
   <211> 14
   <212> DNA
   <213> Artificial
<220>
   <223> Oligo motif
<400> 144
   ctccggcagc agat 14
<210> 145
   <211> 14
   <212> DNA
   <213> Artificial
<220>
   <223> Oligo motif
<400> 145
   cacgtgggca gcag 14
<210> 146
   <211> 14
   <212> DNA
   <213> Artificial
<220>
   <223> Oligo motif
<400> 146
   ccacgtgggc agca 14
<210> 147
   <211> 14
   <212> DNA
   <213> Artificial
<220>
   <223> Oligo motif
<400> 147
   ggagcagctc agca 14
<210> 148
   <211> 14
   <212> DNA
   <213> Artificial
<220>
   <223> Oligo motif
<400> 148
   gagcagctca gcag 14
<210> 149
   <211> 14
   <212> DNA
   <213> Artificial
<220>
   <223> Oligo motif
<400> 149
   atgggtgctg gggg 14
<210> 150
   <211> 14
   <212> DNA
   <213> Artificial
<220>
   <223> Oligo motif
<400> 150
   ccggccgctg acca 14
<210> 151
   <211> 14
   <212> DNA
   <213> Artificial
<220>
   <223> Oligo motif
<400> 151
   catcccggcc gctg 14
<210> 152
   <211> 14
   <212> DNA
   <213> Artificial
<220>
   <223> Oligo motif
<400> 152
   ctatgagggt gccg 14
<210> 153
   <211> 14
   <212> DNA
   <213> Artificial
<220>
   <223> Oligo motif
<400> 153
   cctatgaggg tgcc 14
<210> 154
   <211> 14
   <212> DNA
   <213> Artificial
<220>
   <223> Oligo motif
<400> 154
   aggcctatga gggt 14
<210> 155
   <211> 14
   <212> DNA
   <213> Artificial
<220>
   <223> Oligo motif
<400> 155
   gaataaactc cagg 14
<210> 156
   <211> 14
   <212> DNA
   <213> Artificial
<220>
   <223> Oligo motif
<400> 156
   cgaataaact ccag 14
<210> 157
   <211> 14
   <212> DNA
   <213> Artificial
<220>
   <223> Oligo motif
<400> 157
   tccgaataaa ctcc 14
<210> 158
   <211> 14
   <212> DNA
   <213> Artificial
<220>
   <223> Oligo motif
<400> 158
   tccccaaagt cccc 14
<210> 159
   <211> 14
   <212> DNA
   <213> Artificial
<220>
   <223> Oligo motif
<400> 159
   cccactctgt gaca 14
<210> 160
   <211> 14
   <212> DNA
   <213> Artificial
<220>
   <223> Oligo motif
<400> 160
   agagaagtgg atca 14
<210> 161
   <211> 14
   <212> DNA
   <213> Artificial
<220>
   <223> Oligo motif
<400> 161
   tcagggaacc aggc 14
<210> 162
   <211> 14
   <212> DNA
   <213> Artificial
<220>
   <223> Oligo motif
<400> 162
   accaggttgg gggt 14
<210> 163
   <211> 14
   <212> DNA
   <213> Artificial
<220>
   <223> Oligo motif
<400> 163
   accctcaccc ccaa 14
<210> 164
   <211> 14
   <212> DNA
   <213> Artificial
<220>
   <223> Oligo motif
<400> 164
   ggtggcagtg gaca 14
<210> 165
   <211> 14
   <212> DNA
   <213> Artificial
<220>
   <223> Oligo motif
<400> 165
   tggtggcagt ggac 14
<210> 166
   <211> 14
   <212> DNA
   <213> Artificial
<220>
   <223> Oligo motif
<400> 166
   cctggggcat ggca 14
<210> 167
   <211> 14
   <212> DNA
   <213> Artificial
<220>
   <223> Oligo motif
<400> 167
   tcaagttaca aaag 14
<210> 168
   <211> 14
   <212> DNA
   <213> Artificial
<220>
   <223> Oligo motif
<400> 168
   cccggcgggc agcc 14
<210> 169
   <211> 14
   <212> DNA
   <213> Artificial
<220>
   <223> Oligo motif
<400> 169
   acatggggca actt 14
<210> 170
   <211> 14
   <212> DNA
   <213> Artificial
<220>
   <223> Oligo motif
<400> 170
   gtgcccttcc cttg 14
<210> 171
   <211> 14
   <212> DNA
   <213> Artificial
<220>
   <223> Oligo motif
<400> 171
   ccgaataaac tcca 14
<210> 172
   <211> 14
   <212> DNA
   <213> Artificial
<220>
   <223> Oligo motif
<400> 172
   ctggggagta gagg 14
<210> 173
   <211> 14
   <212> DNA
   <213> Artificial
<220>
   <223> Oligo motif
<400> 173
   tcccactctg tgac 14
<210> 174
   <211> 14
   <212> DNA
   <213> Artificial
<220>
   <223> Oligo motif
<400> 174
   gtgggcagca gcct 14
<210> 175
   <211> 14
   <212> DNA
   <213> Artificial
<220>
   <223> Oligo motif
<400> 175
   gagaagtgga tcag 14
<210> 176
   <211> 14
   <212> DNA
   <213> Artificial
<220>
   <223> Oligo motif
<400> 176
   gtcctcaggg aacc 14
<210> 177
   <211> 14
   <212> DNA
   <213> Artificial
<220>
   <223> Oligo motif
<400> 177
   caggttgggg gtca 14
<210> 178
   <211> 14
   <212> DNA
   <213> Artificial
<220>
   <223> Oligo motif
<400> 178
   ccctcacccc caaa 14
<210> 179
   <211> 14
   <212> DNA
   <213> Artificial
<220>
   <223> Oligo motif
<400> 179
   ggcatggcag cagg 14
<210> 180
   <211> 14
   <212> DNA
   <213> Artificial
<220>
   <223> Oligo motif
<400> 180
   tggggcatgg cagc 14
<210> 181
   <211> 14
   <212> DNA
   <213> Artificial
<220>
   <223> Oligo motif
<400> 181
   tccggcagca gatg 14
<210> 182
   <211> 14
   <212> DNA
   <213> Artificial
<220>
   <223> Oligo motif
<400> 182
   caagttacaa aagc 14
<210> 183
   <211> 14
   <212> DNA
   <213> Artificial
<220>
   <223> Oligo motif
<400> 183
   gggatgctct gggc 14
<210> 184
   <211> 14
   <212> DNA
   <213> Artificial
<220>
   <223> Oligo motif
<400> 184
   cgctccaggt tcca 14
<210> 185
   <211> 14
   <212> DNA
   <213> Artificial
<220>
   <223> Oligo motif
<400> 185
   gggcagcagc ctgt 14
<210> 186
   <211> 14
   <212> DNA
   <213> Artificial
<220>
   <223> Oligo motif
<400> 186
   catggcagca ggaa 14
<210> 187
   <211> 14
   <212> DNA
   <213> Artificial
<220>
   <223> Oligo motif
<400> 187
   catgggtgct gggg 14
<210> 188
   <211> 14
   <212> DNA
   <213> Artificial
<220>
   <223> Oligo motif
<400> 188
   gcatcccggc cgct 14
<210> 189
   <211> 14
   <212> DNA
   <213> Artificial
<220>
   <223> Oligo motif
<400> 189
   gccacgtggg cagc 14
<210> 190
   <211> 14
   <212> DNA
   <213> Artificial
<220>
   <223> Oligo motif
<400> 190
   agacaccctc accc 14
<210> 191
   <211> 15
   <212> DNA
   <213> Artificial
<220>
   <223> Oligo motif
<400> 191
   ctgtctgtgg aagcg 15
<210> 192
   <211> 15
   <212> DNA
   <213> Artificial
<220>
   <223> Oligo motif
<400> 192
   cctgtctgtg gaagc 15
<210> 193
   <211> 15
   <212> DNA
   <213> Artificial
<220>
   <223> Oligo motif
<400> 193
   gcctgtctgt ggaag 15
<210> 194
   <211> 15
   <212> DNA
   <213> Artificial
<220>
   <223> Oligo motif
<400> 194
   ccggccgctg accac 15
<210> 195
   <211> 15
   <212> DNA
   <213> Artificial
<220>
   <223> Oligo motif
<400> 195
   cccggccgct gacca 15
<210> 196
   <211> 15
   <212> DNA
   <213> Artificial
<220>
   <223> Oligo motif
<400> 196
   tcccggccgc tgacc 15
<210> 197
   <211> 15
   <212> DNA
   <213> Artificial
<220>
   <223> Oligo motif
<400> 197
   atcccggccg ctgac 15
<210> 198
   <211> 15
   <212> DNA
   <213> Artificial
<220>
   <223> Oligo motif
<400> 198
   catcccggcc gctga 15
<210> 199
   <211> 15
   <212> DNA
   <213> Artificial
<220>
   <223> Oligo motif
<400> 199
   tatgagggtg ccgct 15
<210> 200
   <211> 15
   <212> DNA
   <213> Artificial
<220>
   <223> Oligo motif
<400> 200
   ctatgagggt gccgc 15
<210> 201
   <211> 15
   <212> DNA
   <213> Artificial
<220>
   <223> Oligo motif
<400> 201
   gcctatgagg gtgcc 15
<210> 202
   <211> 15
   <212> DNA
   <213> Artificial
<220>
   <223> Oligo motif
<400> 202
   ggcctatgag ggtgc 15
<210> 203
   <211> 15
   <212> DNA
   <213> Artificial
<220>
   <223> Oligo motif
<400> 203
   aggcctatga gggtg 15
<210> 204
   <211> 15
   <212> DNA
   <213> Artificial
<220>
   <223> Oligo motif
<400> 204
   caggcctatg agggt 15
<210> 205
   <211> 15
   <212> DNA
   <213> Artificial
<220>
   <223> Oligo motif
<400> 205
   ccaggcctat gaggg 15
<210> 206
   <211> 15
   <212> DNA
   <213> Artificial
<220>
   <223> Oligo motif
<400> 206
   tccaggccta tgagg 15
<210> 207
   <211> 15
   <212> DNA
   <213> Artificial
<220>
   <223> Oligo motif
<400> 207
   ctccaggcct atgag 15
<210> 208
   <211> 15
   <212> DNA
   <213> Artificial
<220>
   <223> Oligo motif
<400> 208
   actccaggcc tatga 15
<210> 209
   <211> 15
   <212> DNA
   <213> Artificial
<220>
   <223> Oligo motif
<400> 209
   aactccaggc ctatg 15
<210> 210
   <211> 15
   <212> DNA
   <213> Artificial
<220>
   <223> Oligo motif
<400> 210
   aaactccagg cctat 15
<210> 211
   <211> 15
   <212> DNA
   <213> Artificial
<220>
   <223> Oligo motif
<400> 211
   taaactccag gccta 15
<210> 212
   <211> 15
   <212> DNA
   <213> Artificial
<220>
   <223> Oligo motif
<400> 212
   ataaactcca ggcct 15
<210> 213
   <211> 15
   <212> DNA
   <213> Artificial
<220>
   <223> Oligo motif
<400> 213
   aataaactcc aggcc 15
<210> 214
   <211> 15
   <212> DNA
   <213> Artificial
<220>
   <223> Oligo motif
<400> 214
   gaataaactc caggc 15
<210> 215
   <211> 15
   <212> DNA
   <213> Artificial
<220>
   <223> Oligo motif
<400> 215
   cgaataaact ccagg 15
<210> 216
   <211> 15
   <212> DNA
   <213> Artificial
<220>
   <223> Oligo motif
<400> 216
   ttccgaataa actcc 15
<210> 217
   <211> 15
   <212> DNA
   <213> Artificial
<220>
   <223> Oligo motif
<400> 217
   ggagtagagg caggc 15
<210> 218
   <211> 15
   <212> DNA
   <213> Artificial
<220>
   <223> Oligo motif
<400> 218
   ggggagtaga ggcag 15
<210> 219
   <211> 15
   <212> DNA
   <213> Artificial
<220>
   <223> Oligo motif
<400> 219
   tggggagtag aggca 15
<210> 220
   <211> 15
   <212> DNA
   <213> Artificial
<220>
   <223> Oligo motif
<400> 220
   ctggggagta gaggc 15
<210> 221
   <211> 15
   <212> DNA
   <213> Artificial
<220>
   <223> Oligo motif
<400> 221
   gctggggagt agagg 15
<210> 222
   <211> 15
   <212> DNA
   <213> Artificial
<220>
   <223> Oligo motif
<400> 222
   gtccccaaag tcccc 15
<210> 223
   <211> 15
   <212> DNA
   <213> Artificial
<220>
   <223> Oligo motif
<400> 223
   gtcccactct gtgac 15
<210> 224
   <211> 15
   <212> DNA
   <213> Artificial
<220>
   <223> Oligo motif
<400> 224
   gccacgtggg cagca 15
<210> 225
   <211> 15
   <212> DNA
   <213> Artificial
<220>
   <223> Oligo motif
<400> 225
   agagaagtgg atcag 15
<210> 226
   <211> 15
   <212> DNA
   <213> Artificial
<220>
   <223> Oligo motif
<400> 226
   ctcagggaac caggc 15
<210> 227
   <211> 15
   <212> DNA
   <213> Artificial
<220>
   <223> Oligo motif
<400> 227
   gtcctcaggg aacca 15
<210> 228
   <211> 15
   <212> DNA
   <213> Artificial
<220>
   <223> Oligo motif
<400> 228
   caggttgggg gtcag 15
<210> 229
   <211> 15
   <212> DNA
   <213> Artificial
<220>
   <223> Oligo motif
<400> 229
   ccaggttggg ggtca 15
<210> 230
   <211> 15
   <212> DNA
   <213> Artificial
<220>
   <223> Oligo motif
<400> 230
   accaggttgg gggtc 15
<210> 231
   <211> 15
   <212> DNA
   <213> Artificial
<220>
   <223> Oligo motif
<400> 231
   atgggtgctg ggggg 15
<210> 232
   <211> 15
   <212> DNA
   <213> Artificial
<220>
   <223> Oligo motif
<400> 232
   tggagcagct cagca 15
<210> 233
   <211> 15
   <212> DNA
   <213> Artificial
<220>
   <223> Oligo motif
<400> 233
   accctcaccc ccaaa 15
<210> 234
   <211> 15
   <212> DNA
   <213> Artificial
<220>
   <223> Oligo motif
<400> 234
   caccctcacc cccaa 15
<210> 235
   <211> 15
   <212> DNA
   <213> Artificial
<220>
   <223> Oligo motif
<400> 235
   acaccctcac cccca 15
<210> 236
   <211> 15
   <212> DNA
   <213> Artificial
<220>
   <223> Oligo motif
<400> 236
   gacaccctca ccccc 15
<210> 237
   <211> 15
   <212> DNA
   <213> Artificial
<220>
   <223> Oligo motif
<400> 237
   agacaccctc acccc 15
<210> 238
   <211> 15
   <212> DNA
   <213> Artificial
<220>
   <223> Oligo motif
<400> 238
   tagacaccct caccc 15
<210> 239
   <211> 15
   <212> DNA
   <213> Artificial
<220>
   <223> Oligo motif
<400> 239
   gcatggcagc aggaa 15
<210> 240
   <211> 15
   <212> DNA
   <213> Artificial
<220>
   <223> Oligo motif
<400> 240
   ggcatggcag cagga 15
<210> 241
   <211> 15
   <212> DNA
   <213> Artificial
<220>
   <223> Oligo motif
<400> 241
   ggggcatggc agcag 15
<210> 242
   <211> 15
   <212> DNA
   <213> Artificial
<220>
   <223> Oligo motif
<400> 242
   tggggcatgg cagca 15
<210> 243
   <211> 15
   <212> DNA
   <213> Artificial
<220>
   <223> Oligo motif
<400> 243
   ctggggcatg gcagc 15
<210> 244
   <211> 15
   <212> DNA
   <213> Artificial
<220>
   <223> Oligo motif
<400> 244
   cggcagcaga tggca 15
<210> 245
   <211> 15
   <212> DNA
   <213> Artificial
<220>
   <223> Oligo motif
<400> 245
   ccggcagcag atggc 15
<210> 246
   <211> 15
   <212> DNA
   <213> Artificial
<220>
   <223> Oligo motif
<400> 246
   tccggcagca gatgg 15
<210> 247
   <211> 15
   <212> DNA
   <213> Artificial
<220>
   <223> Oligo motif
<400> 247
   ctccggcagc agatg 15
<210> 248
   <211> 15
   <212> DNA
   <213> Artificial
<220>
   <223> Oligo motif
<400> 248
   gctccggcag cagat 15
<210> 249
   <211> 15
   <212> DNA
   <213> Artificial
<220>
   <223> Oligo motif
<400> 249
   ggctccggca gcaga 15
<210> 250
   <211> 15
   <212> DNA
   <213> Artificial
<220>
   <223> Oligo motif
<400> 250
   ttaatcaggg agccc 15
<210> 251
   <211> 15
   <212> DNA
   <213> Artificial
<220>
   <223> Oligo motif
<400> 251
   tcaagttaca aaagc 15
<210> 252
   <211> 15
   <212> DNA
   <213> Artificial
<220>
   <223> Oligo motif
<400> 252
   catggggcaa cttca 15
<210> 253
   <211> 15
   <212> DNA
   <213> Artificial
<220>
   <223> Oligo motif
<400> 253
   acatggggca acttc 15
<210> 254
   <211> 15
   <212> DNA
   <213> Artificial
<220>
   <223> Oligo motif
<400> 254
   agatacacct ccacc 15
<210> 255
   <211> 15
   <212> DNA
   <213> Artificial
<220>
   <223> Oligo motif
<400> 255
   gagatacacc tccac 15
<210> 256
   <211> 15
   <212> DNA
   <213> Artificial
<220>
   <223> Oligo motif
<400> 256
   tgggcagcag cctgt 15
<210> 257
   <211> 15
   <212> DNA
   <213> Artificial
<220>
   <223> Oligo motif
<400> 257
   acgtgggcag cagcc 15
<210> 258
   <211> 15
   <212> DNA
   <213> Artificial
<220>
   <223> Oligo motif
<400> 258
   cacgtgggca gcagc 15
<210> 259
   <211> 15
   <212> DNA
   <213> Artificial
<220>
   <223> Oligo motif
<400> 259
   tcctcaggga accag 15
<210> 260
   <211> 15
   <212> DNA
   <213> Artificial
<220>
   <223> Oligo motif
<400> 260
   catgggtgct ggggg 15
<210> 261
   <211> 15
   <212> DNA
   <213> Artificial
<220>
   <223> Oligo motif
<400> 261
   gcagctcagc agctc 15
<210> 262
   <211> 15
   <212> DNA
   <213> Artificial
<220>
   <223> Oligo motif
<400> 262
   gagcagctca gcagc 15
<210> 263
   <211> 15
   <212> DNA
   <213> Artificial
<220>
   <223> Oligo motif
<400> 263
   ggagcagctc agcag 15
<210> 264
   <211> 15
   <212> DNA
   <213> Artificial
<220>
   <223> Oligo motif
<400> 264
   ccacgtgggc agcag 15
<210> 265
   <211> 15
   <212> DNA
   <213> Artificial
<220>
   <223> Oligo motif
<400> 265
   gctggtcctc aggga 15
<210> 266
   <211> 15
   <212> DNA
   <213> Artificial
<220>
   <223> Oligo motif
<400> 266
   tgggtgctgg ggggc 15
<210> 267
   <211> 15
   <212> DNA
   <213> Artificial
<220>
   <223> Oligo motif
<400> 267
   cctgccaggt gggtg 15
<210> 268
   <211> 15
   <212> DNA
   <213> Artificial
<220>
   <223> Oligo motif
<400> 268
   gcatcccggc cgctg 15
<210> 269
   <211> 15
   <212> DNA
   <213> Artificial
<220>
   <223> Oligo motif
<400> 269
   cctatgaggg tgccg 15
<210> 270
   <211> 15
   <212> DNA
   <213> Artificial
<220>
   <223> Oligo motif
<400> 270
   ccgaataaac tccag 15
<210> 271
   <211> 15
   <212> DNA
   <213> Artificial
<220>
   <223> Oligo motif
<400> 271
   tccgaataaa ctcca 15
<210> 272
   <211> 15
   <212> DNA
   <213> Artificial
<220>
   <223> Oligo motif
<400> 272
   tccccaaagt cccca 15
<210> 273
   <211> 15
   <212> DNA
   <213> Artificial
<220>
   <223> Oligo motif
<400> 273
   tcccactctg tgaca 15
<210> 274
   <211> 15
   <212> DNA
   <213> Artificial
<220>
   <223> Oligo motif
<400> 274
   ggcagcagcc tgtga 15
<210> 275
   <211> 15
   <212> DNA
   <213> Artificial
<220>
   <223> Oligo motif
<400> 275
   cgtgggcagc agcct 15
<210> 276
   <211> 15
   <212> DNA
   <213> Artificial
<220>
   <223> Oligo motif
<400> 276
   tcagggaacc aggcc 15
<210> 277
   <211> 15
   <212> DNA
   <213> Artificial
<220>
   <223> Oligo motif
<400> 277
   cctcagggaa ccagg 15
<210> 278
   <211> 15
   <212> DNA
   <213> Artificial
<220>
   <223> Oligo motif
<400> 278
   caccaggttg ggggt 15
<210> 279
   <211> 15
   <212> DNA
   <213> Artificial
<220>
   <223> Oligo motif
<400> 279
   tggtggcagt ggaca 15
<210> 280
   <211> 15
   <212> DNA
   <213> Artificial
<220>
   <223> Oligo motif
<400> 280
   catggcagca ggaag 15
<210> 281
   <211> 15
   <212> DNA
   <213> Artificial
<220>
   <223> Oligo motif
<400> 281
   gggcatggca gcagg 15
<210> 282
   <211> 15
   <212> DNA
   <213> Artificial
<220>
   <223> Oligo motif
<400> 282
   cctggggcat ggcag 15
<210> 283
   <211> 15
   <212> DNA
   <213> Artificial
<220>
   <223> Oligo motif
<400> 283
   gccggctccg gcagc 15
<210> 284
   <211> 15
   <212> DNA
   <213> Artificial
<220>
   <223> Oligo motif
<400> 284
   ccccggcggg cagcc 15
<210> 285
   <211> 15
   <212> DNA
   <213> Artificial
<220>
   <223> Oligo motif
<400> 285
   ctgccaggtg ggtgc 15
<210> 286
   <211> 15
   <212> DNA
   <213> Artificial
<220>
   <223> Oligo motif
<400> 286
   gggcagcagc ctgtg 15
<210> 287
   <211> 15
   <212> DNA
   <213> Artificial
<220>
   <223> Oligo motif
<400> 287
   gtgggcagca gcctg 15
<210> 288
   <211> 15
   <212> DNA
   <213> Artificial
<220>
   <223> Oligo motif
<400> 288
   ggtcctcagg gaacc 15
<210> 289
   <211> 15
   <212> DNA
   <213> Artificial
<220>
   <223> Oligo motif
<400> 289
   tggtcctcag ggaac 15
<210> 290
   <211> 15
   <212> DNA
   <213> Artificial
<220>
   <223> Oligo motif
<400> 290
   gccaccaggt tgggg 15
<210> 291
   <211> 15
   <212> DNA
   <213> Artificial
<220>
   <223> Oligo motif
<400> 291
   cagctcagca gctcc 15
<210> 292
   <211> 15
   <212> DNA
   <213> Artificial
<220>
   <223> Oligo motif
<400> 292
   agctgcagcc tgtga 15
<210> 293
   <211> 15
   <212> DNA
   <213> Artificial
<220>
   <223> Oligo motif
<400> 293
   ccggctccgg cagca 15
<210> 294
   <211> 15
   <212> DNA
   <213> Artificial
<220>
   <223> Oligo motif
<400> 294
   caagttacaa aagca 15
<210> 295
   <211> 15
   <212> DNA
   <213> Artificial
<220>
   <223> Oligo motif
<400> 295
   gggagtagag gcagg 15
<210> 296
   <211> 15
   <212> DNA
   <213> Artificial
<220>
   <223> Oligo motif
<400> 296
   agcagctcag cagct 15
<210> 297
   <211> 15
   <212> DNA
   <213> Artificial
<220>
   <223> Oligo motif
<400> 297
   atggcagcag gaagc 15
<210> 298
   <211> 15
   <212> DNA
   <213> Artificial
<220>
   <223> Oligo motif
<400> 298
   tgccaggtgg gtgcc 15
<210> 299
   <211> 16
   <212> DNA
   <213> Artificial
<220>
   <223> Oligo motif
<400> 299
   cctgtctgtg gaagcg 16
<210> 300
   <211> 16
   <212> DNA
   <213> Artificial
<220>
   <223> Oligo motif
<400> 300
   gcctgtctgt ggaagc 16
<210> 301
   <211> 16
   <212> DNA
   <213> Artificial
<220>
   <223> Oligo motif
<400> 301
   cccggccgct gaccac 16
<210> 302
   <211> 16
   <212> DNA
   <213> Artificial
<220>
   <223> Oligo motif
<400> 302
   tcccggccgc tgacca 16
<210> 303
   <211> 16
   <212> DNA
   <213> Artificial
<220>
   <223> Oligo motif
<400> 303
   atcccggccg ctgacc 16
<210> 304
   <211> 16
   <212> DNA
   <213> Artificial
<220>
   <223> Oligo motif
<400> 304
   catcccggcc gctgac 16
<210> 305
   <211> 16
   <212> DNA
   <213> Artificial
<220>
   <223> Oligo motif
<400> 305
   gcatcccggc cgctga 16
<210> 306
   <211> 16
   <212> DNA
   <213> Artificial
<220>
   <223> Oligo motif
<400> 306
   ctatgagggt gccgct 16
<210> 307
   <211> 16
   <212> DNA
   <213> Artificial
<220>
   <223> Oligo motif
<400> 307
   cctatgaggg tgccgc 16
<210> 308
   <211> 16
   <212> DNA
   <213> Artificial
<220>
   <223> Oligo motif
<400> 308
   gcctatgagg gtgccg 16
<210> 309
   <211> 16
   <212> DNA
   <213> Artificial
<220>
   <223> Oligo motif
<400> 309
   ggcctatgag ggtgcc 16
<210> 310
   <211> 16
   <212> DNA
   <213> Artificial
<220>
   <223> Oligo motif
<400> 310
   aggcctatga gggtgc 16
<210> 311
   <211> 16
   <212> DNA
   <213> Artificial
<220>
   <223> Oligo motif
<400> 311
   caggcctatg agggtg 16
<210> 312
   <211> 16
   <212> DNA
   <213> Artificial
<220>
   <223> Oligo motif
<400> 312
   ccaggcctat gagggt 16
<210> 313
   <211> 16
   <212> DNA
   <213> Artificial
<220>
   <223> Oligo motif
<400> 313
   tccaggccta tgaggg 16
<210> 314
   <211> 16
   <212> DNA
   <213> Artificial
<220>
   <223> Oligo motif
<400> 314
   ctccaggcct atgagg 16
<210> 315
   <211> 16
   <212> DNA
   <213> Artificial
<220>
   <223> Oligo motif
<400> 315
   actccaggcc tatgag 16
<210> 316
   <211> 16
   <212> DNA
   <213> Artificial
<220>
   <223> Oligo motif
<400> 316
   aactccaggc ctatga 16
<210> 317
   <211> 16
   <212> DNA
   <213> Artificial
<220>
   <223> Oligo motif
<400> 317
   aaactccagg cctatg 16
<210> 318
   <211> 16
   <212> DNA
   <213> Artificial
<220>
   <223> Oligo motif
<400> 318
   taaactccag gcctat 16
<210> 319
   <211> 16
   <212> DNA
   <213> Artificial
<220>
   <223> Oligo motif
<400> 319
   ataaactcca ggccta 16
<210> 320
   <211> 16
   <212> DNA
   <213> Artificial
<220>
   <223> Oligo motif
<400> 320
   aataaactcc aggcct 16
<210> 321
   <211> 16
   <212> DNA
   <213> Artificial
<220>
   <223> Oligo motif
<400> 321
   gaataaactc caggcc 16
<210> 322
   <211> 16
   <212> DNA
   <213> Artificial
<220>
   <223> Oligo motif
<400> 322
   cgaataaact ccaggc 16
<210> 323
   <211> 16
   <212> DNA
   <213> Artificial
<220>
   <223> Oligo motif
<400> 323
   ccgaataaac tccagg 16
<210> 324
   <211> 16
   <212> DNA
   <213> Artificial
<220>
   <223> Oligo motif
<400> 324
   ttccgaataa actcca 16
<210> 325
   <211> 16
   <212> DNA
   <213> Artificial
<220>
   <223> Oligo motif
<400> 325
   gggagtagag gcaggc 16
<210> 326
   <211> 16
   <212> DNA
   <213> Artificial
<220>
   <223> Oligo motif
<400> 326
   ggggagtaga ggcagg 16
<210> 327
   <211> 16
   <212> DNA
   <213> Artificial
<220>
   <223> Oligo motif
<400> 327
   tggggagtag aggcag 16
<210> 328
   <211> 16
   <212> DNA
   <213> Artificial
<220>
   <223> Oligo motif
<400> 328
   ctggggagta gaggca 16
<210> 329
   <211> 16
   <212> DNA
   <213> Artificial
<220>
   <223> Oligo motif
<400> 329
   gctggggagt agaggc 16
<210> 330
   <211> 16
   <212> DNA
   <213> Artificial
<220>
   <223> Oligo motif
<400> 330
   gtccccaaag tcccca 16
<210> 331
   <211> 16
   <212> DNA
   <213> Artificial
<220>
   <223> Oligo motif
<400> 331
   gtcccactct gtgaca 16
<210> 332
   <211> 16
   <212> DNA
   <213> Artificial
<220>
   <223> Oligo motif
<400> 332
   gggcagcagc ctgtga 16
<210> 333
   <211> 16
   <212> DNA
   <213> Artificial
<220>
   <223> Oligo motif
<400> 333
   tgggcagcag cctgtg 16
<210> 334
   <211> 16
   <212> DNA
   <213> Artificial
<220>
   <223> Oligo motif
<400> 334
   gtgggcagca gcctgt 16
<210> 335
   <211> 16
   <212> DNA
   <213> Artificial
<220>
   <223> Oligo motif
<400> 335
   acgtgggcag cagcct 16
<210> 336
   <211> 16
   <212> DNA
   <213> Artificial
<220>
   <223> Oligo motif
<400> 336
   gccacgtggg cagcag 16
<210> 337
   <211> 16
   <212> DNA
   <213> Artificial
<220>
   <223> Oligo motif
<400> 337
   ctcagggaac caggcc 16
<210> 338
   <211> 16
   <212> DNA
   <213> Artificial
<220>
   <223> Oligo motif
<400> 338
   cctcagggaa ccaggc 16
<210> 339
   <211> 16
   <212> DNA
   <213> Artificial
<220>
   <223> Oligo motif
<400> 339
   tcctcaggga accagg 16
<210> 340
   <211> 16
   <212> DNA
   <213> Artificial
<220>
   <223> Oligo motif
<400> 340
   gtcctcaggg aaccag 16
<210> 341
   <211> 16
   <212> DNA
   <213> Artificial
<220>
   <223> Oligo motif
<400> 341
   ggtcctcagg gaacca 16
<210> 342
   <211> 16
   <212> DNA
   <213> Artificial
<220>
   <223> Oligo motif
<400> 342
   gctggtcctc agggaa 16
<210> 343
   <211> 16
   <212> DNA
   <213> Artificial
<220>
   <223> Oligo motif
<400> 343
   ccaggttggg ggtcag 16
<210> 344
   <211> 16
   <212> DNA
   <213> Artificial
<220>
   <223> Oligo motif
<400> 344
   accaggttgg gggtca 16
<210> 345
   <211> 16
   <212> DNA
   <213> Artificial
<220>
   <223> Oligo motif
<400> 345
   caccaggttg ggggtc 16
<210> 346
   <211> 16
   <212> DNA
   <213> Artificial
<220>
   <223> Oligo motif
<400> 346
   ccaccaggtt gggggt 16
<210> 347
   <211> 16
   <212> DNA
   <213> Artificial
<220>
   <223> Oligo motif
<400> 347
   tgggtgctgg ggggca 16
<210> 348
   <211> 16
   <212> DNA
   <213> Artificial
<220>
   <223> Oligo motif
<400> 348
   atgggtgctg gggggc 16
<210> 349
   <211> 16
   <212> DNA
   <213> Artificial
<220>
   <223> Oligo motif
<400> 349
   catgggtgct gggggg 16
<210> 350
   <211> 16
   <212> DNA
   <213> Artificial
<220>
   <223> Oligo motif
<400> 350
   gcagctcagc agctcc 16
<210> 351
   <211> 16
   <212> DNA
   <213> Artificial
<220>
   <223> Oligo motif
<400> 351
   agcagctcag cagctc 16
<210> 352
   <211> 16
   <212> DNA
   <213> Artificial
<220>
   <223> Oligo motif
<400> 352
   gagcagctca gcagct 16
<210> 353
   <211> 16
   <212> DNA
   <213> Artificial
<220>
   <223> Oligo motif
<400> 353
   ggagcagctc agcagc 16
<210> 354
   <211> 16
   <212> DNA
   <213> Artificial
<220>
   <223> Oligo motif
<400> 354
   tggagcagct cagcag 16
<210> 355
   <211> 16
   <212> DNA
   <213> Artificial
<220>
   <223> Oligo motif
<400> 355
   ctggagcagc tcagca 16
<210> 356
   <211> 16
   <212> DNA
   <213> Artificial
<220>
   <223> Oligo motif
<400> 356
   caccctcacc cccaaa 16
<210> 357
   <211> 16
   <212> DNA
   <213> Artificial
<220>
   <223> Oligo motif
<400> 357
   acaccctcac ccccaa 16
<210> 358
   <211> 16
   <212> DNA
   <213> Artificial
<220>
   <223> Oligo motif
<400> 358
   gacaccctca ccccca 16
<210> 359
   <211> 16
   <212> DNA
   <213> Artificial
<220>
   <223> Oligo motif
<400> 359
   agacaccctc accccc 16
<210> 360
   <211> 16
   <212> DNA
   <213> Artificial
<220>
   <223> Oligo motif
<400> 360
   tagacaccct cacccc 16
<210> 361
   <211> 16
   <212> DNA
   <213> Artificial
<220>
   <223> Oligo motif
<400> 361
   catggcagca ggaagc 16
<210> 362
   <211> 16
   <212> DNA
   <213> Artificial
<220>
   <223> Oligo motif
<400> 362
   gcatggcagc aggaag 16
<210> 363
   <211> 16
   <212> DNA
   <213> Artificial
<220>
   <223> Oligo motif
<400> 363
   ggcatggcag caggaa 16
<210> 364
   <211> 16
   <212> DNA
   <213> Artificial
<220>
   <223> Oligo motif
<400> 364
   gggcatggca gcagga 16
<210> 365
   <211> 16
   <212> DNA
   <213> Artificial
<220>
   <223> Oligo motif
<400> 365
   ggggcatggc agcagg 16
<210> 366
   <211> 16
   <212> DNA
   <213> Artificial
<220>
   <223> Oligo motif
<400> 366
   tggggcatgg cagcag 16
<210> 367
   <211> 16
   <212> DNA
   <213> Artificial
<220>
   <223> Oligo motif
<400> 367
   ctggggcatg gcagca 16
<210> 368
   <211> 16
   <212> DNA
   <213> Artificial
<220>
   <223> Oligo motif
<400> 368
   cctggggcat ggcagc 16
<210> 369
   <211> 16
   <212> DNA
   <213> Artificial
<220>
   <223> Oligo motif
<400> 369
   ccggcagcag atggca 16
<210> 370
   <211> 16
   <212> DNA
   <213> Artificial
<220>
   <223> Oligo motif
<400> 370
   tccggcagca gatggc 16
<210> 371
   <211> 16
   <212> DNA
   <213> Artificial
<220>
   <223> Oligo motif
<400> 371
   ctccggcagc agatgg 16
<210> 372
   <211> 16
   <212> DNA
   <213> Artificial
<220>
   <223> Oligo motif
<400> 372
   gctccggcag cagatg 16
<210> 373
   <211> 16
   <212> DNA
   <213> Artificial
<220>
   <223> Oligo motif
<400> 373
   ggctccggca gcagat 16
<210> 374
   <211> 16
   <212> DNA
   <213> Artificial
<220>
   <223> Oligo motif
<400> 374
   cggctccggc agcaga 16
<210> 375
   <211> 16
   <212> DNA
   <213> Artificial
<220>
   <223> Oligo motif
<400> 375
   gccggctccg gcagca 16
<210> 376
   <211> 16
   <212> DNA
   <213> Artificial
<220>
   <223> Oligo motif
<400> 376
   caagttacaa aagcaa 16
<210> 377
   <211> 16
   <212> DNA
   <213> Artificial
<220>
   <223> Oligo motif
<400> 377
   tcaagttaca aaagca 16
<210> 378
   <211> 16
   <212> DNA
   <213> Artificial
<220>
   <223> Oligo motif
<400> 378
   acatggggca acttca 16
<210> 379
   <211> 16
   <212> DNA
   <213> Artificial
<220>
   <223> Oligo motif
<400> 379
   gagatacacc tccacc 16
<210> 380
   <211> 16
   <212> DNA
   <213> Artificial
<220>
   <223> Oligo motif
<400> 380
   cacgtgggca gcagcc 16
<210> 381
   <211> 16
   <212> DNA
   <213> Artificial
<220>
   <223> Oligo motif
<400> 381
   ccacgtgggc agcagc 16
<210> 382
   <211> 16
   <212> DNA
   <213> Artificial
<220>
   <223> Oligo motif
<400> 382
   tggtcctcag ggaacc 16
<210> 383
   <211> 16
   <212> DNA
   <213> Artificial
<220>
   <223> Oligo motif
<400> 383
   ctgccaggtg ggtgcc 16
<210> 384
   <211> 16
   <212> DNA
   <213> Artificial
<220>
   <223> Oligo motif
<400> 384
   cctgccaggt gggtgc 16
<210> 385
   <211> 16
   <212> DNA
   <213> Artificial
<220>
   <223> Oligo motif
<400> 385
   tccgaataaa ctccag 16
<210> 386
   <211> 16
   <212> DNA
   <213> Artificial
<220>
   <223> Oligo motif
<400> 386
   cgtgggcagc agcctg 16
<210> 387
   <211> 16
   <212> DNA
   <213> Artificial
<220>
   <223> Oligo motif
<400> 387
   agctgcagcc tgtgag 16
<210> 388
   <211> 16
   <212> DNA
   <213> Artificial
<220>
   <223> Oligo motif
<400> 388
   ccggctccgg cagcag 16
<210> 389
   <211> 16
   <212> DNA
   <213> Artificial
<220>
   <223> Oligo motif
<400> 389
   ctggtcctca gggaac 16
<210> 390
   <211> 16
   <212> DNA
   <213> Artificial
<220>
   <223> Oligo motif
<400> 390
   gccaccaggt tggggg 16
<210> 391
   <211> 16
   <212> DNA
   <213> Artificial
<220>
   <223> Oligo motif
<400> 391
   ggccaccagg ttgggg 16
<210> 392
   <211> 16
   <212> DNA
   <213> Artificial
<220>
   <223> Oligo motif
<400> 392
   agctcagcag ctcctc 16
<210> 393
   <211> 16
   <212> DNA
   <213> Artificial
<220>
   <223> Oligo motif
<400> 393
   cagctcagca gctcct 16